# EUROPEAN PATENT APPLICATION

(11) **EP 2 597 163 A1**
(43) Date of publication of application: **29.05.2013**
(21) Application number: 13151181.8
(22) Date of filing: 20.05.2009
(51) Int. Cl.: C12Q 1/70, A61K 38/45, C12Q 1/68, C12N 9/12

(54) **The large form of human 2',5'-oligoadenylate synthetase OAS3 for preventing or treating infection with positive-sense single-stranded RNA viruses**

(30) Priority: 20.05.2008 EP 08290470
(62) Divisional of application: 09750188.6
(71) Applicant: Institut Pasteur, 75015 Paris (FR)
(72) Inventor: Brehin, Anne-Claire, 75011 Paris (FR); Sakuntabhai, Anavaj, 75011 Paris (FR); Despres, Philippe, 92250 La Garenne-Colombes (FR); Casademont, Isabelle, 78370 Plaisir (FR); Julier, Cécile, 75014 Paris (FR); Chuansumrit, Ampaiwan, 10100 Bangkok (TH); Malasit, Prida, 73710 Nakhon Pathom (TH); Paulous, Sylvie, 95200 Sarcelles (FR)
(74) Representative: Leblois-Préhaud, Hélène Marthe Georgette

(57) **Abstract**

Use of the large form of human 2',5'-Oligoadenylate Synthetase (OAS3) for diagnosis, prevention and treatment of infection with positive-sense single-stranded RNA viruses and for prediction of human genetic susceptibility to positive-sense single-stranded RNA virus-related diseases.

## Description

The invention relates to the large form of human 2',5'-OligoAdenylate Synthetase (OAS3) as a medicament for preventing infection with or treating positive-sense single-stranded RNA viruses.

The invention relates also the large form of human 2',5'-OligoAdenylate Synthetase (OAS3) as a marker for determining the genetic susceptibility to infection with positive-sense single-stranded RNA viruses.

*Flaviviridae* and *Togaviridae* are two positive-sense single-stranded (ss)RNA virus families comprising pathogens that can affect human and animal health world wide. Examples of these viruses include members of dengue, yellow fever (YF), Japanese encephalitis (JE) and tick-borne encephalitis (TBE) antigenic complexes of the *Flavivirus* genus (Flaviviridae family), members of Eastern Equine Encephalitis (EEE)/ Venezuelan Equine Encephalitis (VEE), Semliki Forest (SF), and Sindbis (SIN) groups of *Alphavirus* genus (Toagaviridae family), and members of *Hepacivirus* genus (Flaviviridae family) such as Hepatitis C (HCV) and Hepatitis G (HGV) viruses.

Dengue virus, (DV; DEN antigenic complex of flavivirus genus) is endemic in most urban centers of the tropics since a dramatic increase in urbanization created ideal conditions for increased transmission of mosquito-borne dengue disease. The four serotypes of dengue virus (DV-1 to DV-4) are transmitted to humans by the mosquito vector *Ae. aegypti.* DV infection results in a spectrum of illnesses, ranging form a flu-like disease (dengue fever, DF) to dengue hemorrhagic fever (DHF) that can progress to dengue shock syndrome (DSS) and death. To date, dengue illness is the most important arbovirosis in humans with an estimated 100 millions cases and over 500,000 cases of DHF/DSS occurring each year, including about 25,000 fatal cases, mainly in children under the age 15. Epidemics with a high frequency of DHF/DSS continue to expand geographically in Asia and South America. Despite increased health and economic impacts, the pathogenesis of dengue disease is currently poorly understood. There is no available anti-dengue vaccine or specific therapy for the treatment of dengue virus infection.

West Nile virus (WNV; JE antigenic complex of flavivirus genus) circulates in natural transmission cycles involving mosquitoes (*Culex* species) and birds, and horses and human are incidental hosts. Zoonotic WNV became a major health concern in North America, the Middle East, and Europe, due to the emergence of a highly neuroinvasive strain in Israel in 1998 (variant Isr98/NY99 from clade la of the WN virus lineage I) (Ceccaldi et al., FEMS Microbiol. Lett., 2004, 233, 1-6). WNV infects the central nervous system and causes viral encephalitis in a large range of animal species. In Humans, clinical infections can range in severity from uncomplicated West Nile fever to fatal meningo-encephalitis. The emergence of WNV has been associated with a dramatic increase in the severity of infection in humans drawing the attention to viral encephalitis as a public health concern. Within the last 10 years, WNV has spread across the Western Hemisphere and the Caribbeans. The US outbreaks of WNV have involved thousands of patients causing severe neurological diseases (meningoencephalitis and poliomyelitis-like syndrome) and hundreds of associated deaths. Although mosquito-borne transmission of WNV is the predominant mode, WNV infection transmitted by blood transfusion, organ donation and transplacental transmission to the foetus were also recognized. There is no available vaccine or anti-viral therapy for WNV-related disease.

Chikungunya virus (CHIKV; SF group of alphavirus) is widespread throughout Africa, Southeast Asia, India and Western Pacific, and numerous epidemics have been reported in these areas. Clinically, infection by CHIKV results in fever, rash and intense, invalidating and sometimes persistent arthralgia. In 2005-06, CHIK virus has spread to the south of the Indian Ocean, particularly on La Réunion Island (France), where the outbreak has involved hundreds of thousand of patients. More recently the virus emerged in the east of Italy, where more than 200 people were infected.

Hepatitis C virus (HCV) infection is common worldwide; it is estimated that about 3 % of the world's population have HCV and there are about 4 million carriers in Europe alone. Between 20 and 30 % of these individuals will develop hepatic cirrhosis and its long-term sequelae such as hepatocellular carcinoma. Treatment of HCV infection with pegylated interferon alpha (IFN-α) in combination with ribavirin may achieve a sustained response in patients infected with HCV viral genotypes 2 or 3 (80 %) but the response rate is much lower in patients infected with HCV viral genotype 1 (42 %).

Innate antiviral mechanisms mediated by Type-I interferons (IFN-α/β) are potentially the most important pathways of host cell defense limiting viral replication. Indeed, IFN-α/β are able to trigger the activation of a specific signal transduction pathway leading to the induction of IFN-stimulated genes (ISGs) that are responsible for the establishment of an antiviral state. The ISGs believed to affect RNA virus replication in single cells are the RNA-specific Adenosine Desaminase (ADAR), the proteins of the myxovirus resistance (Mx) family, the double-stranded RNA-dependent protein kinase (PKR), and the 2',5'-oligoadenylate synthetase (2',5'-OAS or OAS) family associated to endoribonuclease RNase L.

The OAS/RNase L system is a RNA decay pathway known to play an important role in the established endogeneous antiviral pathway. Binding of enzymatically active OAS to activator double-stranded (ds) viral RNA results in the production of 2'- to 5'-linked oligoadenylates (2-5A). Latent monomeric RNase L is enzymatically activated through homodimerization induced by binding to 2-5A oligomers. Once activated RNase L degrades single-stranded RNA molecules including mRNA and viral RNA, suppressing viral replication (Silverman, J. Virol. 81: 12720,2007).

Human OAS is a family of enzymes encoded by three closely linked genes on chromosome 12q24.2, with the following order: small (OAS1, p40/46), medium (OAS2, p69/71), and large (OAS3, p100) OAS isoforms (Hovnanian et al., Genomics, 1998, 52, 267-277; Rebouillat, D. and Hovanessian, A.G., Journal of Interferon and Cytokine Research, 1999, 19, 295-308; Rebouillat et al., Genomics, 2000, 70, 232-240; Justesen et al., Cellular and Molecular Life Sciences, 2000, 57, 1593-1612; Rebouillat; Hovanessian, A.G., Cytokine and Growth Factor Reviews, 2007, 18, 351-361). Each *OAS* gene consists of a conserved *OAS* unit composed of five translated exons (exons A-E). *OAS1* has one unit, whereas *OAS2* and *OAS3* have two and three units, respectively, and all three genes encode active 2',5'-Oligoadenylate Synthetase. Another gene, OASL (OAS-Like) encodes a single-unit of OAS-like protein, which however, lacks 2'-5' synthetase activity (Hartmann et al., Nucleic Acids Res., 1998, 26, 4121-4128; Rebouillat et al., Eur. J. Biochem., 1998; 257, 319-330). Within each size class, multiple members arise as a result of alternate splicing of the primary transcript. The OAS proteins share a conserved unit/domain of about 350 amino acids (OAS unit); OAS1 (p40/p46), OAS2 (p69/71) and OAS3 (p100) contains one, two and three tandem copies of the OAS unit, respectively. Each OAS protein accumulates in different cellular locations, require different amounts of dsRNA to be activated, and catalyse the formation of differently sized 2-5A products. Whether OAS1 functions as a tetramer, and OAS2 is only active as a dimer, OAS3 has been observed only as a monomer. In addition, the large form of human OAS is presumably not involved in RNase L activation (*for review,* Rebouillat, D. and Hovanessian, A.G., Journal of Interferon and Cytokine Research, 1999, 19, 295-308).

The first direct evidence for the involvement of OAS family in the antiviral effect exhibited by IFN was provided by transfection of 2',5'-oligoadenylate synthetase (OAS) cDNA into cells. Overexpression of OAS1 or OAS2 leads to resistance of cells to picornavirus replication (Hovanessian, A.G., Cytokine and Growth Factor Reviews, 2007, 18, 351-361). The importance of OAS1 for clearing WNV infection *in vivo* was also supported by the finding that murine *Oas1b,* the orthologous gene of human *OAS1,* may play a key role into the susceptibility/resistance phenotype of mice to WNV-induced encephalitis (Mashimo, T. et al., Proc. Natl. Acad. Sci. USA, 2002, 99, 11311-11316; Lucas et al., Immunol. Cell. Biol., 2003, 81, 230-236; Kajaste-Rudnitski et al., Journal of Biological Chemistry, 2006, 281, 46244637; International PCT Application WO 02/081741). Analysis of the *OAS* genetic polymorphism in human demonstrated that genetic markers in *OAS* genes were the most strongly associated with enzyme activity. Given that *OAS1* is an excellent candidate for a human gene that influences host susceptibility to viral infection (Bonnevie-Nielsen et al., Am. J. Hum. Genet., 2005, 76, 623-633), genetic variations in human OAS1 as well as OASL genes were associated to the risk of viral encephalitis, type 1 DM, HCV related disease and other virus infection. With a particular emphasis on HCV disease, a series of *OAS1* genotypes linked with the outcome of HCV infection has been reported (International PCT Application WO 03/089003 and WO 2005/040428).

Whether antiviral activity of OAS family is selective for positive-sense ssRNA viruses is a critical issue that it remains to be investigated. It has been reported ectopic expression of OAS 1 leads to resistance to encephalomyocarditis virus (picornavirus) replication, but not VSV virus replication. However, *OAS* is considered to display no role in the elimination of HCV in patients treated with IFN-α, as opposed to other ISGs such as IFN-inducible RNA-dependent protein kinase (*PKR*) and myxovirus resistance 1 (*MxA*) genes. Transcription levels of *OAS, PKR* and Mx were up-regulated after DV infection in non-human primate model (Sariol et al., Clinical and Vaccine Immunology, 14, 2007, 756-766). The analysis of gene transcription pattern associated to Dengue Shock Syndrome showed that *OAS3* gene transcripts were less abundant in dengue patients with DSS than in those with non-DSS (Simmons et al., J.Infect.Dis., 195:1097, 2007). However, the role of these ISGs in the pathogenesis of DV infection remains poorly understood.

It has been reported that CHIKV is highly sensitive to the antiviral action of type I interferons (IFN-α/β), (Couderc et al., PloS Pathogens, 2007, 4,e29). In the case of alphaviruses, a body of evidence exists to suggest that IFN-mediated inhibition of virus growth does not require RNase L (Ryman et al., J. Virol., 2005, 79, 1487-1499; *for review,* Silverman, J. Virol., 2007, 81, 12720-9). Whether any members of human ISGs such as *OAS* family could exert antialphaviral activity is a critical issue that it remained to be investigated.

For the first time, the inventors demonstrate a role for OAS3 in the established endogenous antiviral pathway against positive-sense ssRNA viruses such as alphaviruses (CHIKV, SINV, and SFV). They show that OAS3 acts on the stages of CHIKV growth in blocking viral protein synthesis and viral RNA replication inside the infected human epithelial cells. Very little information is available on the human genetic susceptibility to alphavirus infection. Screening the *OAS3* gene for polymorphism in healthly Caucasian individuals identified a single-nucleotide polymorphism (SNP) at the first position of codon CGA-844 where the substitution T for C resulted in a non-sense mutation (OAS3.R844X). The SNP at position OAS3.R844X is expected to result in a truncated form of OAS3 protein, lacking about 20 % from the carboxy-terminus. Ectopic expression of mutant OAS3 resulted in a lower efficiency of CHIKV inhibition as compared to full-length OAS3 protein. The notion that genetic polymorphism of OAS3 could control its antialphaviral activity suggests a role of human OAS genes in the pathogenesis of alphavirus-related disease such as Chikungunya fever.

For the first time, the inventors demonstrate that OAS3 exerts antiflaviviral activity in human cells infected with DV (hepatoma cells) and WNV (hepatoma and epithelial cells). Inspection of the complete *OAS3* gene in DSS patients and non-DSS dengue patients identified the SNP *rs2285993* at the 3^{rd} position of codon AGG-381 where the substitution G to C resulted to amino acid change from Arg to Ser. Our genetic data suggest that variant Ser-381 is associated with a dominant protection against the risk of DSS in dengue Thai patients. The inventors demonstrate here that OAS3 with Ser-381 is a potent inhibitor of DV growth in human hepatocytes.

The inventors also demonstrate that live-attenuated vaccine strain 17D-204 of YFV (STAMARIL, Sanofi-Pasteur) has inherent resistance to OAS3-mediated antiviral pathway in infected human epithelial and hepatoma cells. However IFN-α was able to establish an antiviral state against vaccine strain 17D-204 of YFV in human cells.

These findings are useful for the development of OAS3-based prophylaxis and therapy against positive-sense ssRNA viruses of major medical importance including CHIKV, WNV and DV. They are also useful for the development of new *OAS3*-based molecular tools for the prediction of human susceptibility to the infection with alphaviruses, flaviviruses and other positive-sense ssRNA viruses of major medical importance, and in particular for the prediction of severe forms of disease in dengue, West Nile and Chikungunya patients.

A subject of the invention is an isolated 2',5'-oligoadenylate synthetase 3 protein or an isolated polynucleotide encoding said 2'-5'-oligoadenylate synthetase 3 protein, as a medicament.

### Definitions

- "polynucleotide" refers to a genomic DNA fragment, a cDNA fragment or an RNA molecule.
- "2'-5'-oligoadenylate synthetase 3", "OAS3", "OAS 3", "2'-5'-oligoadenylate synthetase 3 (100 kD)", (2-5')oligo(A)synthetase 3", "oligoadenylate synthetase p100", "p100 OAS" or "p1000AS" refers to a protein which is encoded by the *OAS3* gene of a mammal and has 2'-5'-oligoadenylate synthetase activity, and to the derived variants, including natural variants resulting from polymorphism in the *OAS3* gene and artificial variants resulting from mutation (insertion, deletion, substitution) of one or more nucleotides in the *OAS3* gene/open reading frame (ORF) sequences, providing that the variant has 2'-5'-oligoadenylate synthetase activity and is capable of inhibiting positive-sense single-stranded RNA virus replication. Preferably, the variant comprises three OAS domains. The *OAS3* gene and the deduced OAS3 ORF and amino acid sequence of various mammals are available in sequence databases and other OAS3 gene/ORF sequences may be determined by standard cloning and sequencing techniques which are known by one skilled in the art.
- "human *OAS3* gene" is a 34806 bp sequence corresponding to positions 111860632 to 111895437 on GenBank sequence accession number NC_000012. The human *OAS3* gene is located on chromosome 12 (12q24.2) and comprises 16 Exons: Exon 1: 1 to 264 ; Exon 2 :3127 to 3409 ; Exon 3 : 6033 to 6208 ; Exon 4 : 8300 to 8538 ; Exon 5 : 9503 to 9656 ; Exon 6 : 10418 to 10762 ; Exon 7 : 12250 to 12532 ; Exon 8 : 22628 to 22803 ; Exon 9 : 24209 to 24459 ; Exon 10 : 24870 to 25014 ; Exon 11 : 25792 to 25965 ; Exon 12 : 27301 to 27586 ; Exon 13 : 28975 to 29150 ; Exon 14 : 29493 to 29731 ; Exon 15 : 31165 to 31312 ; Exon 16 : 31519 to 34806.
- "human OAS3 open reading frame (ORF)" is the sequence SEQ ID NO: 1 which corresponds to positions 88 to 3351 on the 6646 bp human cDNA sequence GenBank accession number: NM_006187 (SEQ ID NO: 27). The positions of Exons 1 to 16 on the cDNA sequence NM_006187 are : 1 to 264, 265 to 547, 548 to 723, 724 to 962, 963 to 1116, 1117 to 1461, 1462 to 1744, 1745 to 1920, 1921 to 2171, 2172 to 2316, 2317 to 2490, 2491 to 2776, 2777 to 2952, 2953 to 3191, 3192 to 3339, 3340 to 6627, respectively.
- "human OAS3 protein" is a 1087 amino acid sequence corresponding to GenBank accession number NP_006178 or SEQ ID NO: 2 ; the three OAS domains correspond to positions 6 to 343 (SEQ ID NO : 3), 411 to 742 (SEQ ID NO : 4) and 750 to 1084 (SEQ ID NO : 5), respectively.
- "OAS3 activity" refers to both 2'-5'-oligoadenylate synthetase and positive-sense single-stranded RNA virus replication inhibition activities.

The 2',5'-oligoadenylate synthetase activity of the OAS3 protein of the invention may be assayed by chromatographic or electrophoretic methods to determine the end-point amounts of oligoadenylates formed (St Laurent et al., Cell, 1983, 33, 95-102; Johnston et al., In : Interferon 3: Mechanisms of Production and Action, 1984, 189-298, Friedman, R.M, Ed, Elsevier, Amsterd*am;* Justesen et al., Proc. Natl. Acad. Sci., USA, 1980, 77, 4618-4622; Justesen et al., Nucleic Acids Res., 1980, 8, 3073-3085; Justesen, J. and Kjelgaard, N.O., Anal. Biochem., 1992, 207, 90-93).
- "inhibition of positive-sense ssRNA virus replication by the OAS3 protein of the invention" refers to the partial or total reduction of virus growth (virus multiplication) when exogenous OAS3 protein (not encoded by the genome of the cells; recombinant OAS3 protein, for example) is present in the virus-infected cells. This inhibition may be determined by infecting an appropriate recombinant cell line expressing the OAS3 protein with a positive-sense single-stranded RNA virus. Non-recombinant cells of the same type infected with the virus are used as control. Then, progeny virus production in the supernatant of the virus-infected cells may be measured by any well-known virus titration assay. Alternatively, viral proteins production may be analyzed by Western-Blot or Immunolabeling of viral antigens or viral genomic and subgenomic RNAs production may be analyzed by Northern-Blot or RT-PCR.
- "positive-sense ssRNA virus" refers to a virus that has positive-sense single-stranded ribonucleic acid (ssRNA) as its genetic material and does not replicate using a DNA intermediate. Positive-sense ssRNA viruses belong to *Group IV* of the Baltimore classification system of classifying viruses.
- "identity" with respect to both amino acid sequences and nucleic acid sequences, refers to a measure of the degree of identity of two sequences based upon alignment of the sequences which maximizes identity between aligned amino acid residues or nucleotides, an which is a function of the number of identical residues or nucleotides, the number of total residues (1087 residues in the case of the present invention) or nucleotides (3261 nucleotides in the case of the present invention) , and the presence and length or gaps in the sequence alignment. Various alignment algorithms and/or computer programs are available for determining sequence identity using standard parameters, including FASTA, or BLAST which are available as a part of the GCG sequence analysis package (University of Wisconsin, Madison, Wis.), and can be used with, e.g., default settings.

A protein having 75 % identity with residues 1 to 1087 of SEQ ID NO: 2 is a protein whose sequence may include up to (25 x 10.87 = 271) changes when the protein sequence is aligned and compared to residues 1 to 1087 of with SEQ ID NO: 2. One change refers to the deletion, substitution or insertion of one amino acid as compared to residues 1 to 1087 of SEQ ID NO: 2. For example, a 1080 amino acid sequences having 50 changes with the first 1080 residues of SEQ ID NO: 2 has 1080-50/10.87= 94.75 % identity with SEQ ID NO: 2. For example, a 1090 amino acid sequences having 50 changes with residues 1 to 1087 of SEQ ID NO: 2 has 1087-50/10.87= 95.4 % identity with SEQ ID NO: 2.
- "similarity" refers to a measure of the degree of similarity of two amino acid sequences based upon alignment of the sequences which maximizes similarity between aligned amino acid residues, and which is a function of the number of identical or similar residues, the number of total residues (1087 residues in the case of the present invention), and the presence and length or gaps in the sequence alignment. Various alignment algorithms and/or computer programs are available for determining sequence similarity using standard parameters, including FASTA, or BLAST which are available as a part of the GCG sequence analysis package (University of Wisconsin, Madison, Wis.), and can be used with, e.g., default settings. Similar residues refer to residues having comparable chemical properties (size, charge (neutral, basic, acidic), hydrophilicity/hydrophobicity).
- "individual" includes mammals, as well as other vertebrates (e.g., birds, fish and reptiles). The terms "mammal" and "mammalian", as used herein, refer to any vertebrate animal, including monotremes, marsupials and placental, that suckle their young and either give birth to living young (eutharian or placental mammals) or are egg-laying (metatharian or nonplacental mammals). Examples of mammalian species include humans and other primates (e.g., monkeys, chimpanzees), rodents (e.g., rats, mice, guinea pigs) and others such as for example: cows, pigs and horses.
- by "mutation" is intended the substitution, deletion, insertion of one or more nucleotides/amino acids in a polynucleotide (cDNA, gene) or a polypeptide sequence. Said mutation can affect the coding sequence of a gene or its regulatory sequence. It may also affect the structure of the genomic sequence or the structure/stability of the encoded mRNA.

The invention encompasses modified OAS3 protein including one or more modifications selected from the group consisting of : the mutation (insertion, deletion, substitution) of one or more amino acids in the OAS3 amino acid sequence, the addition of an amino acid fusion moiety, the substitution of amino acid residues by non-natural amino acids (D-amino-acids or non-amino acid analogs), the modification of the peptide bond, the cyclization, the addition of chemical groups to the side chains (lipids, oligo-or -polysaccharides), and the coupling to an appropriate carrier. These modifications which are introduced by procedures well-known in the art, result in a modified OAS3 protein which is still active for 2'-5'-oligoadenylate synthetase and inhibition of positive-sense single-stranded RNA virus replication activities.

According to a preferred embodiment of the invention, said 2'-5'-aligoadenylate synthetase 3 (OAS3) is human 2'-5'-oligoadenylate synthetase 3.

According to another preferred embodiment of the invention, said OAS3 protein has at least 70 % amino acid sequence identity or 80 % amino acid sequence similarity, preferably at least 80 % amino acid sequence identity or 90 % amino acid sequence similarity to residues 1 to 1087 of SEQ ID NO: 2.

According to a more preferred embodiment of the invention, said OAS3 protein comprises a Serine at position 381 of SEQ ID NO: 2. Preferably, said OAS3 protein comprises or consists of an amino acid sequence selected in the group consisting of: SEQ ID NO: 2 and SEQ ID NO: 7.

According to another preferred embodiment of the invention, said OAS3 polynucleotide is coding for a protein as defined above, more preferably it comprises or consists of a nucleotide sequence selected in the group consisting of: SEQ ID NO: 1 which encodes the protein SEQ ID NO: 2 and SEQ ID NO: 6 which encodes the protein SEQ ID NO: 7.

According to another preferred embodiment of the invention, said polynucleotide is inserted in an expression vector.

A vector refers to a nucleic acid molecule capable of transporting another nucleic acid to which it has been linked. A vector which can be used in the present invention includes, but is not limited to, a viral vector, a plasmid, a RNA vector or a linear or circular DNA or RNA molecule which may consists of a chromosomal, non chromosomal, semi-synthetic or synthetic nucleic acids. Preferred vectors are those capable of autonomous replication (episomal vector) and/or expression of nucleic acids to which they are linked (expression vectors). Large numbers of suitable vectors are known to those of skill in the art and commercially available.

Viral vectors include retrovirus, adenovirus, parvovirus (e. g. adeno-associated viruses or AAVs), coronavirus, negative strand RNA viruses such as ortho-myxovirus (e. g., influenza virus), rhabdovirus (e. g., rabies and vesicular stomatitis virus), paramyxovirus (e. g. measles and Sendai), positive strand RNA viruses such as picornavirus and alphavirus, and double-stranded DNA viruses including adenovirus, herpesvirus (e. g., Herpes Simplex virus types 1 and 2, Epstein-Barr virus, cytomegalovirus), and poxvirus (e. g., vaccinia, fowlpox and canarypox). Other viruses include Norwalk virus, togavirus, flavivirus, reoviruses, papovavirus, hepadnavirus, and hepatitis virus, for example. Examples of retroviruses include: avian leukosis-sarcoma, mammalian C-type, B-type viruses, D type viruses, HTLV-BLV group, lentivirus, spumavirus (Coffin, J. M., Retroviridae: The viruses and their replication, In Fundamental Virology, Third Edition, B. N. Fields, et al., Eds., Lippincott-Raven Publishers, Philadelphia, 1996).

Preferably said vectors are expression vectors, wherein the sequence encoding the OAS3 protein of the invention is placed under control of appropriate transcriptional and translational control elements to permit production or synthesis of said protein. Therefore, said polynucleotide is comprised in an expression cassette. More particularly, the vector comprises a replication origin, a promoter operatively linked to said encoding polynucleotide, a ribosome-binding site, an RNA-splicing site (when genomic DNA is used), a polyadenylation site and a transcription termination site. It also can comprise an enhancer. Selection of the promoter will depend upon the cell in which the polypeptide is expressed. Suitable promoters include tissue specific and/or inducible promoters. Examples of inducible promoters are: eukaryotic metallothionine promoter which is induced by increased levels of heavy metals, heat shock promoter which is induced by increased temperature. Examples of tissue specific promoters are skeletal muscle creatine kinase, prostate-specific antigen (PSA), α-antitrypsin protease, human surfactant (SP) A and B proteins and β-casein. Vectors can comprise selectable markers, for example: neomycin phosphotransferase, histidinol dehydrogenase, dihydrofolate reductase, hygromycin phosphotransferase, herpes simplex virus thymidine kinase, adenosine deaminase, glutamine synthetase, and hypoxanthine-guanine phosphoribosyl transferase for eukaryotic cell culture; *TRP1, URA3* and *LEU2* for *S. cerevisiae;* tetracycline, rifampicin or ampicillin resistance in *E. coli.*

The choice of the vector depends on their use (stable or transient expression) or and on the host cell; viral vectors and "naked"nucleic acid vectors are preferred vectors for expression in mammal cells (human and animal). Use may be made, inter alia, of viral vectors such as adenoviruses, retroviruses, lentiviruses and AAVs, into which the sequence of interest has been inserted beforehand.

The subject-matter of the present invention is also a pharmaceutical composition characterized in that it comprises at least one OAS3 protein or one OAS3 polynucleotide, preferably inserted in an expression vector, as defined above, and at least one acceptable vehicle, carrier, additive and/or immunostimulating agent.

Any suitable carrier known to those of ordinary skill in the art may be employed in the pharmaceutical composition of the present invention, the type of carrier varying depending on the mode of administration. For parenteral administration, such as subcutaneous injection, the carrier preferably comprises water, saline buffer, lactose, mannitol, glutamate, a fat or a wax and the injectable pharmaceutical composition is preferably an isotonic solution (around 300-320 mosmoles). For oral administration, any of the above carriers or a solid carrier, such as mannitol, lactose, starch, magnesium stearate, sodium saccharine, talcum, cellulose, glucose, sucrose, and magnesium carbonate, may be employed. Biodegradable microspheres (e.g. polylactic galactide) may also be employed as carriers for the pharmaceutical compositions of this invention. Suitable biodegradable microspheres are disclosed, for example in US Patent 4,897,268 and 5,075,109. The additive may be chosen among antiaggregating agents, antioxidants, dyes, flavor enhancers, or smoothing, assembling or isolating agents, and in general among any excipient conventionally used in the pharmaceutical industry. Any of the variety of immunostimulating agent may be employed in the compositions of the present invention to enhance the immune response.

The pharmaceutical composition may be in a form suitable for oral administration. For example, the composition is in the form of tablets, ordinary capsules, gelatine capsules or syrup for oral administration. These gelatine capsules, ordinary capsules and tablet forms can contain excipients conventionally used in pharmaceutical formulation, such as adjuvants or binders like starches, gums and gelatine, adjuvants like calcium phosphate, disintegrating agents like cornstarch or algenic acids, a lubricant like magnesium stearate, sweeteners or flavourings. Solutions or suspensions can be prepared in aqueous or non-aqueous media by the addition of pharmacologically compatible solvents. These include glycols, polyglycols, propylene glycols, polyglycol ether, DMSO and ethanol.

The OAS3 protein or the OAS3 polynucleotide (isolated or inserted in a vector) are introduced into cells, *in vitro, ex vivo* or *in vivo,* by any convenient means well-known to those in the art, which are appropriate for the particular cell type, alone or in association with at least either an appropriate vehicle and/or carrier. For example, the OAS3 protein/polynucleotide may be associated with a substance capable of providing protection for said sequences in the organism or allowing it to cross the host-cell membrane. The OAS3 protein may be advantageously associated with liposomes, polyethyleneimine (PEI), and/or membrane translocating peptides (Bonetta, The Scientist, 2002, 16, 38; Ford et al., Gene Ther., 2001, 8, 1-4 ; Wadia and Dowdy, Curr. Opin. Biotechnol., 2002, 13, 52-56; Langel, U. In Handbook of cell penetrating peptides (2nd Ed.), 2006, Lavoisier, FRANCE); in the latter case, the sequence of the OAS3 protein is fused with the sequence of a membrane translocating peptide (fusion protein). Polynucleotide encoding OAS3 (isolated or inserted in a vector) may be introduced into a cell by a variety of methods (e.g., injection, direct uptake, projectile bombardment, liposomes, electroporation). OAS3 protein can be stably or transiently expressed into cells using appropriate expression vectors as defined above.

In one embodiment of the present invention, the OAS3 protein/polynucleotide is substantially non-immunogenic, i.e., engenders little or no adverse immunological response. A variety of methods for ameliorating or eliminating deleterious immunological reactions of this sort can be used in accordance with the invention. In a preferred embodiment, the OAS3 protein is substantially free of N-formyl methionine. Another way to avoid unwanted immunological reactions is to conjugate protein/polynucleotide to polyethylene glycol ("PEG") or polypropylene glycol ("PPG") (preferably of 500 to 20,000 daltons average molecular weight (MW)).

Another subject of the present invention is an OAS3 protein or a polynucleotide coding for said OAS3 protein as defined above for preventing or treating an infection with a positive-sense single-stranded RNA virus.

According to a more preferred embodiment, said virus is of the *Alphavirus* genus. More preferably, it is selected from the group consisting of: Chikungunya (CHIK), Sindbis (SIN), Semliki Forest (SF), Eastern Equine Encephalitis (EEE), Western Equine Encephalitis (WEE), Venezuelan Equine Encephalitis (VEE), Ross River (RR), O'Nyong Nyong (ONN) and Barma Forest (BF) viruses.

According to another more preferred embodiment, said virus is of the *Flavivirus* genus. More preferably, said virus is selected from the group consisting of: Dengue, Japanese Encephalitis, Kyasanur Forest Disease, Murray Valley Encephalitis, St. Louis Encephalitis, Tick-Borne Encephalitis, West Nile, Yellow Fever and Omsk hemorrhagic fever (OHF) virus.

According to another more preferred embodiment, said virus is of the *Hepacivirus* genus. More preferably, said virus is the Hepatitis C virus.

The subject-matter of the present invention is also products containing at least an OAS3 protein or an OAS3 polynucleotide, preferably inserted in an expression vector, as defined above and a second product which is different from the first one, said second product being selected from the group consisting of: antiviral, anti-inflammatory and immunomodulatory drugs, as a combined preparation for simultaneous, separate or sequential use in the prevention or the treatment of a positive-sense single-stranded RNA virus infection.

The subject-matter of the present invention is also a method for preventing or curing a positive-sense single-stranded RNA virus infection in an individual in need thereof, said method comprising the step of administering to said individual a composition as defined above, by any means.

In general, the composition may be administered by parenteral injection (e.g., intradermal, intramuscular, intravenous or subcutaneous), intranasally (e.g. by aspiration or nebulization), orally, sublingually, or topically, through the skin or through the rectum.

The amount of OAS3 (protein/polypeptide) present in the composition of the present invention is a therapeutically effective amount. A therapeutically effective amount of OAS3 (protein/polypeptide) is that amount necessary so that OAS3 protein performs its role of inhibiting positive-sense single-stranded RNA virus replication without causing, overly negative effects in the subject to which the composition is administered. The exact amount of OAS3 (protein/polypeptide) to be used and the composition to be administered will vary according to factors such as the positive-sense single-stranded RNA virus species and the individual species (human, animal) being treated, the mode of administration, the frequency of administration as well as the other ingredients in the composition.

Preferably, the composition is composed of from about 10 µg to about 10 mg and more preferably from about 100 µg to about 1 mg, of OAS3 (protein/polypeptide). By "about", it is meant that the value of said quantity (µg or mg) of OAS3 can vary within a certain range depending on the margin of error of the method used to evaluate such quantity.

For instance, during an oral administration of the composition of the invention, individual to be treated could be subjected to a 1 dose schedule of from about 10 *g to about 10 mg of OAS3 (protein/polypeptide) per day during 3 consecutive days. The treatment may be repeated once one week later.

For parenteral administration, such as subcutaneous injection, the individual to be treated could be subjected to a 1 dose of from about 10 µg to about 10 mg and more preferably from about 100 µg to about 1 mg, of OAS3 (protein/polypeptide). The treatment may be repeated once one week later.

A subject of the invention is also a *method in vitro* for evaluating the susceptibility of an individual to an infection with a positive-sense single-stranded RNA virus as defined above, comprising: the detection of a polymorphism in the *OAS3* gene in a nucleic acid sample obtained from said individual.

The nucleic acid sample may be genomic DNA, total mRNA or cDNA.

The polymorphism is detected by any method known in the art that allows the detection of mutation in nucleic acid sequences as those described for example *In* Current Protocols in Human Genetics, 2008, John Wiley & Sons, Inc. Examples of genotyping assays include with no limitation: RAPD, RFLP, AFLP, sequence specific oligonucleotide hybridization, SnapShot PCR, Ligase detection reaction, PCR and Maldi-TOF, Pyrosequencing. This assay may use the OAS3 specific primers of Table II and III and in particular the primers SEQ ID NO: 11, 12, 62 to 86 and 118 to 142.

According to a preferred embodiment of said method, said positive-sense single-stranded RNA virus is an alphavirus such as Chikungunya virus or a flavivirus such as Dengue virus.

According to another preferred embodiment of said method, said polymorphism is the mutation of the Arg844 codon to a stop codon (R844X); said polymorphism detected in the Caucasian population, is associated with an increased susceptibility to positive-sense single-stranded RNA virus infection, particularly Chikungunya virus infection. The R844X mutation may be detected by PCR-RFLP using the pair of primers (SEQ ID NO: 11 and SEQ ID NO: 12), followed by digestion of the 183 bp PCR product with *Bgl*II; the presence of two fragments of 115 bp and 68 bp indicates the presence of OAS3.R844X mutation.

According to another preferred embodiment of said method, said polymorphism is a single nucleotide polymorphism (SNP) at the third position of codon 381. Preferably, said SNP is a G to C substitution of codon AGG-381 that changes amino acid from Arg to Ser (R381S). Genetic data suggest that variant Ser-381 is associated with a dominant protection against the risk of DSS in dengue patients (Table VI). This SNP may be detected by any appropriate genotyping assay as defined above. For example, this assay may comprise the direct sequencing of genomic DNA amplified with a pair of *OAS3* specific primers such as the pair of PCR primers specific to *OAS3* exon 6 that is presented in Table III (SEQ ID NO: 70 and 126).

A subject of the invention is also an isolated OAS3 protein comprising or consisting of the sequence SEQ ID NO: 7 or SEQ ID NO: 30.

A subject of the invention is also an isolated OAS3 protein fragment comprising or consisting of the sequence SEQ ID NO: 9; this OAS3 fragment which includes residues 1 to 843 of SEQ ID NO: 7 comprises the first and the second OAS domains of OAS3 but lacks most of the third (C-terminal) OAS domain.

A subject of the invention is also:
- an isolated polynucleotide comprising or consisting of the sequence SEQ ID NO: 6, which encodes the OAS3 protein of SEQ ID NO: 7,
- an isolated polynucleotide comprising or consisting of the sequence SEQ ID NO: 8, which encodes the OAS3 protein fragment of SEQ ID NO: 9,
- an isolated polynucleotide comprising or consisting of the sequence SEQ ID NO: 28 or 29, which encodes the OAS3 protein variant SEQ ID NO: 30.

A subject of the invention is also a recombinant vector, preferably an expression vector comprising a polynucleotide having a sequence selected in the group consisting of : SEQ ID NO: 6, SEQ ID NO: 8, SEQ ID NO: 28 and SEQ ID NO: 29.

A subject of the invention is also a host cell transfected or transformed by a polynucleotide comprising or consisting of a sequence selected in the group consisting of: SEQ ID NO: 6, SEQ ID NO: 8, SEQ ID NO: 28 and SEQ ID NO: 29.

According to a preferred embodiment of the invention, it is an HeLaTet-Off cell line expressing a recombinant human OAS3, named HeLa-Tet-Off/OAS3#C417-1, deposited February 26, 2008, at the Collection Nationale de Cultures de Microorganismes, 25 rue du Docteur Roux, 75724 Paris Cedex 15, under the accession number I-3927.

According to another preferred embodiment of the invention, it is an α-Tet-Off cell line expressing a truncated recombinant human OAS3, named HeLaTet-Off/OAS3/delta/1C, deposited April 17, 2008, at the Collection Nationale de Cultures de Microorganismes, 25 rue du Docteur Roux, 75724 Paris Cedex 15, under the accession number I-3968.

According to a preferred embodiment of the invention, it is an HepG2-Tet-Off cell line expressing a recombinant human OAS3, named HeLa-Tet-Off/OAS3#F8, deposited May 15 2009, at the Collection Nationale de Cultures de Microorganismes, 25 rue du Docteur Roux, 75724 Paris Cedex 15, under the accession number I-4158.

These cell lines which are derived from HeLa or HepG2 cells which can be infected with various viruses are useful for assaying the susceptibility of a virus to OAS3 mediated antiviral activity.

A subject of the invention is also a non-human transgenic animal comprising a polynucleotide as defined above.

A subject of the invention is also a transgenic plant comprising a polynucleotide as defined above.

The OAS3 protein/polynucleotide of the invention are prepared using well-known recombinant DNA and genetic engineering techniques. For example, a sequence comprising the OAS3 ORF is amplified from a DNA template, by polymerase chain reaction with specific primers. The PCR fragment is then cloned in an expression vector by using appropriate restriction sites. The OAS3 protein is expressed in a host cell or a transgenic animal/plant modified by the expression vector, under conditions suitable for the expression of the OAS3 protein, and the OAS3 protein is recovered from the host cell culture or from the transgenic animal/plant.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory); Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); and Manipulating the Mouse Embryo, (Cold Spring Harbor Laboratory Press, Cold Spring Harbor, N.Y., 1986). Current Protocols in Human Genetics (John Wiley & Sons, Inc, 2008), specifically Chapter 12 "Vectors For Gene Therapy" and Chapter 13 "Delivery Systems for Gene Therapy").

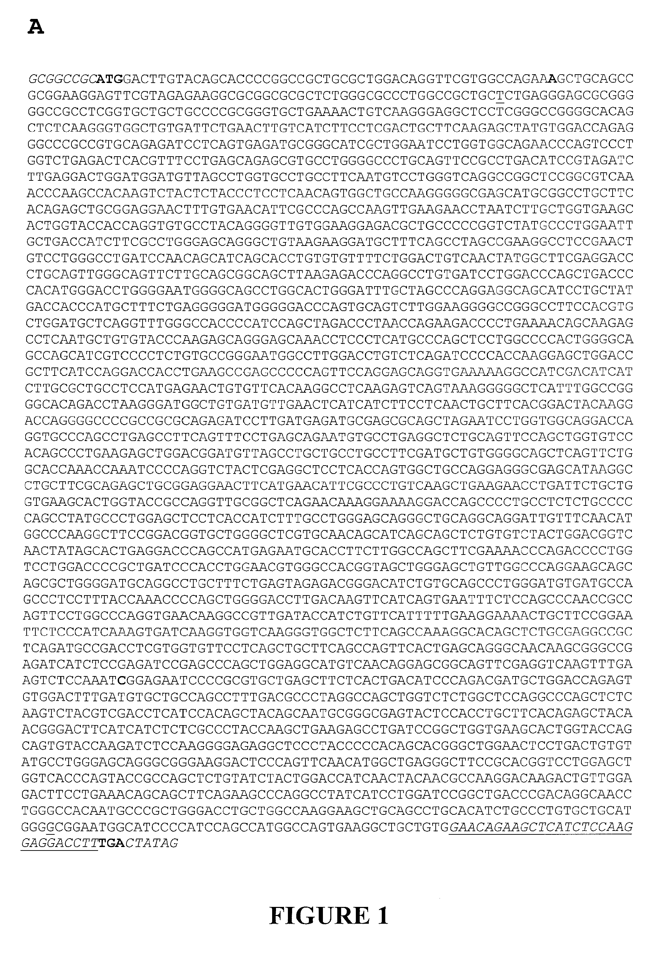

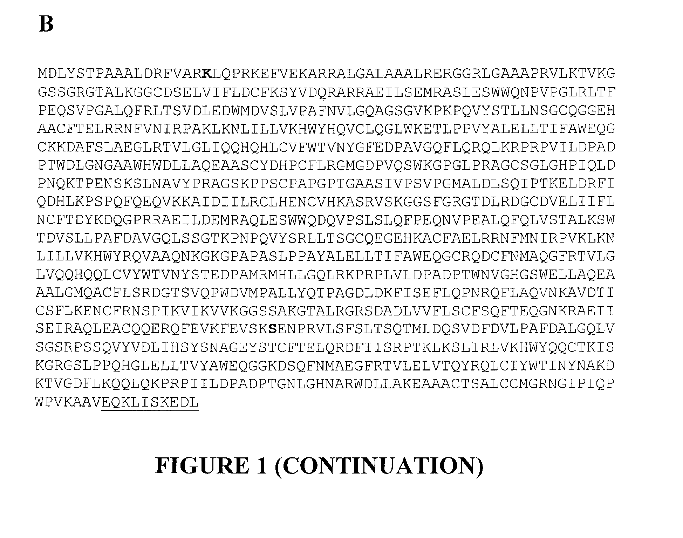

- figure 1 represents the nucleotide (A) and amino acid (B) sequences of human recombinant OAS3 (clone OAS3C 17.1) tagged with a c-myc epitope. These nucleotide and amino acid sequences correspond to SEQ ID NO: 23 and 24, respectively. Changes between OAS3 mRNA reference sequence (SEQ ID NO: 27) and cloned OAS3 cDNA are underlined if synonymous or shown in bold if non synonymous. The translation initiation and stop codons are in bold. The additional nucleotide and amino acid sequences corresponding to the C_terminal c-myc epitope are underlined. The 5' *Not*I and 3' *EcoRV* restriction sites are shown in italics.

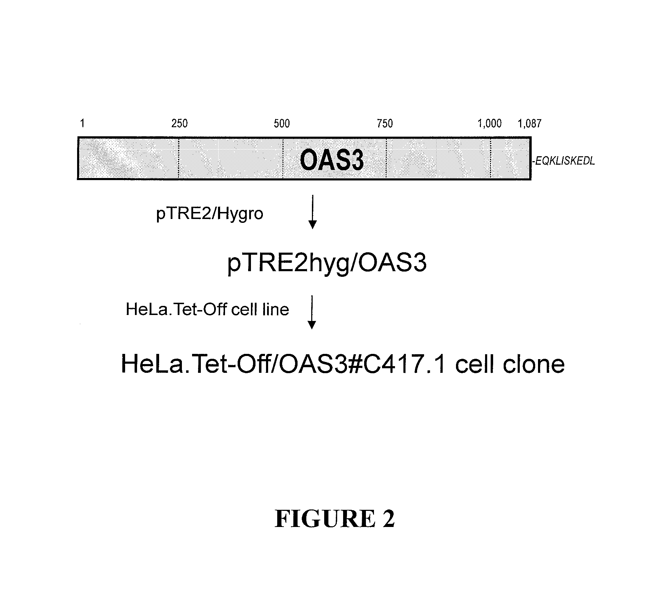

- figure 2 illustrates the establishment of an inducible HeLa-Tet-Off/OAS3#C417-1 cell line expressing recombinant human OAS3.

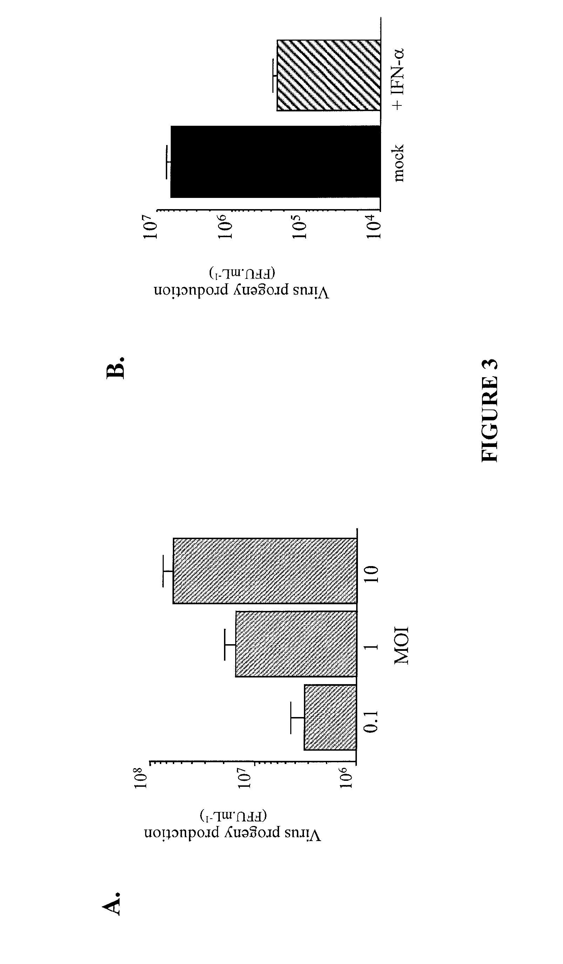

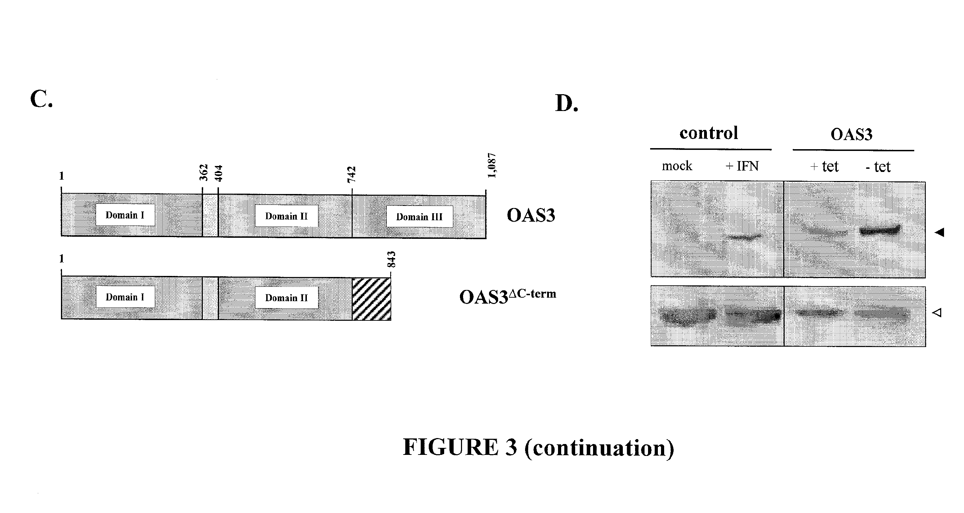

- figure 3 illustrates the detection of OAS3 in HeLa cells. *In* (A), HeLa.Tet-Off cells were infected with different Focus Forming Units (AP61FFU) per cell of CHIK virus strain 06-49 (CHIKV 06-49). At 20 h post-infection (p.i.), virus particles produced in the supernatants of infected cells were titered on mosquito *Aedes pseudoscutellaris* AP61 cells by focus immunoassay. In (B), HeLa.Tet-Off cells were treated with 1,000 IU/ml human IFN-α or mock-treated (control) 5 hours prior to CHIKV input at a multiplicity of infection of one AP61FFU per cell (1 MOI). Virus progeny productions were determined at 18 h p.i as described above. *In* (C), the graph depicts the structures of full-length and truncated form of OAS3. *In* (D), expression of ectopic OAS3 in induced (- Tet) or uninduced (+ Tet) Tet-Off/OAS3 cells was analyzed by immunoblot analysis using OAS3-specific antibody As controls, HeLa.Tet-Off cells were treated with 1,000 IU IFN-α (+ IFN) or mock-treated (mock). The ß-action served as a house-keeping protein control.

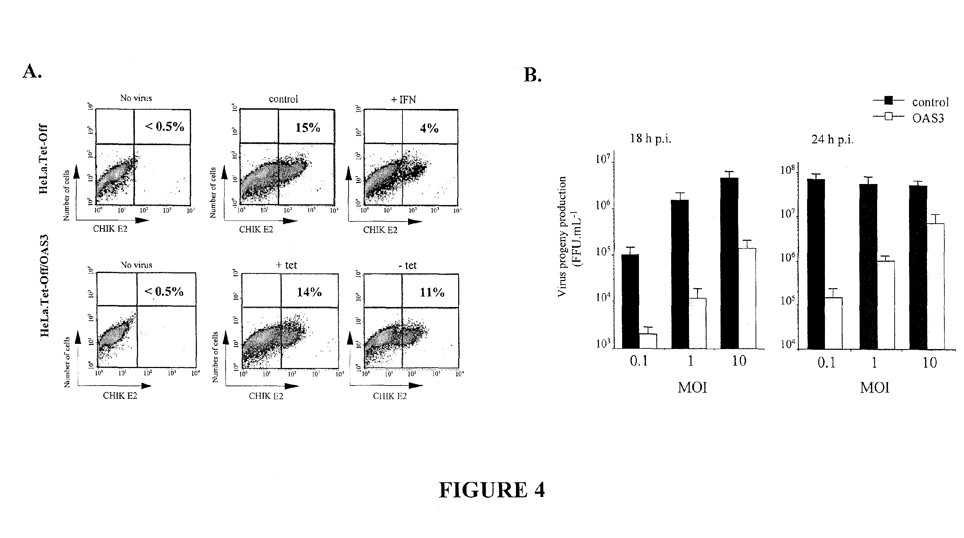

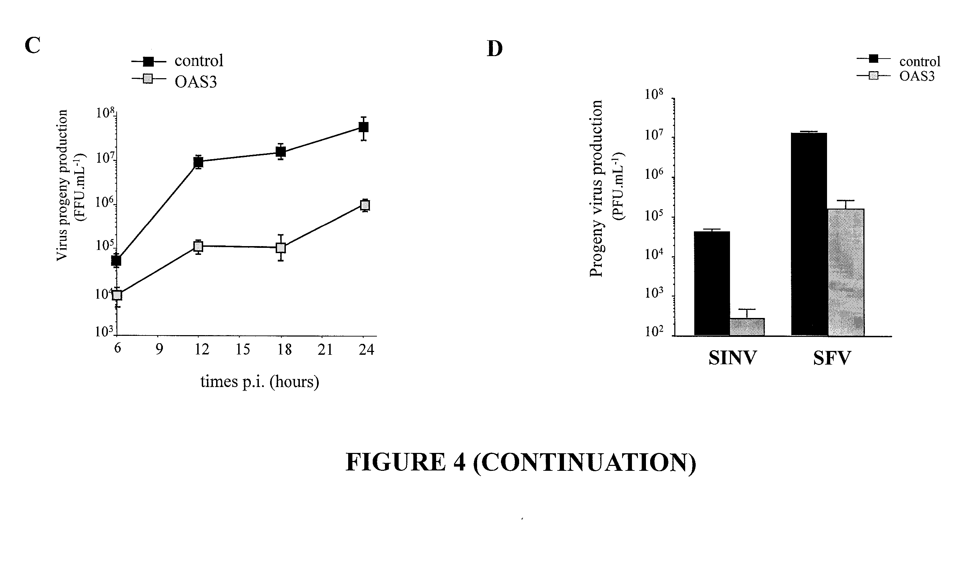

- figure 4 illustrates the inhibition of CHIKV growth in OAS3-expressing HeLa cells. *In* (A), cells were infected 18 h with CHIKV at 1 MOI and analyzed by flow cytometry using anti-CHIK.E2 MAb 3E4. Analysis of CHIKV E2 protein production in mock-infected cells (No virus), in CHIKV-infected HeLa.Tet-off cells incubated with1,000 IU/ml human IFN-α (+ IFN) 5 h prior virus input or mock-treated (control), and CHIKV-infected Tet-Off/OAS3 cells in the presence (+ tet) or in absence (- tet) of tetracycline. In (B), cells were infected with CHIKV at different MOI and virus progeny productions were determined at 18 and 24 h p.i. *In* (C), inhibition of CHIKV growth was monitored at various times p.i. *In* (D), cells were infected with SINV or SFV at 1 MOI and virus progeny productions were determined at 18 h p.i.

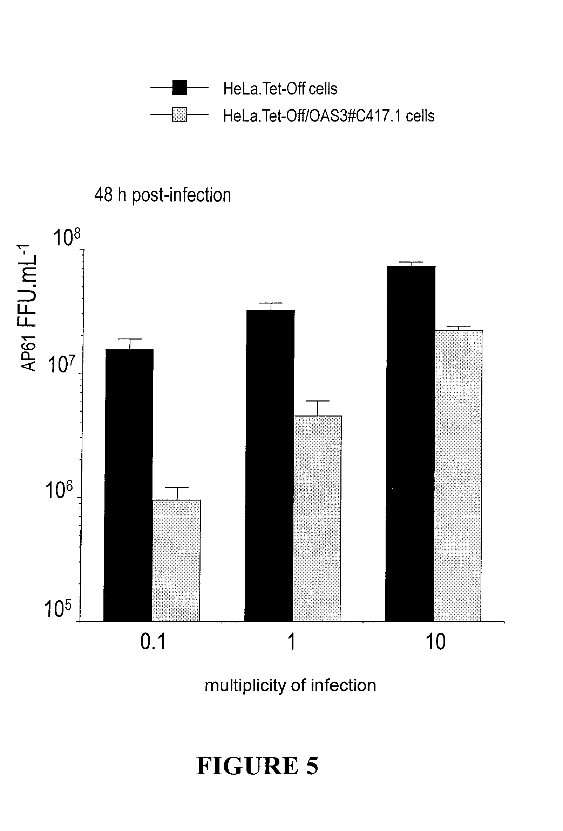

- figure 5 illustrates WN virus sensitivity to antiviral activities of OAS3. HeLa/Tet-off and induced HeLa.Tet-Off/OAS3#C417-1 cells were infected with 0.1, 1.0 or 10 AP61FFU per cell of WN virus strain IS-98-ST1. Infectious virus particles produced in supernatants of infected cells were titered at 48 hours post-infection by focus immunoassay on mosquito *Aedes pseudoscutellaris* AP61 cells.

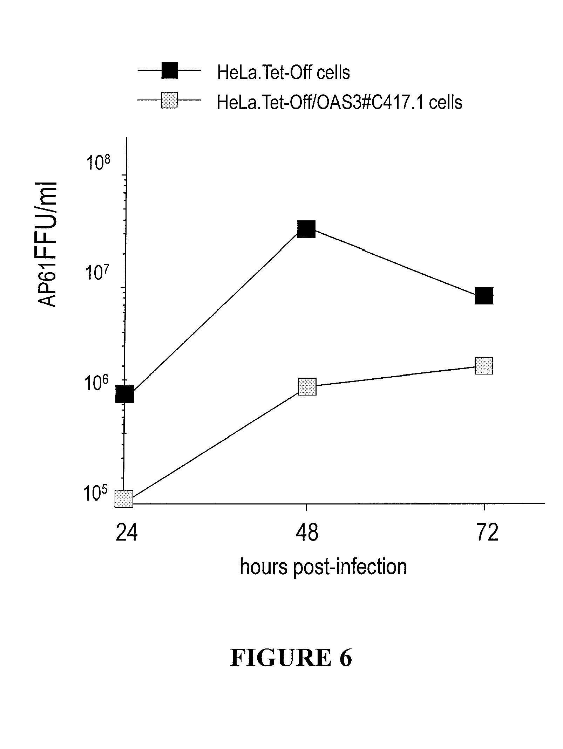

- figure 6 illustrates inhibition of WN virus growth in OAS3-expressing cells. HeLa/Tet-Off and induced HeLa.Tet-Off/OAS3#C417-1 cells were infected with 0.1 AP61FFU per cell of WN virus strain IS-98-ST1 and infectious virus particles produced in supernatants of infected cells were titered at various times post-infection (24, 48 and 72 hours) by focus immunoassay on mosquito *Aedes pseudoscutellaris* AP61 cells.

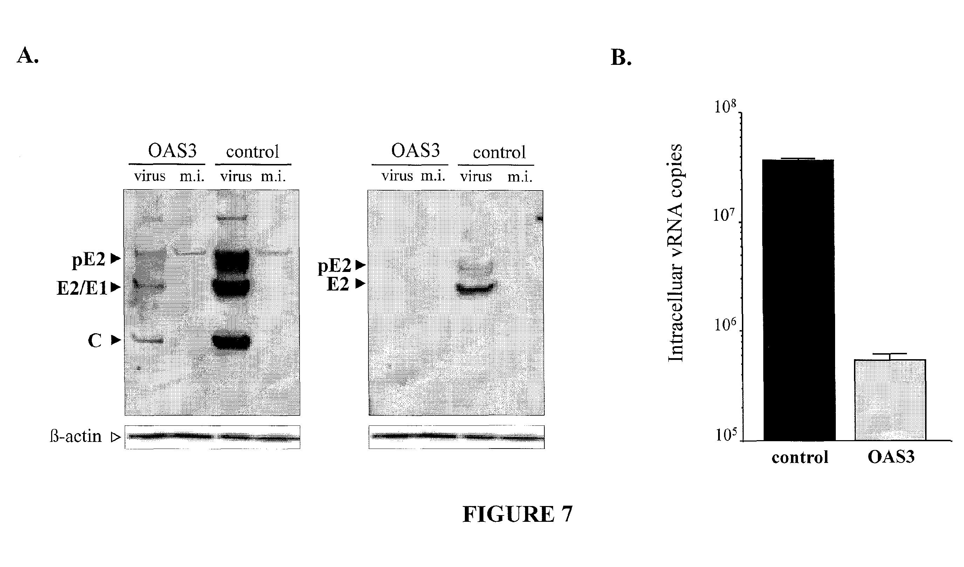

- figure 7 illustrates the consequences of OAS3 expression for CHIKV replication. In (A), immunoblot assay was performed on cell extracts from HeLa.Tet-off cells (control) and HeLa.Tet-Off/OAS3 (OAS3) cells infected with CHIKV (virus) or mock-infected (m.i.) using anti-CHIK HMAF (*left*) or anti-CHIKV E2 MAb 3E4 *(right).* The ß-actin served as protein control. *In* (B), real-time RT-PCR analysis of viral RNA production in HeLa.Tet-Off cells (control) and HeLa.Tet-Off/OAS3 cells (OAS3) infected with CHIKV at 1 MOI was performed at 8 h p.i.

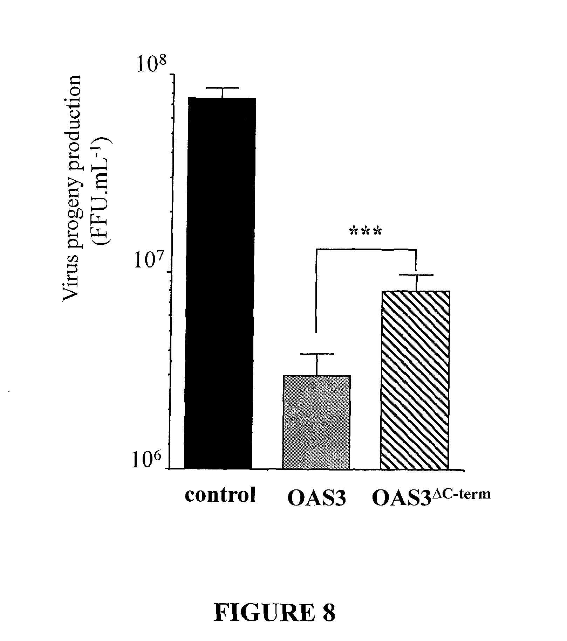

- figure 8 illustrates the anti-alphaviral activity of truncated form of OAS3. HeLa.Tet-off (control), HeLa.Tet-Off/OAS3 (OAS3) and HeLa.Tet-Off/OAS3/delta/1C (OAS3^{ΔC-term}) cells were infected with CHIKV at 1 MOI and virus progeny productions were determined at 18 h p.i. The values were compared statistically according to Student's *t* tests (*** : P < 0.001).

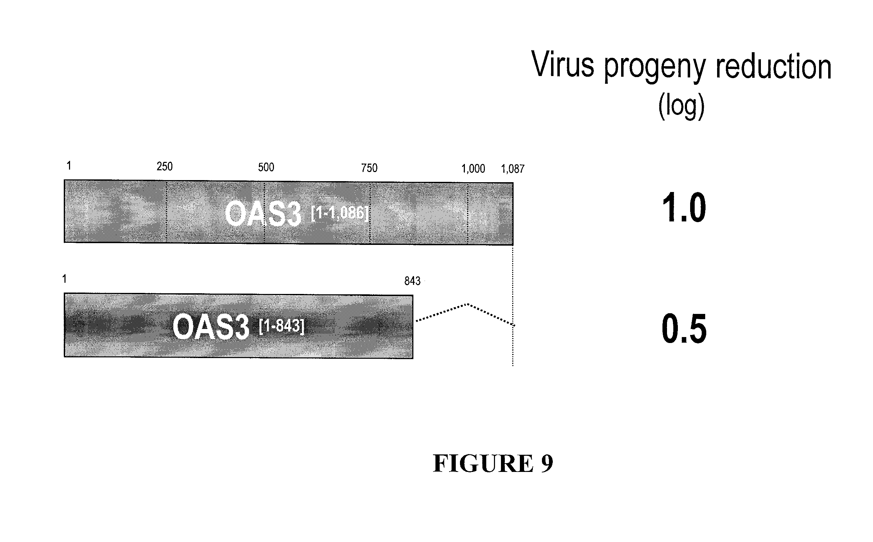

- figure 9 illustrates the anti-flaviviral activity of truncated form of OAS3. HeLa.Tet-off (control), HeLa.Tet-Off/OAS3 (OAS3) and HeLa.Tet-Off/OAS3/delta/1C (OAS3^{ΔC-term}) cells were infected with WNV at 1 MOI and virus progeny productions were determined at 18 h p.i. The values were compared statistically according to Student's *t* tests (*** : P < 0.001).

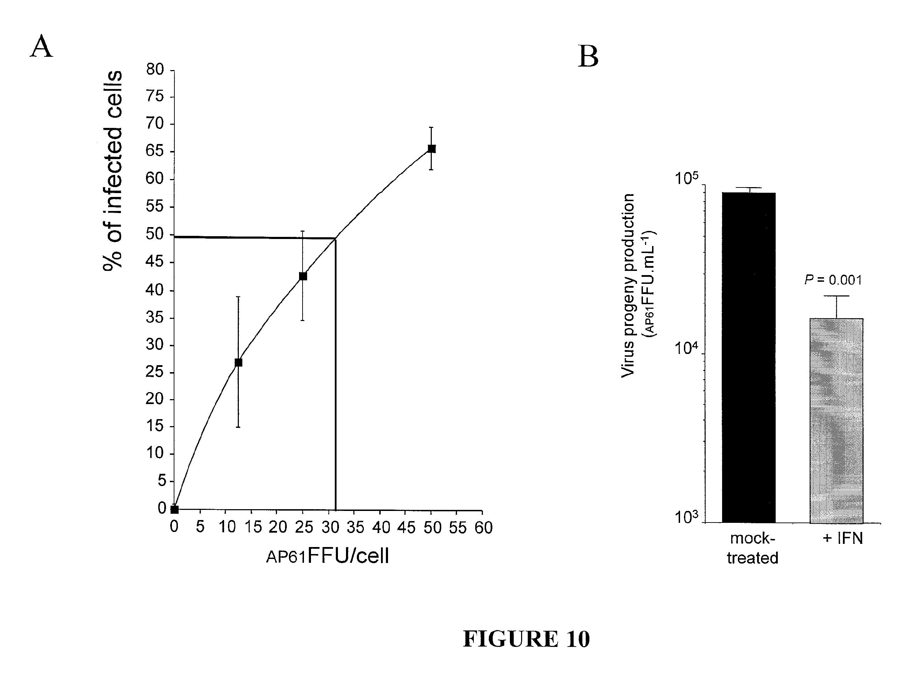

- figure 10 illustrates DV-1 virus sensitivity to Type-I IFN pathway. A: Susceptibility of HepG2.Tet-Off cell line to DV infection. Cells were infected with increasing input (AP61FFU/ per cell ) of DV-1 virus strain FGA/NA d1d. At 40 h post-infection, the cells were analyzed by flow cytometry using MAb 4E11 reactive to DV E glycoprotein. B: DV-1 virus sensitivity to Type-I IFN pathway. HepG2.Tet-off cells were pretreated with 1,000 IU.mL⁻¹ human IFN-α (+IFN) or not treated (mock-treated) 5 hours prior DV infection (10 MOI). At 40 h p.i., virus progeny production was determined as previously described (Duarte dos Santos et al., Virology, 2000, 274, 292-).

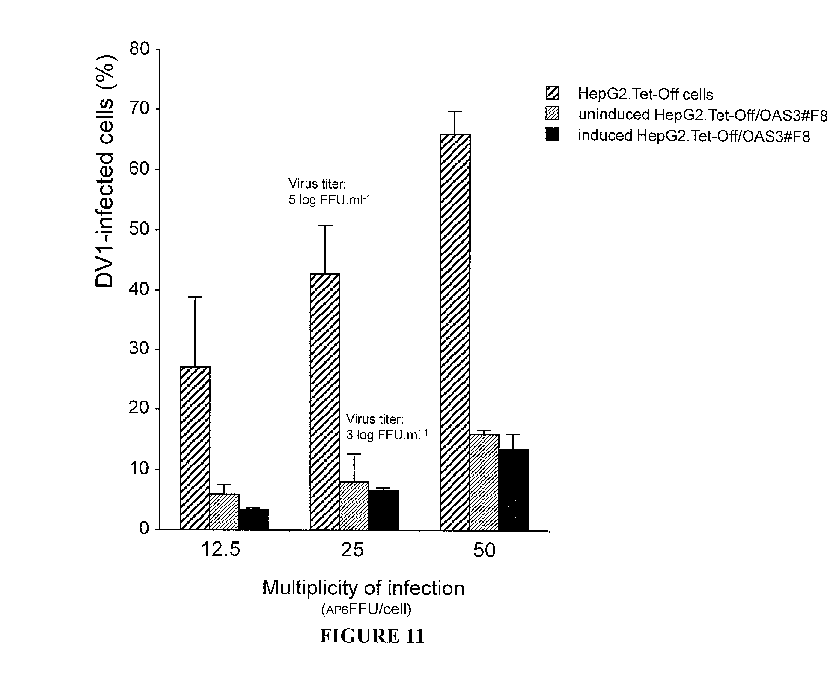

- figure 11: Inhibition of dengue virus growth in OAS3-expressing HepG2 cells. Parental HepG2.Tet-Off cells and HepG2.Tet-Off/OAS3#F8 cell clone in the presence (uninduced) or absence (induced) of 2 µg.mL-1 tetracycline were infected with dengue virus type-1 strain FGA/NA d1d at different multiplicities of infection. At 40 hours post-infection, cells were fixed with methanol/acetone at -20°C for 20 min. Immunofluorescence assay was performed using FITC-conjugated anti-dengue E mAb 4E1. Nuclei were stained with DAPI. The percentage of HepG2 cells positive for viral antigens was determined in a triplicate experiment. Virus progeny production was determined at the multiplicity of infection of 25. Virus titration was performed as previously described (Duarte dos Santos et al., Virology, 2000, 274, 292-).

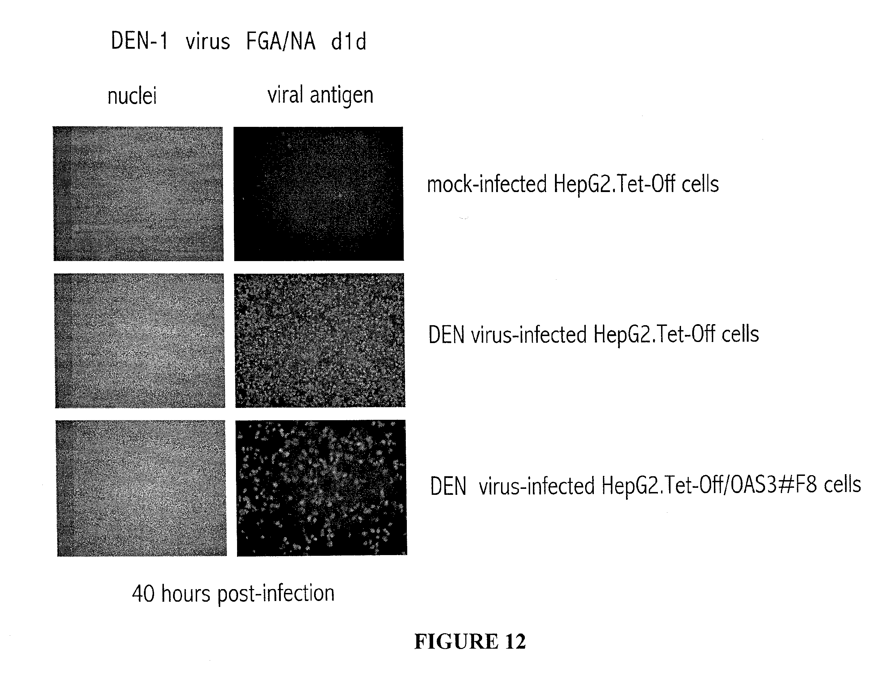

- figure 12: OAS3-expressing HepG2 cells show resistance to dengue virus infection. Parental HepG2.Tet-Off and induced HepG2.Tet-Off/OAS3#F8 cell clone were infected with dengue virus type-1 strain FGA/NA d1d (Duarte dos Santos et al., Virology, 2000, 274, 292-) at multiplicity of infection 30 AP61FFU/cell or mock-infected. At 40 hours post-infection, immunofluorescence assay was performed as described in figure legend 11.

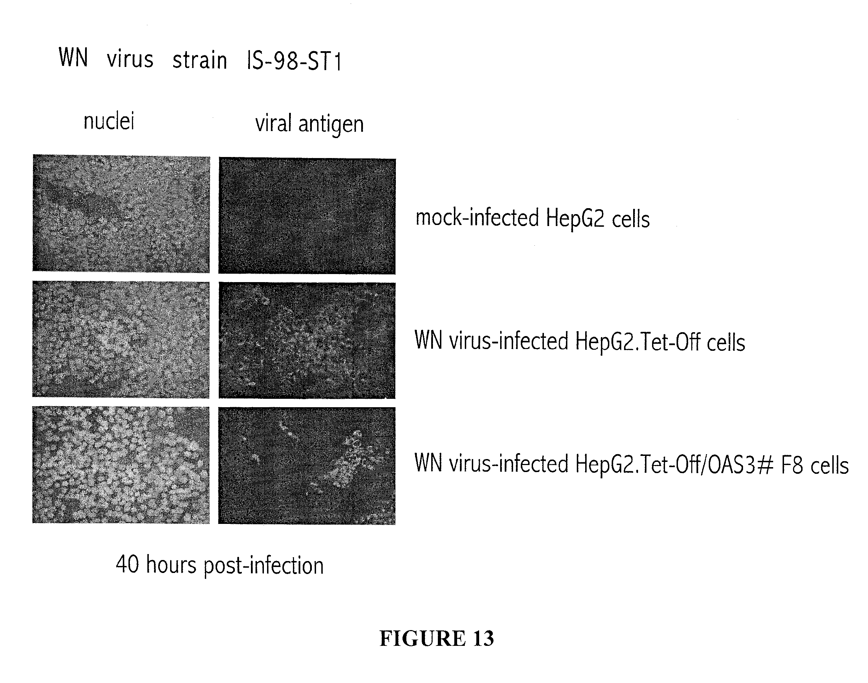

- figure 13: OAS3-expressing HepG2 cells show resistance to West Nile virus infection. Parental HepG2.Tet-Off and induced HepG2.Tet-Off/OAS3#F8 cell clone were infected with West Nile virus strain IS-98-ST1 (Lucas et al., Virology J., 2004, 1, 9-) at multiplicity of infection 1 AP61FFU/cell or mock-infected. At 40 hours post-infection, cells were fixed with methanol/acetone at -20°C for 20 min. Immunofluorescence assay was performed using cy3-conjugated anti-West Nile E mAb E24. Nuclei were stained with DAPI.

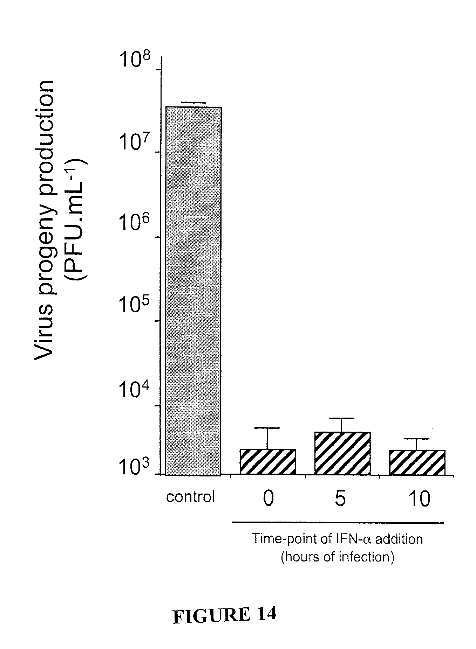

- figure 14 shows that IFN-α is able to establish an antiviral state against YFV 17D in human epithelial HeLa cells. HeLa cells were infected with YFV 17D at 1 PFU/cell and then treated with 1,000 IU/ml human IFN-α or mock-treated (control) at various time-points post-infection. Virus progeny productions were determined at 72 h post-infection.

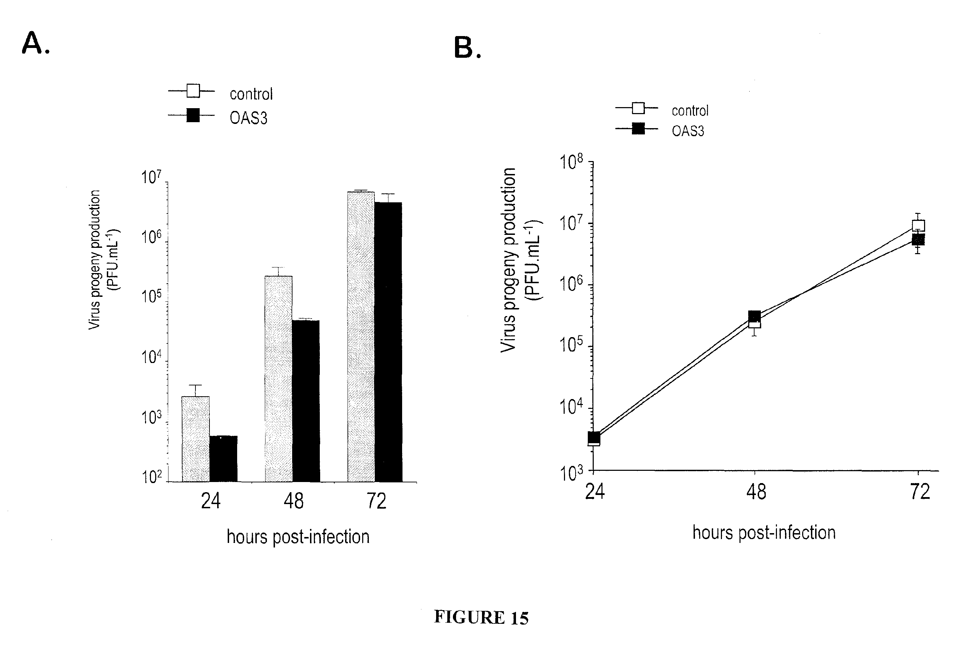

- figure 15 shows that 17D live-attenuated strain of YFV shows unexpected resistance to OAS3-mediated antiviral effects in human cells regardless either the timepoints of post- infection or the multiplicity of infection. HeLa (control) and induced HeLa.Tet-Off/OAS3 (OAS3) cells were exposed to YFV strain 17D at low (panel A; 1PFU/cell) or high (panel B; 10 PFU/cell) virus input.

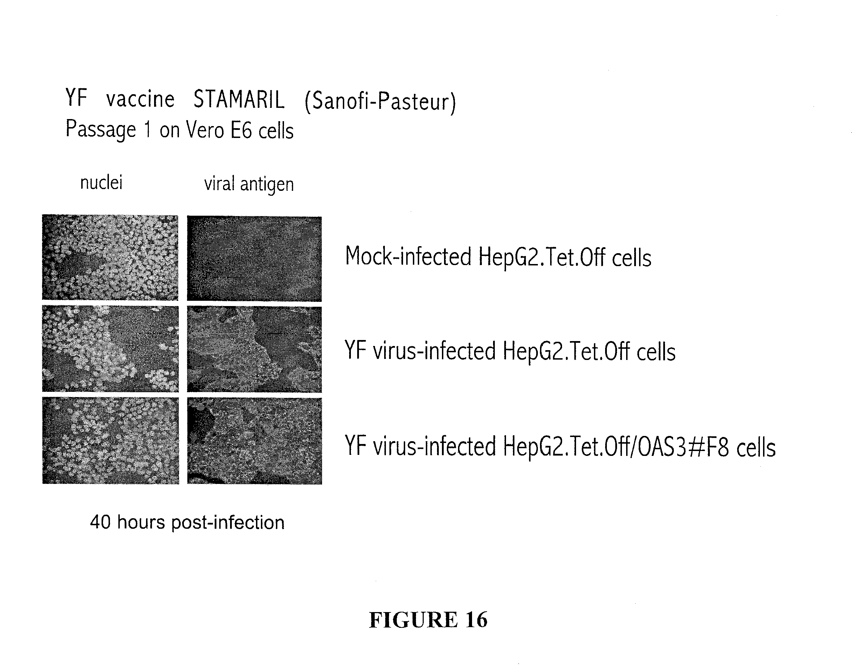

- figure 16: No inhibition of yellow fever vaccine 17D-204 in OAS3-expressing HepG2 cells. Parental HepG2.Tet-Off and induced HepG2.Tet-Off/OAS3#F8 cell clone were infected with live-attenuated STAMARIL vaccine of yellow fever (Sanofi-Pasteur) grown once on Vero E6 cells or mock-infected. At 40 hours post-infection, cells were fixed with methanol/acetone at -20°C for 20 min. Immunofluorescence assay was performed using anti-French Neurotropic Virus HMAF and FITC-conjugated goat anti-mouse Ig. Nuclei were stained with DAPI.

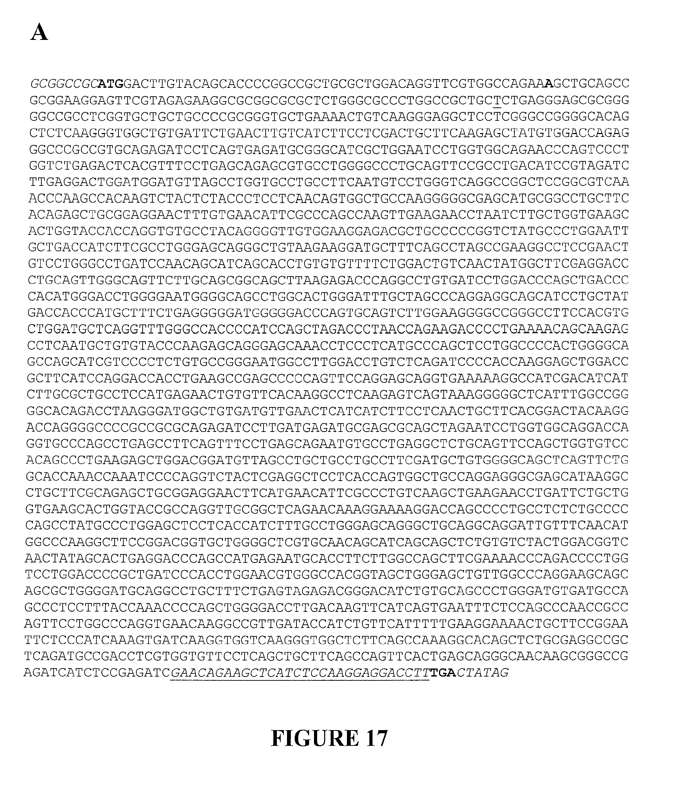

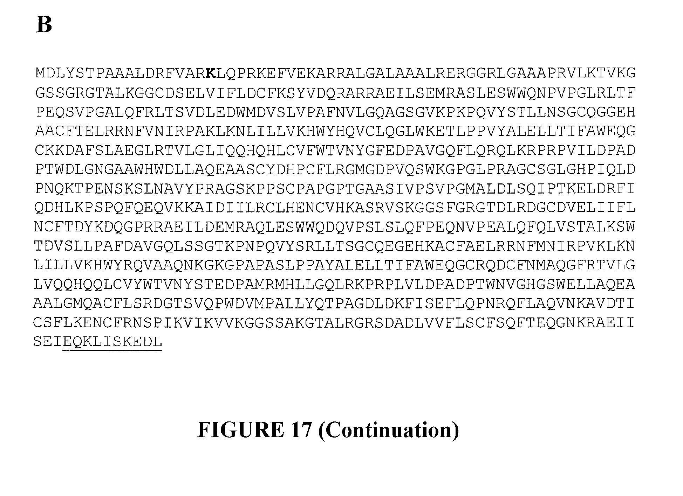

- figure 17 represents the nucleotide (A) and amino acid (B) sequences of truncated human recombinant OAS3 (OAS3 1-843). These nucleotide and amino acid sequences correspond to SEQ ID NO: 25 and SEQ ID NO: 26, respectively. Changes between OAS3 mRNA reference sequence and cloned OAS3 cDNA fragment are underlined if synonymous or shown in bold if non synonymous. The translation initiation and stop codons are in bold. The additional nucleotide and amino acid sequences corresponding to the C_terminal c-myc epitope are underlined. The 5' *Not*I and 3' *EcoRV* restriction sites are shown in italics.

### Example 1: Establishment and evaluation of HeLaTet-Off/OAS3 cell lines

### 1) Material and methods

### a) Cell cultures

The HeLa.Tet-Off cell line was purchased from BD BIOSCIENCES CLONTECH. HeLa.Tet-Off cells are maintained in 5 % CO₂ at 37 °C, in DMEM (INVITROGEN), supplemented with 10 % heat-inactivated fetal calf serum (FCS), 4 mM L-glutamine, 100 UI/ml penicillin, 10 µg/ml streptomycin and 200 µg.mL⁻¹ G418 (INVITROGEN).

The HeLa.Tet-Off/OAS3 cell line (CNCM I-3927) is maintained in DMEM, 10 % FCS, 4 mM L-glutamine, 100 UI/ml penicillin, 10 µg/ml streptomycin, 200 µg.mL⁻¹ G418, 100 µg.mL⁻¹ hygromycin B (BD BIOSCIENCES CLONTECH), and 2 µg.mL⁻¹ tetracycline (SIGMA-ALDRICH). The cells are grown in monolayers; the expected cell density is of 80 % to 100 %; the population doubling time is of about two days. The cells are harvested by trypsinization; they are sub-cultured 1/10 every week. The cells have a limited lifespan (15 to 20 passages). The cells are frozen in DMEM supplemented with 20 % FCS, 10 % DMSO, 4 mM glutamine, 100 UI/ml penicillin, 10 µg/ml streptomycin, 200 µg.mL⁻¹ G418, 100 µg.mL⁻¹ hygromycin B, and 2 µg.mL⁻¹ tetracycline.

The HeLa.Tet-Off/OAS3/delta/1C (OAS3^{ΔC-term}) cell line (CNCM I-3968) is maintained in DMEM, 10 % FCS, 20 mM L-glutamine, 10,000 UI/ml penicillin, 10 µg/ml streptomycin, 200 µg.mL⁻¹ G418, 100 µg.mL⁻¹ hygromycin B (BD BIOSCIENCES CLONTECH), and 10 µg.mL⁻¹ tetracycline (SIGMA-ALDRICH). The cells are grown in monolayers; the expected cell density is of 90 %; the population doubling time is of about two days. The cells are harvested by trypsinization; they are sub-cultured 1/10 every week. The cells have a limited lifespan (25 passages). The cells are frozen in DMEM supplemented with 20 % FCS, 10 % DMSO, 4 mM glutamine, 10,000 UI/ml penicillin, 10 µg/ml streptomycin, 200 µg.mL⁻¹ G418, 100 µg.mL⁻¹ hygromycin B, and 10 µg.mL⁻¹ tetracycline.

### b) Establishing HeLa. Tet-Off cell lines expressing OAS3 proteins

Two overlapping cDNA fragments of human OAS3 cloned separately in the PCR4-TOPO vector (INVITROGEN), were used as templates for expression of the full-length OAS3 protein (aa 1 to 1,087; SEQ ID NO: 24 and Figure 1) or the truncated form OAS3^{ΔC-term} or OAS3^{[1-843]} (aa 1 to 843; SEQ ID NO: 26 and Figures 3C, 9 and 17). Nucleotide and amino acids differences between reference OAS3 sequence (GenBank accession number NM-006187; SEQ ID NO: 27) and OAS3 cDNA used in this study (Figure 1) are listed in Table I.

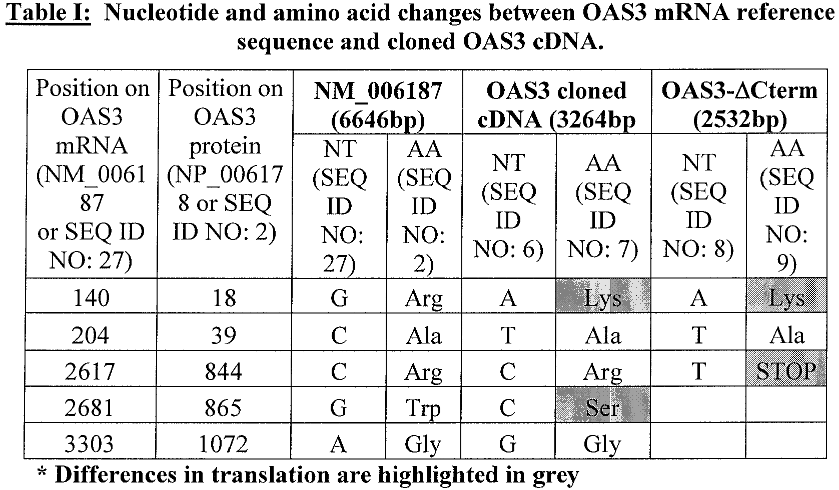

The OAS3 sequence was modified by PCR to be flanked on the 3' open reading frame end by the additional 10-residue sequence EQKLISKEDL (SEQ ID NO: 10) followed by a stop-codon with the couple of primers pTet-OAS3-univl and pTet-OAS3-rev2 for OAS3 and the couple of primers pTet-OAS3-univlbis and pTet-OAS3-rev2bis for OAS3^{[1-831]} (Table II).

**Table II: Primers sequences (SEQ ID NO: 11 to 22)**

| **primer** | **Nucleotide sequence (5' -> 3')** |
|---|---|
| OAS3.R844X-F | gtggtgttcctcagctgctt |
| OAS3.R844X-R | catcgtctgggatgtcagtg |
| pTet-OAS3-univ1 | attaatgcggccgcaacgaaaccagaaatccgaaggcc |
| pTet-OAS3-rev2 | aactaactatagtcaaaggtcctccttggagatgagcttctgttc cacagcagccttcactggccatggctggatggg |
| pTet-OAS3-univ-1bis | attaatgcggccgcatggacttgtacagcaccccggccgctgcg |
| pTet-OAS3-rev-3bis | aactaactatagtcaaaggtcctccttggagatgagcttctgttcgatct cggagatgatctcggcccgccgcttgttgcc |
| OAS3-For | cggacggtgctggggctcgtg |
| OAS3-Rev | cttgtcttcgagtagaggcttctc |
| Chik/E2/9018/+ | caccgccgcaactaccg |
| Chik/E2/9235/- | gattggtgaccgcggca |
| GAPDH-For | gggagccaaaaggg |
| GAPDH-Rev | ggggacacggaagg |

Enzyme recognition site are underlined. Sequence complementary to a stop codon are shown in bold. The OAS3 sequences (SEQ ID NO: 23 and 25) are flanked by the *Not*I and *EcoR*V restriction enzymatic sites at the downstream and upstream ends, respectively. The PCR products were digested with *Not*I and *EcoR*V and then inserted into the unique sites *Not*I and *EcoRV* of pTRE2hyg expression vector (BD BIOSCIENCES CLONTECH) to generate pTRE2hyg-OAS3 (Figure 1) and pTRE2hyg/OAS3CΔ^{C-term} (OAS3 1-843). In this configuration, the OAS3 insert is under the control of the Tet-Off expression system. The Tet-Off system allows the induction of a foreign gene expression by the withdrawal of repressor tetracycline (Tet). HeLa.Tet-Off cells (BD BIOSCIENCES CLONTECH) were transfected with pTRE2hyg-OAS3 or pTRE2hyg/OAS3CΔ^{C-term}, using transfectant reagent Fugene 6 (ROCHE)) according to the manufacturer's recommended procedure. The Tet-Off expression system was repressed by adding 2 µg/ml of Tet to culture medium.The transfected cells were selected on growth medium containing inhibitors G418 and hygromycin and then cloned from single cells by limiting dilution in presence of 10 µg.mL⁻¹ repressor Tetracycline (Tet). For Tet withdrawal, cell monolayers were trypsined and cells were washed at least five times with non-supplemented DMEM before replacing with DMEM/10 % FBS supplemented with genotoxic drugs only. The level of recombinant OAS3 mRNA production in induced cells (- Tet) relative to that in uninduced cells (+ Tet) was determined by RT-PCR analysis using the couple of primers OAS3-For and OAS3-Rev (Table II). Based on these experiments, the inducible HeLa.Tet-Off/OAS3#C417-1 and HeLa.Tet-Off/OAS3/delta/1C (OAS3^{ΔC-term}) clones were selected. The HeLa.Tet-Off/OAS3#C417-1 cell line was deposited February 26, 2008, at the Collection Nationale de Cultures de Microorganismes, 25 rue du Docteur Roux, 75724 Paris Cedex 15, under the accession number I-3927. The HeLa-Tet-Off/OAS3/delta/1C cell line was deposited April 17, 2008, at the Collection Nationale de Cultures de Microorganismes, 25 rue du Docteur Roux, 75724 Paris Cedex 15, under the accession number I-3968. Both HeLa.Tet-Off/OAS3 cell lines were maintained under repressing condition in the presence of 2 µg.mL⁻¹ Tet.

### c) Virus

Production of clinical isolate Chikungunya virus strain 06-49 (CHIKV 06-49; Schuffenecker et al., PLoS Medicine 3, 2006, e263) on mosquito *Aedes pseudoscutellaris* (AP61) cell monolayers and virus titration by focus immunodetection assay (Desprès et al., Virology, 1993, 196, 209-219) were performed as previously described (Brehin et al., Virology, 2008, 371, 185-195). Infectivity titers were expressed as focus forming units (FFU) on AP61 cells. In order to assay its antiviral effect, human IFN-α (BIOSOURCE) was directly added to culture medium at 1,000 IU.mL⁻¹.

### d) Immunoblot assays

Cellular proteins were subjected to immunoblot analysis as previously described (Brehin et al., Virology, 2008, 371, 185-195). Viral protein expression was detected using anti-CHIKV HMAF or anti-CHIK.E2 MAb 3E4 (Bréhin et al., Virology, 2008, 371, 185-195). OAS3 protein expression was detected using anti-OAS3 N-term (Santa-Cruz) or C-term (ABGENT) antibodies.

### e) Flow cytometry analysis

Cells were detached and then fixed with 3.2 % paraformaldehyde in PBS. Fixed cells were permeabilized, stained with anti-CHIKV HMAF and analyzed by flow cytometry as described previously (Bréhin et al., Virology, 2008, 371, 185-195).

### 2) Results

First, the susceptibility of parental HeLa.Tet-Off cell line to CHIKV infection, was evaluated (Figure 3A). An analysis of CHIKV replication in these cells infected at 1 multiplicity of infection (MOI) showed that production of progeny virus reached ~ 7.0 log FFU.mL⁻¹ at 18 h p.i. By flow cytometry analysis using MAb 3E4 reactive to CHIKV E2 glycoprotein (Brehin et al., Virology, 2008, 371, 185-195), about 50 % of CHIKV-infected HeLa.Tet-Off cells were positive for viral antigens. Then, the ability of IFN-α to establish an antiviral state in HeLa.Tet-Off cells, was investigated. Pretreatment of HeLa.Tet-off cells with 1,000 IU.mL⁻¹ human IFN-α 5 hours prior CHIK exposure (1 MOI) resulted in about 1.5 log reduction in virus titer at 18 h p.i. (Figure 3B). Thus, IFN-dependent antiviral pathways are functional in HeLa.Tet-Off cells and provide protection against CHIKV at cellular level. However, CHIKV-infected cells showed complete resistance to IFN-α at 5 h p.i. once virus replication is established inside infected HeLa.Tet-Off cells.

The stable HeLa.Tet-Off/OAS3#C417-1 cell clone that up-regulates OAS3 protein expression under the control of the Tet-Off expression system was selected to assess the antiviral activity of the large form of OAS. The recombinant OAS3 protein is composed of three adjacent OAS units (domain I, II, and III) including three potential active catalytic sites (Figure 3C). The expression of recombinant OAS3 protein was analyzed by immunoblotting of cell lysates with a polyclonal immune serum directed against the C-terminal region of human OAS3 protein (Figure 3D). Cells incubated with 1,000 IU.mL⁻¹ of IFN-α for 5 hours served as a positive control. Upon IFN-α treatment, the production of endogenous OAS3 molecules was clearly detected in HeLa.Tet-Off cells whereas no basal expression of OAS3 was observed in resting cells. Thus, OAS3 gene expression may be actively up-regulated by IFN-α/β. A basal level of recombinant OAS3 protein was observed in uninduced HeLa.Tet-Off/OAS3 cells (2 µg.mL⁻¹ Tet) indicating that shut-off was incomplete (Figure 3D). When the repressor Tet was removed 24 h from the culture medium, there was a significant increase in production level of recombinant OAS3 protein compared with that found in uninduced cells.

### Example 2: Antiviral effects of OAS3 against CHIKV

### 1) Material and methods

The experimental procedures are as described in example 1.

### 2) Results

To investigate the effect of OAS3 protein on CHIKV growth in human epithelial cells, HeLa.Tet-Off/OAS3 cells were infected 18 h with CHIKV.06-49 at 1 MOI. As determined by flow cytometry using MAb 3E4, uninduced HeLa.Tet-Off/OAS3 and HeLa.Tet-Off cells displayed similar susceptibility to CHIKV infection (Figure 4A). Induction of HeLa.Tet-Off/OAS3 cells 24 h prior to virus exposure resulted in reduction of CHIKV-infected cells by at least 20 %. As a positive control, IFN-α treatment reduced the percentage of infected HeLa.Tet-Off cells by 75 %. Thus, CHIKV is sensitive to ectopic expression of OAS3 in human epithelial cells.

To further assess the efficiency with which HeLa.Tet-Off/OAS3 cells inhibit CHIKV growth, induced cells were exposed to increasing input of CHIKV-06.49 (Figure 4B). At 18 h p.i., expression of recombinant OAS3 protein reduced the progeny virus production by 1.5 to 2.0 log regardless of the MOI tested. At 24 h p.i., there was about 3.0 log reduction in the viral titer at the lower MOI tested. Thus, expression of OAS3 can suppress the cell-to-cell spread of CHIKV in human fibroblastic cells. At moderate MOI, the virus progeny was still reduced by at least 2.0 log. At 10 MOI, viral growth was reduced only by about 0.5 log suggesting that CHIKV has the ability to avoid the antiviral activities of OAS3 when infection was performed at high virus input.

Kinetic studies showed that OAS3-mediated inhibition of CHIKV was effective during virus life cycle (Figure 4C). At 12 h p.i., there was a 2.0 log reduction in the viral titer recovered from HeLa.Tet-Off/OAS3 cells as compared with HeLa.Tet-Off cells. Induction of OAS3 protein expression was still able to reduce the progeny virus production by at least 1.5 log at 24 h p.i.

Measurements of Lactate dehydrogenase (LDH), a cytoplasmic enzyme that is released into the culture medium upon cell lysis and therefore is a measure of membrane integrity, showed no significant loss of viability of infected HeLa/Tet-Off/OAS3 cells within the first 24 h of infection as compared to infected parental cells. Thus, the resistance to CHIKV infection was directly related to the antiviral activity of the OAS3 rather than elimination of virus-infected cells through apoptosis induction.

### Example 3: Antiviral action of OAS3 against other positive-sense single-stranded RNA viruses in HeLa.Tet-Off recombinant cell line expressing human OAS3

### 1) Material and methods

The experimental procedures are as described in example 1. In addition virus production and titration were performed as described below.

### Viruses

Production of low passaged West Nile Virus (WNV) IS-98-ST1 strain (GenBank accession number AF 481864) on mosquito *Aedes pseudoscutellaris* (AP61) cell monolayers and virus titration by focus immunodetection assay (Desprès et al., Virology, 1993, 196, 209-219) were performed as previously described (Bréhin et al., Virology, 2008, 371, 185-195; International Application WO 02/081741). Infectivity titers were expressed as focus forming units (FFU) on AP61 cells. Sindbis virus strain AR339 (SINV AR339) and Semliki Forest Virus strain SF 64 (SFV 64) were propagated on African green monkey kidney (VERO) cell line and infectivity titers were expressed as plaque forming units (PFU) on VERO cells. In order to assay its antiviral effect, human IFN-α (BIOSOURCE) was directly added to culture medium at 1,000 IU.mL⁻¹.

### 2) Results

The ability of the large form of OAS to inhibit growth of SINV strain AR339, SFV strain SF 64 and WNV strain IS-98-ST1 in human epithelial cells was examined (Figures 4D, 5 and 6). HeLa.Tet-off and induced HeLa.Tet-Off/OAS3 cells were infected with alphaviruses at 1 MOI. In response to OAS3 protein expression, there was a comparable degree of inhibition (~ 2 log) in progeny virus production between SINV and SFV at 18 h p.i (Figure 4D). HeLa.Tet-off and induced HeLa.Tet-Off/OAS3 cells were infected 48 hours with flavivirus (WNV) at 0.1, 1 or 10 MOI (Figure 5 and 6). Based on the measurement of viral titers, it was shown that human epithelial cells respond to OAS3 expression by efficiently inhibiting WN virus replication. At 0.1 MOI, there was a 1.5 log reduction in the viral titer recovered from HeLa.Tet-Off/OAS3 as compared with HeLa.Tet-Off cells (Figure 6). Induction of OAS3 protein expression was able to reduce the progeny virus production by at least 1.0 log at 72 h post-infection. Thus, OAS3-expressing HeLa cells show lower susceptibility to WN virus infection as compared to parental cells. In conclusion, the data show that OAS3 has antiviral action against RNA viruses such as alphaviruses and flaviviruses inside human cells.

### Example 4: Mechanisms of OAS3-mediated CHIKV inhibition

### 1) Material and methods

The experimental procedures are as described in example 1. In addition quantitative RT-PCR was performed as described below.

### Quantitative RT-PCR

Real-Time RT-PCR analysis of viral RNA accumulation was performed with an ABI Prism 7700 sequence detection using the SYBR® Green PCR essentially as described previously (Kajaste-Rudnistki et al., J. Biol. Chem., 2006, 281, 4624-4637). The primers for CHIKV E2 gene are Chik/E2/9018/+ and Chik/E2/9235/- (Table II). GAPDH mRNA was used as an endogenous sequence control for the normalization of each sample. The primers GAPDH-For and GAPDH-Rev are listed in Table II. For the quantification of viral RNA copies, an *in vitro* CHIK RNA transcript encoding the N-terminal region of E2 was performed to build the standard curve as previously described (Vazeille et al., PLoS One, 2007, 2(11): e1168).

### 2) Results

In an effort to resolve the molecular basis of the antiviral action of OAS3, it was determined whether inhibition of CHIKV growth was due to a lack of accumulation of structural virus proteins. Total proteins were extracted from Hela.Tet-off and induced HeLa.Tet-Off/OAS3 cells at the 18-h timepoint post-infection and capsid protein (C), envelope glycoproteins pE2 (precursor of E2), E2, and E1 were detected by immunoblot using anti-CHIKV HMAF, whereas envelope glycoproteins pE2 (precursor of E2) and E2 were detected specifically by immunoblot using anti-CHIKV E2 MAb 3E4. As shown in Figure 7A, there was a background level of structural virus proteins in response to OAS3 protein expression. Thus, the inability of CHIKV to productively infect OAS3-expressing HeLa cells is associated with a severe reduction in viral protein synthesis.

It was determined whether the inefficiency of viral protein synthesis was due to a lack of accumulation of viral RNA. Total RNA was extracted from Hela.Tet-off and induced HeLa.Tet-Off/OAS3 cells at the 8 h time-point post-infection and production of genomic and subgenomic viral RNAs was analyzed using RT-PCR assay with primers designed on the basis of CHIKV E2 gene. As determined by real-time RT-PCR analysis, there was a 2 log reduction in viral RNA level recovered from OAS3-expressing cells when compared to that found in parental cells (Figure 7B). Thus, OAS3 expression prevents viral RNA accumulation inside infected cells. On the basis of these results, it can be concluded that OAS3 expression affects alphaviral replication inside infected human cells.

### Example 5: Impact of OAS3 genetic polymorphism in healthy individuals on OAS3 antiviral effect

### 1) Material and methods

The experimental procedures are as described in example 1. In addition, genotyping of OAS3 polymorphism was assayed as described below.

### Genotyping

The SNP OAS3.R844X was genotyped by PCR-RFLP assay using the couple of primers OAS3.R844X-F(SEQ ID NO: 11) and OAS3.R844X-R (SEQ ID NO: 12) which are presented in Table II. The 183 bp-long PCR product was subjected to digestion with *Bgl* II and the detection of two fragments of 115 and 68 bp indicated the presence of OAS3.R844X.

### 2) Results

The *OAS3* gene was screened for polymorphisms. Re-sequencing total 16 exons, flanking introns and 2 kb 5' to the *OAS3* gene in fourty-eight healthy Caucasians individuals identified a single-nucleotide polymorphism (SNP) at the first position of codon CGA-844 where the substitution T for C resulted in non-sense mutation (OAS3.R844X) (Table I). This SNP was further screened in 180 healthy Caucasian individuals and identified two heterozygotes which gave allele frequency of 0.5 %. Because OAS3.R844X possibly truncates about 20 % of the OAS3 protein from the carboxy terminus (Figure 3C), the stable HeLa.Tet-off/OAS3^{Δ-term} cell clone which contains the OAS3-1 to OAS3-843 sequence, was generated. It is of interest to note that OAS3^{ΔC-term} lacks the D⁸⁸⁹ residue which composes the triad defining the active catalytic site of the third domain of OAS3 (Sarkar *et al.,* 1999; Rambouillat et *al.,* 2000). To assess the ability of mutant OAS3 to inhibit CHIKV and WNV growth, induced HeLa.Tet-Off/OAS3 and HeLa.Tet-off/OAS3^{ΔC-term} cells were infected at 1 MOI with either CHIKV-06-49 or West Nile Virus (WNV) IS-98-ST1 strain. As shown in Figures 8 and 9, OAS3^{ΔC-term} -expressing cells displayed resistance to CHIKV and WNV infection indicating that OAS-1 to OAS3-843 sequence is still able to provide protection. Analysis of viral growth showed that ectopic expression of OAS3^{ΔC-term} resulted in a lower efficiency of CHIKV and WNV inhibition as compared to full-length OAS3. Such result suggests that SNP at codon CGA-844 may have an impact on the antiviral activity.

### Example 6: Antiviral action of OAS3 against DV and WNV in Hep.G2.Tet-Off cell line expressing human OAS3

### 1) Material and methods

### a) Cell culture

The HepG2.Tet-Off cell line was purchased from CLONTECH (# 632106). HepG2.Tet-Off cells are maintained in 5 % CO₂ at 37 °C, in DMEM (GIBCO, # 41965), supplemented with 10 % heat-inactivated fetal calf serum (FCS), 4 mM L-glutamine, 100 UI/ml penicillin G sodium, 100 µg/ml streptomycin sulfate (GIBCO, # 15140-122), 0.1 mM non-essential amino acids, and 100 µg.mL⁻¹ G418 (GIBCO, # 10131-019).

The HepG2.Tet-Off/OAS3#F8 cell line (CNCM I-4158) is maintained in DMEM, 10 % FCS, 4 mM L-glutamine, 100 UI/ml penicillin, 100 µg/ml streptomycin, 100 µg.mL⁻¹ G418, 100 µg.mL⁻¹ hygromycin B (CLONTECH, # 631309), and 2 µg.mL⁻¹ tetracycline (SIGMA-ALDRICH, # T-7660).

### b) Establishing HepG2.Tet-Off cell lines expressing OAS3 protein

An inducible HepG2.Tet-Off/OAS3#F8 clone expressing the full-length human OAS3 protein (SEQ ID NO: 24) was selected as described in example 1 for the inducible HeLa.Tet-Off/OAS3#C417-1. The HepG2.Tet-Off/OAS3#F8 cell line was deposited May 15 2009, at the Collection Nationale de Cultures de Microorganismes, 25 rue du Docteur Roux, 75724 Paris Cedex 15, under the accession number I-4158

This cell line is maintained under repressing condition in the presence of 2 µg.mL⁻¹ Tet.

### c) Virus

Production of low passaged West Nile Virus (WNV) IS-98-ST1 strain (GenBank accession number AF 481864; Lucas et al. Virology J., 2004, 1, 9-) on mosquito *Aedes pseudoscutellaris* (AP61) cell monolayers and virus titration by focus immunodetection assay (Desprès et al., Virology, 1993, 196, 209-219) were performed as previously described (Bréhin et al., Virology, 2008, 371, 185-195). Production of DV1 strain FGA/NA d1d and virus titration were performed as previously described (Duarte dos Santos et al. Virology, 2000, 274, 292-). Infectivity titers were expressed as focus forming units (FFU) on AP61 cells. In order to assay its antiviral effect, human IFN-α (BIOSOURCE) was directly added to culture medium at 1,000 IU.mL⁻¹.

### d) Immunofluorescence assay

Virus-infected cells were fixed with methanol/acetone at -20°C for 20 min. Immunofluorescence assay was performed using FITC-conjugated anti-dengue E mAb 4E1 or cy3-conjugated anti-WNV.E MAb E24. Nuclei were stained with DAPI.

### 2) Results

First, the susceptibility of parental HepG2.Tet-Off cell line to DV infection was evaluated (Figure 10A). Cells were infected with different Focus Forming Units (AP61FFU) per cell of DV-1 virus strain FGA/NA d1d. At 40 h post-infection, the cells were analyzed by flow cytometry using MAb 4E11 reactive to DV E glycoprotein. With a DV-1 virus input of 30 AP61FFU per cell, about 50 % of HepG2.Tet-Off cells were infected with DV-1 virus as evidenced by a positive signal for the dengue E glycoprotein.

Then, the ability of IFN-α to establish an antiviral state in HepG2.Tet-Off cells was investigated. Pretreatment of HepG2.Tet-off cells with 1,000 IU.mL⁻¹ human IFN-α 5 hours prior DV infection (10 MOI) resulted in about 0.7 log reduction in virus titer at 40 h p.i. (Figure 10B). Thus, IFN-dependent antiviral pathways are functional in HepG2.Tet-Off cells and provide protection against DV at cellular level.

To investigate the effect of OAS3 protein on DV growth in Hep.G2 cells, induced (-Tet) and uninduced (+Tet) HepG2.Tet-Off/OAS3 cells and parental HepG2.Tet-Off cells were infected with increasing input of DV-1 virus strain FGA/NA d1d. At 40 h p.i., immunofluorescence assay using anti-dengue E mAb was performed and the percentage of HepG2 cells positive for viral antigen was determined (Figures 11 and 12). The progeny virus production was determined at the multiplicity of infection of 25 (Figure 11).Expression of recombinant OAS3 protein reduced the percentage of DV-infected cells by at least 75 % (Figures 11 and 12) and progeny virus production by 2 log (Figure 11).

Thus, OAS3-expressing HepG2 cells show resistance to DV infection.

The effect of OAS3 protein on WNV growth in Hep.G2 cells was also analyzed by immunofluorescence assay. Parental HepG2.Tet-Off and induced HepG2.Tet-Off/OAS3#F8 cell clone were infected with West Nile virus strain IS-98-STl at multiplicity of infection 1 AP61FFU/cell or mock-infected. At 40 h post-infection viral antigen (WNV E glycoprotein) was detected by immunofluorescence using cy3-conjugated mAb E24. The results (Figure 13) confirmed those obtained in HeLa cells (example 3), showing that OAS3-expressing human cells show resistance to WNV infection.

### Example 7: Live-attenuated 17D-204 vaccine strain of yellow fever virus shows surprising resistance to the antiviral effects mediated by the large form of human 2',5'-Oligoadenylate Synthetase (OAS3) in human cells

### 1) Material and methods

Attenuated Yellow Fever Virus (YFV) vaccine strain (STAMARIL®, AVENTIS-PASTEUR) was propagated on African green monkey kidney (VERO) cell line and infectivity titers were expressed as plaque forming units (PFU) on VERO cells. In order to assay its antiviral effect, human IFN-α (BIOSOURCE) was directly added to culture medium at 1,000 IU.mL⁻¹. Immunofluorescence assay was performed as described in example 6, using anti-French Neurotropic Virus HMAF and FITC-conjugated goat anti-mouse Ig.

### 2) Results

Examples 2, 3, 4 and 6 provided the first evidence that OAS3-dependent antiviral activity mediated by IFN-α/β represents a major human cell defence strategy against alphaviruses such as Chikungunya virus and flaviviruses such as West Nile and dengue viruses in human cells.

It was investigated whether OAS3 displays antiviral activity against yellow fever virus (YFV). For this, live-attenuated 17D-204 vaccine strain of YFV (STAMARIL, Aventis-Pasteur) were twice propagated on African green monkey kidney (VERO) cell line and infectivity titers were expressed as Plaque Forming Units (PFU) on VERO cells. HeLa cells were infected with YFV 17D at 1 PFU/cell and then treated with 1,000 IU/ml human IFN-α or mock-treated (control) at various time-points post-infection. Virus progeny productions were determined at 72 h post-infection. As shown in Figure 14, IFN-α is able to establish an antiviral state against YFV 17D in human epithelial HeLa cells. To assess the ability of OAS3 to inhibit viral growth, HeLa (control) and induced HeLa.Tet-Off/OAS3 (OAS3) cells were exposed to YFV strain 17D at low (1PFU/cell; Figure 15A) or high (10 PFU/cell; Figure 15B) virus input. There was no obvious differences in YFV replication between both cell populations (Figure 15). Kinetic studies showed that 17D is resistant to OAS3-mediated antiviral effects in human cells regardless either the time points of post- infection or the multiplicity of infection.

The resistance of YFV 17D to OAS-3 mediated antiviral effect was confirmed by immunofluorescence assay in parental HepG2.Tet-Off and HepG2.Tet-Off/OAS3#F8 cell clone infected with YFV 17D (Figure 16).

The observation that live-attenuated strains of YFV showunexpected resistance to antiflaviviral action of OAS3 opens a new avenue for elucidating the mechanism of flavivirus attenuation.

### Example 8: A non-synonymous variant of OAS3 is associated with the pathogenesis of dengue shock syndrome.

### 1) Material and methods

### a) Patients and controls

750 patients (male:female ratio = 0.99) with symptomatic dengue viral (DV) infection during the 3 year period (June 2000-2003) were enrolled in the study from two medical centers in Bangkok, Ramathibodi Hospital and Siriraj Hospital, Mahidol University and 1 hospital in Khon-Kaen province, Thailand. Their ages ranged from 1 to 25 years with a mean of 9.6 years. Patients with suspicion of dengue viral infection based on clinical features including high fever, severe headache, retro-orbital pain, myalgia, arthalgia, nausea and vomiting, and rash were admitted to the hospitals for clinical observation and treatment. The diagnosis of dengue virus infection was later confirmed by a comparable IgG and IgM enzyme-linked immunosorbent assay titer on a late acute and/or convalescent sera. Differential diagnosis of DF (Dengue fever) and DHF (Dengue Hemorrhagic Fever) was established based on the absence (DF) or presence (DHF) of evidence for increased vascular permeability manifested by hemoconcentration or pleural effusion. Specifically, the diagnosis of DHF was made based on all of the four following characteristics: 1) high continuous fever lasting 2-7 days, 2) hemorrhagic tendency such as a positive tourniquet test, petechii, purpura or hematemesis, 3) thrombocytopenia (platelet count ≤ 100,000/µl and 4) evidence of plasma leakage due to increased vascular permeability manifested by hemoconcentration (an increased in hematocrit of 20 % or more) or pleural effusion. Some dengue patients who could not be classified as DF or DHF because of unclear clinical symptoms were assigned an unknown DF/DHF status. The severity of DHF was categorized by four grades according to WHO criteria. Grades III and IV were DHF with narrowing pulse pressure with a characteristically elevated diastolic pressure to profound shock. Secondary infection was defined as a dengue-specific IgM/IgG ratio < 1.8. The project protocol and objectives of the study were carefully explained to the patients and their parents or relatives. Informed consent was individually obtained from all subjects. The protocol has been approved by the ethical committee of each hospital.

The controls consisted of 296 blood donors from Ramathibodi hospital and 216 blood donors from Siriraj Hospital and 184 ethnic matched healthy controls from Khon-Kaen region (male:female ratio = 1). Both cases and control groups came from Bangkok and Central part of Thailand. Whole blood samples were collected from patients and controls on EDTA, and DNA was extracted using a standard phenol/chloroform extraction method.

During 2004-2006, we enrolled additional 254 patients (male:female ratio = 1.07) from Ramathibodi and Khon-Kaen hospital using the same clinical and viral diagnosis criteria.

### b) Polymorphisms identification and genotyping

Polymorphisms were identified by direct sequencing of PCR amplified genomic DNA, on individual DNA samples (24 dengue patients and 32 Thai controls). Primers used for sequencing OASs exons, part of introns and 5' and 3' regions are shown in Table III.

**Table III: OAS3 sequencing primers (SEQ ID NO: 31 to 142)**

| **gene/exon** | **Forward primer sequence** | **Reverse primer sequence** | **PCR product size** |
|---|---|---|---|
| OAS1/pro1 | CCAGGAGTTCGAGACCAGAC | TGCAGGGACAGTCACAGAAC | 546 |
| OAS1/pro1 | GGTGAGGCATAGGGGATTTT | ATAGGAGGTGGGGCTTGACt | 409 |
| OAS1/pro3 | CCTTGAACCCAGGAAGTTGA | CCTTTGTCCTTTAGCCAGCA | 498 |
| OAS1/pro4 | CTTTTTGCAGTGGGCATGTA | TGTCAATGGCATGGTTGATT | 503 |
| OAS1/exon1 | TTTGCAAAAGGAAAGTGCAA | GAAACCACCATCCCAGAAGA | 503 |
| OAS1/exon2 | AGCATCCATTTTCCCATCTG | CCCACCCTGCTTTAGAGAGA | 604 |
| OAS1/exon3 | GGATCAGGAATGGACCTCAA | GGCTCCTCTCTCCACCTCTT | 499 |
| OAS1/exon4 | AATGAATGAGCCTGGATTCG | TACAATGATGGGCAACAGGA | 493 |
| OAS1/exon5 | GAGCCCTTCCTCATGTTCTG | ATAAATGGCCTTTGGCAAGA | 396 |
| OAS1/exon6-1 | AGCTGGGGCTTGTTAGTCCT | TGGAATGAAGTGAGGCTGTG | 502 |
| OAS1/exon6-2 | GCTCCTCAGTGAGCTGGTGT | TCCATGAGGACAGGGATTTT | 508 |
| OAS1/exon6-3 | CGGCCATGTCATTGTTTTTAT | ACTGCCAGAGTGGAGATGCT | 560 |
| OAS2/pro1 | AAAGCACAGGCTTTGGAGTC | TGAGCCCAGGTACCCAGATA | 600 |
| OAS2/pro2 | TTGGGGGAGCTCAGTCTTTA | CTGACTTGCTCAGGGACACA | 583 |
| OAS2/pro3 | TTTCTGGCTCACACACTTGG | AGCAGGGAAACCAAAACTGA | 602 |
| OAS2/pro4 | CGATGTGCTCAAGGACAGAC | CCATTTCCCATTGCTCTCAG | 480 |
| OAS2/exon1 | CCTCCCATCCTACCATTCAC | GTCACACAGCTCTGGAGCAA | 394 |
| OAS2/exon2 | ATGAGCATCCCAATCAGGTC | GAGAGCGAGTCCAGGGTAGA | 597 |
| OAS2/exon3 | TGACCGATTAAGCAAGAGTCG | TGTTGCACTTTACTGGATGTCA | 379 |
| OAS2/exon4 | TAAGAGAGGTCCCTGCTCCA | GGACCTCCCCTCTTTCAGAC | 595 |
| OAS2/exon5 | GAGGGGCTTGATGTCGTAGA | CTCTGCCAACACCCTGATTT | 511 |
| OAS2/exon6 | TTGTGTGCCACTCCAAACTC | TAACTCCTGGCTTCCCTTCC | 500 |
| OAS2/exon7 | CAGAACTTGGCCTTGGAAAC | CCCATGCTTGGAAAAGAGAA | 594 |
| OAS2/exon8 | GGAGATGCTCCCTGTGTCTT | GGGTTAGTGATGGGAACGTC | 499 |
| OAS2/exon9 | TCCAAGCTGCAGAGTGTGAG | GCCCCTGCTAGGTTTTATCC | 532 |
| OAS2/exon10-1 | CGCGGCCTCTAGAAAGTTAC | GGTTGTCACTGGGAGAGGAG | 593 |
| OAS2/exon10-2 | AGGCAGGTAACCCCAGATTC | GCACAAGAGAGCCTCCAAAC | 606 |
| OAS2/exon10-3 | TGGTTCTCTCCACCAGGTTC | GGATGGATCCTAGCTCCACA | 496 |
| OAS2/exon11-1 | GTAGGGAAGAGGTGGCCAAG | GACTGGAAGGAAAAAGGAGGA | 600 |
| OAS2/exon11-2 | GTCTTGCCTTGCTGAACTCC | AAGGCTAAAGCCCAAGGAAA | 484 |
| OAS2/exon11-3 | CCAGCCAACCCTTTCATTAG | CACATGCTTGGTCTTTCTGG | 450 |
| OAS3/pro1 | TATTGACCCTTCTGCGTTCC | GCACACCTGAGCTCTCTTCC | 607 |
| OAS3/pro2 | GGAGAGAGGACCTGGGAGTT | CGACCATGGTGTAGGTGCTA | 602 |
| OAS3/pro3 | CCAGCCAGACAACTTTCCAG | TCGGATTTCTGGTTTCGTTT | 609 |
| OAS3/exon1 | AAGTGGGTGTCAGGTCCAAG | TGTCAAGCGGGCTTAAGAGT | 615 |
| OAS3/exon2 | ACAGTCTCTCCCCAGCACAC | CCCAGGTCAAACAGCAAGTT | 490 |
| OAS3/exon3 | AAAGCTCCAGGCTCTCTTCC | TGCACAAGGGACATAAACAGA | 481 |
| OAS3/exon4 | GGCTGAAGCTACCAGTGAGG | CTAATGTGCATCTGGGCTGA | 580 |
| OAS3/exon5 | TCGAGGCTCAGAGAGGGTAA | GCCTCCCAATGTGTTGAGAT | 451 |
| OAS3/exon6 | GGCACCTTTTTCAACTCTGC | CCAGCAAATCATCCTTCCTG | 654 |
| OAS3/exon7 | CATGCAGTCGACCTTTGTCA | TCAATGCTTGGTCAAGTAGGAG | 545 |
| OAS3/exon8 | CCACTGTGCAGTTACTGGATG | AAGCAAAGGAGGGGAGATGT | 508 |
| OAS3/exon9 | CTAGGCAAAAGCCACGTCTG | GGAAGCAGAGTGAGGAGTGG | 606 |
| OAS3/exon10 | CCCAATAGCTTTCCAACCAA | GCTGGGACGTAGCAATCTGT | 500 |
| OAS3/exon11 | CCTCAGAACCTACCCACCAG | GGACATATCTGGGCCCTCTC | 494 |
| OAS3/exon12 | TGACTTGTCCAAGGTCACACA | AAAGGGGCCCTCTTCTTTAG | 566 |
| OAS3/exon13 | ATCCCAGACCACCCTTAGGA | CTCCACCAACCCAGGATCT | 407 |
| OAS3/exon14 | AAAGTTTTGGGGTTGCTTTG | ATTGACCGTTTGTCCCTCAG | 490 |
| OAS3/exon15 | TCTAGCCCCTGCAAAGTGTT | ATGGGAAAATGGAAGCACAG | 395 |
| OAS3/exon16-1 | ATCTGTGGTGCCAAAGGAAG | GCAGGAGGAAAGATGTGAGC | 591 |
| OAS3/exon16-2 | CCTCCCATGGCTTACACACT | CATAGCATGCATGTCCCAAC | 599 |
| OAS3/exon16-3 | CAGGAGAGCATGCCCATATT | GAGAGCCTCCTGGATGGAC | 259 |
| OAS3/exon16-4 | GAGATTCTGCATCCCCACAG | TAGACCCACTCCCTCATCCA | 609 |
| OAS3/exon16-5 | CTCTGGCTGCAGAGGAGTCT | GCCCTGAGCTCTTGGATATG | 597 |
| OAS3/exon16-6 | AACGCTAAGGGCACCTTCTT | GGGTGTTTTTAGGGTGACCA | 603 |
| OAS3/exon16-7 | CACCCTGGTGTTGGCATATT | AGAAGGGTGAGAGCTTTATGG | 634 |

Sequencing results were analyzed by Genalys Software (Takahashi et al., J. Bioinform. Comput Biol., 2003, 1, 253-265). OAS3-R381S was genotyped by TaqMan assay using ABI Prism 7000 Sequence Detection System, with recommended protocols. The codon 381 of the more common variant of *OAS3* sequence is AGG which corresponds to a arginine. The polymorphism *OAS3*-R381S, named rs2285933, has a G to C substitution at the third nucleotide of codon 381 of *OAS3* (AGG to AGC) that changes amino acid from arginine to serine. The 2 variants of *OAS3*-(R38 and S381) mRNA, ORF and amino acid sequences correspond to SEQ ID NO: 28, 29, 30 (R381) and SEQ ID NO: 27, 1 and 2 (S381), respectively.

### c) Statistical analyses

We performed association study using allele count by Pearson chi-square test implemented in the Haploview program (Barrett et al., Bioinformatics, 2005, 21, 263-265). This program allows us to perform permutation test to obtain an empirical P value. We performed logistic regression analysis coding the genotype results according to the genetic model tested using STATA version 9.

### 2) Results

During 2000-2003, we have recruited 580 dengue patients from two hospitals (Siriraj, SI and Ramathibodi, RA) in Bangkok, and 170 from another hospital in Khonkaen province (KK), north-east of Thailand. We collected DNA samples from 512 blood donors from the same hospitals in Bangkok and 184 healthy volunteer from the north-east region of Thailand to represent gene frequency in general populations. Genetic study of part of these patients and controls has been reported previously (Sakuntabhai et al., Nature Genetics, 2005, 37, 507-513). Viral diagnosis was confirmed by serologic tests, and patients were classified into 4 groups according to WHO criteria: DF (no evidence of plasma leakage), and DHF1 (evidence of plasma leakage), DHF2 (with spontaneous bleeding) and DSS (with shock) (Table IV).

**Table IV: Disease severity in the studied population**

| | **2001-2003** | | | | **2004-2006** | | |
|---|---|---|---|---|---|---|---|
| | Hospital | | | Hospital | | | |
| Clinical Diagnosis | Siriraj | Ramathibodi | Khonkaen | Total | Ramathibodi | Khonkaen | Total |
| DF | 115 | 62 | 49 | 226 | 29 | 70 | 99 |
| DHF1 | 36 | 60 | 41 | 137 | 23 | 21 | 44 |
| DHF2 | 78 | 97 | 48 | 223 | 33 | 29 | 62 |
| DSS | 43 | 46 | 24 | 113 | 15 | 20 | 35 |
| unclassified | 7 | 36 | 8 | 51 | 1 | 13 | 14 |
| **Total** | **279** | **301** | **170** | **750** | **101** | **153** | **254** |

There were 51 patients who could not be classified as having DF or DHF according to WHO criteria (unclassified; UC). Proportion of patients with each severity category was significantly different among hospitals (P =1.5 x 10⁻⁸). This reflects difference in type of hospital (secondary care for KK or medical school for SI and RA) and inclusion criteria (fever less than 3 days in KK and suspected dengue cases in SI and RA).

There was an highly significant increased risk of plasma leakage (DHF1/DHF2/DSS versus DF) and spontaneous bleeding (DHF2/DSS versus DF/UC/DHF1) in patients who had a predominant IgG response compared to patients with predominant IgM response to dengue infection (P = 1.1 x 10⁻⁷ and P = 7.6 x 10⁻⁵, respectively) but only marginal significance for shock (DSS versus the rest, P = 0.038) (Table V).

**Table V: Disease severity and immune response**

| | Immune Response | |
|---|---|---|
| Clinical Diagnosis | IgM predominant | IgG predominant |
| DF | 51 | 162 |
| % | 53.7 | 27.1 |
| DHF1 | 15 | 121 |
| % | *15.8* | *20.2* |
| DHF2 | 19 | 199 |
| % | 20 | 33.3 |
| DSS | 8 | 99 |
| % | 8.4 | 16.6 |
| unclassified | 2 | 17 |
| % | *2.1* | 2.8 |
| **Total** | **95** | **598** |

These findings are consistent with the notion that individuals with IgG response to DV are predisposed to the more severe forms of the disease.

For screening of tagged polymorphisms, tests for association were performed using allele count on the risk of plasma leakage (DHF1/DHF2/DSS versus DF), risk of spontaneous hemorrhage (DHF2/DSS versus DF/UC/DHF1), risk of shock (DSS versus non-shock DV patients) in patients from the 2 hospitals in Bangkok (Table VI).

**Table VI: Association studies of OAS3-R381S in dengue patients**

| | Genotype | | | Freq | Allele | | | Case versus (vs) Control | Case, Control Ratios | p |
|---|---|---|---|---|---|---|---|---|---|---|
| OAS3- R381S | GG | CG | CC | CG + CC | G | C | MAF* | | | |
| **Siriraj** | | | | | | | | | | |
| control | 162 | 49 | 3 | **0.24** | 373 | 55 | **0.13** | | | |
| DF | 57 | 29 | 3 | **0.36** | 143 | 35 | **0.20** | Leak vs NoLeak | 38:276, 46:190 | **0.02** |
| DHF1 | 25 | 11 | 0 | **0.31** | 61 | 11 | **0.15** | Bleed vs NoBleed | 27:215, 59:263 | **0.02** |
| DHF2 | 56 | 21 | 1 | **0.28** | 133 | 23 | **0.15** | Shock vs NoShock | 4:82, 82:390 | **0.0027** |
| DSS | 40 | 2 | 1 | **0.07** | 82 | 4 | 0.05 | | | |
| | | | | | | | | | | |

| **Rama** | | | | | | | | | | |
|---|---|---|---|---|---|---|---|---|---|---|
| control | 210 | 82 | 4 | **0.29** | 502 | 90 | **0.15** | | | |
| DF | 24 | 11 | 2 | **0.35** | 59 | 15 | **0.20** | Leak vs NoLeak | 59:349, 21:103 | 0.50 |
| DHF1 | 42 | 18 | 0 | **0.30** | 102 | 18 | **0.15** | Bleed vs NoBleed | 41:247, 54:260 | 0.32 |
| DHF2 | 66 | 28 | 2 | **0.31** | 160 | 32 | **0.17** | Shock vs NoShock | 8:84, 86:420 | **0.04** |
| DSS | 39 | 6 | 1 | **0.15** | 84 | 8 | **0.09** | | | |

| **SirirajRama** | | | | | | | | | | |
|---|---|---|---|---|---|---|---|---|---|---|
| control | 372 | 131 | 7 | **0.27** | 875 | 145 | **0.14** | | | |
| DF | 81 | 40 | 5 | **0.36** | 202 | 50 | **0.20** | Leak vs NoLeak | 97:625, 67:293 | **0.0253** |
| DHF1 | 67 | 29 | 0 | **0.30** | 163 | 29 | **0.15** | Bleed vs NoBleed | 68:462, 113:523 | **0.0204** |
| DHF2 | 122 | 49 | 3 | **0.30** | 293 | 55 | **0.16** | Shock vs NoShock | 13:169, 168:816 | **7.10⁻⁴** |
| DSS | 79 | 8 | 2 | **0.11** | 166 | 12 | **0.07** | | | |

| **Khonkaen** | | | | | | | | | | |
|---|---|---|---|---|---|---|---|---|---|---|
| Control | 134 | 45 | 5 | **0.27** | 313 | 55 | **0.15** | | | |
| DF | 23 | 7 | 0 | **0.23** | 53 | 7 | **0.12** | Leak vs NoLeak | 33:181, 12:74 | 0.75 |
| DHF1 | 30 | 8 | 1 | **0.23** | 68 | 10 | **0.13** | Bleed vs NoBleed | 23:113, 27:153 | 0.64 |
| DHF2 | 26 | 17 | 1 | **0.41** | 69 | 19 | **0.22** | Shock vs NoShock | 4:44, 46:222 | 0.12 |
| DSS | 20 | 4 | 0 | **0.17** | 44 | 4 | **0.08** | | | |
| | | | | | | | | | | |

| **Total** | | | | | | | | | | |
|---|---|---|---|---|---|---|---|---|---|---|
| control | 506 | 176 | 12 | **0.27** | 1188 | 200 | **0.14** | | | |
| DF | 104 | 47 | 5 | **0.33** | 255 | 57 | **0.18** | Leak vs NoLeak | 130:806, 79:367 | 0.06 |
| DHF1 | 97 | 37 | 1 | **0.28** | 231 | 39 | **0.14** | Bleed vs NoBleed | 91:575, 140:676 | 0.07 |
| DHF2 | 148 | 66 | 4 | **0.32** | 362 | 74 | **0.17** | Shock vs NoShock | 16:210, 214:1032 | **0.0001** |
| DSS | 99 | 12 | 2 | **0.12** | 210 | 16 | **0.07** | | | |

| | | | | | | | | | | |
|---|---|---|---|---|---|---|---|---|---|---|
| *MAF: Mean Allele Frequency | | | | | | | | | | |

As shown in Table VI, none of the polymorphisms showed evidence of association with the risk of disease. However, one non-synonymous polymorphism of *OAS3* had highly significantly different allele count distributions depending on the severity of the disease. This variant (*OAS3-R381S: rs2285933)* is a G to C substitution at the 3^{rd} nucleotide of codon 381 of *OAS3,* that changes amino acid from arginine to serine. In both hospitals, the C allele of this polymorphism was more frequent in DF, DHF1 and DHF2, than in DSS. There was no significance difference in allele frequency between each group of patients and controls between the 2 hospitals. In combined analyses, the C allele had a frequency of 14% in general population, 20% in DF, and 15-16% in DHF1 and DHF2 and 7% in DSS. The difference between DSS and non-shock DV cases was highly significant (P = 7.10⁻⁴). We performed 10,000 permutations and obtained an empirical P value of 0.0032.

### Mode of inheritance

In order to find out mode of inheritance of the *OAS3-R381S,* we compared the likelihood of the models based on 3 different mode of inheritance: additive, dominant and recessive of the C allele using logistic regression on the risk of DSS. While recessive model of the C allele did not show significant association, highly significant level was obtained with dominant model of the C allele (< 10⁻⁵ P < 10⁻⁵, OR = 0.30 95CI = 0.17-0.53) and additive (17.93, P < 10⁻⁵). By log likelihood ratio test, the dominant model is significantly fitter than the additive model (P = 0.04). We therefore used dominant model for the rest of our analyses.

### Replication in the third cohort

We performed a replication study of the *OAS3-R381S* in patients from the 3^{rd} hospital (KK) during the same epidemic (2001-2003). The C allele showed less frequency in DSS group compared with DV patients without shock similar to previous results (8% versus 17% for C allele frequency and 17% versus 32% for CG+GG frequency). Although, this difference was not statistically significant because of small number of patients, higher significance association was obtained when combining all patients from the 3 hospitals (OR = 0.48, 95 CI = 0.31-0.75, P= 7 x 10⁻⁴, for dominant model of the C allele).

### OAS3 and type of immune response

We investigated further whether the effect of OAS3-R381S was depending on type of immune response to the virus. There was no significant association of the polymorphism and DSS in patients with predominant IgM immune response. However, significant and nearly significant association was obtained in patients with predominant IgG response in each hospital and combined (Chi2 = 19.41, OR = 0.27 95 CI = 0.14-0.53, P < 10-5) (Table VII).

**Table VII: OAS3-R381S in IgM and IgG immune response**

| | | IgM response | | | | | IgG response | | | | |
|---|---|---|---|---|---|---|---|---|---|---|---|
| | | Number | | Frequency (%) | | | Number | | Frequency (%) | | |
| Hospital and group | | GG | CG+CC | GG | CG+CC | P | GG | CG+CC | GG | CG+CC | *P* |
| Siriraj | | | | | | | | | | | |
| | Non-shock DENV | 28 | 14 | 66.7 | 33.3 | | 126 | 59 | 68.1 | 31.9 | |
| | DSS | 2 | 0 | 100 | 0 | 1 | 35 | 3 | 92.1 | 7.9 | 0.002 |
| | | | | | | | | | | | |
| Rama | | | | | | | | | | | |
| | Non-shock DV | 33 | 16 | 67.3 | 32.7 | | 128 | 53 | 70.7 | 29.3 | |
| | DSS | 3 | 2 | 60 | 40 | 1 | 33 | 5 | 86.8 | 13.2 | 0.044 |
| | | | | | | | | | | | |
| Khon-Kaen | | | | | | | | | | | |
| | Non-shock DV | 17 | 4 | 80.9 | 19.1 | | 81 | 41 | 66.4 | 33.6 | |
| | DSS | 0 | 1 | 0 | 100 | 0.23 | 20 | 3 | 87 | 13 | 0.052 |

### Association study of patients during 2004-2006 epidemics

During 2004-2006, we obtained additional patients from previous 2 hospitals (RA and KK, Table IV). Association study of *OAS3-R381S* did not reveal a significant association with all hypotheses studied, however, significant association remained when these patients were added to the original case/control study (OR = 0.48, 95 CI = 0.31-0.75, P= 7 x 10⁻⁴). This result prompted us to investigate the discordance of the results between the 2 periods.

One factor that could contribute to severity of dengue disease is a viral factor. We investigated prevalence of DV-4 serotypes during the 2001-2006 using data of patients from Siriraj Hospital and Khonkaen hospital of which we have data from 471 patients (Table VIII).

**Table VIII: Dengue virus serotype**

| A. Dengue serotype by year of study | | | | | |
|---|---|---|---|---|---|
| | DV serotype | | | | |
| Year | 1 | 2 | 3 | | 4 |
| | | 45 | 13 | | |
| 2001 | 57 (46%) | (36.3%) | (10.5%) | | 9 (7.2%) |
| | 52 | 50 | | | |
| 2002 | (46.4%) | (44.7%) | 3 (2.7%) | | 7 (6.2%) |
| 2003 | 53 (53%) | 36 (36%) | 4 (4%) | | 7 (7%) |
| | | | | | 12 |
| 2004 | 5 (13.5%) | 20 (54%) | 0 (0%) | | (32.4%) |
| | 11 | | 21 | | |
| 2005 | (30.6%) | 3 (8.3%) | 1 (2.8%) | | (58.3%) |
| | 33 | | 25 | | |
| 2006 | (53.2%) | 3 (4.8%) | 1 (1.6%) | | (40.3%) |

| B. Dengue serotype and clinical severity | | | | | |
|---|---|---|---|---|---|
| | DV serotype | | | | |
| Clinical Diagnosis | 1 | 2 | 3 | | 4 |
| DF | 93 | 46 | 10 | | 39 |
| DHF1 | 32 | 32 | 3 | | 8 |
| DHF2 | 50 | 40 | 7 | | 19 |
| DSS | 25 | 35 | 1 | | 8 |
| UC | 11 | 3 | 1 | | 6 |

| C. Dengue serotype and type of immune response | | | | | |
|---|---|---|---|---|---|
| | | DV serotype | | | |
| Type of Immune Response | | 1 | 2 | 3 | 4 |
| IgM predominant | | 44 | 5 | 7 | 0 |
| | % | *20.6* | 3 | 21.9 | 0 |
| IgG predominant | | 170 | 159 | 25 | 82 |
| | % | 79.4 | 97 | 78.1 | 100 |

DV1 and DV2 serotypes were predominant during 2001-2003 while in 2004 DV1 decreased while epidemic of DV4 started in 2004 and was, then, predominant until 2006 (Table 5A). Severity of dengue disease was significantly different among different serotypes of the virus (P = 0.012, Table 5B) with DV2 serotype having more severe cases than the other DV serotypes. Surprisingly, serotypes of the viruses were significantly different according to the type of immune response (Fisher exact P < 10-4, Table 5C) with DV2 and DV4 showing with the predominant IgG. These results were similar to data from Ministry of Public Health. We hypothesized that the effect of *OAS3-R381S* could be specific to some viral serotypes. We found significant dominant protective effect of OAS3-R381S against DSS only with DV2 (OR = 0.24, 95 CI = 0.08-0.74, P = 0.0045, Table IX).

**Table IX: OAS3-R381S and Dengue serotype**

| | Number | | Frequency (%) | | P |
|---|---|---|---|---|---|
| | GG | CG + CC | GG | CG + CC | |
| Serotype 1 | | | | | |
| non-shock DV | 125 | 57 | 68.7 | 31.3 | |
| DSS | 21 | 6 | 77.8 | 22.2 | 0.32 |

| Serotype 2 | | | | | |
|---|---|---|---|---|---|
| non-shock DV | 82 | 42 | 66.1 | 33.9 | |
| DSS | 32 | 4 | 88.9 | 11.1 | **0.0045** |

| Serotype 4 | | | | | |
|---|---|---|---|---|---|
| non-shock DV | 49 | 21 | 70 | 30 | |
| DSS | 6 | 2 | 75 | 25 | 0.77 |

Based on a systematic screening of OAS genes in DF/DHF/DSS patients and controls, we have shown association between polymorphisms of the *OAS3* gene and the risk of shock (DSS), and the severity of the disease in patients with dengue virus infection. The variant (*OAS3-R381S: rs2285933)* is a G to C substitution 3^{rd} nucleotide of codon 381 of *OAS3,* that changes amino acid from arginine to serine. The C variant is associated with dominant protection against DSS comparing with non-shock dengue disease, with frequency ratio of 0.13:0.32 (OR = 0.48, 95 CI = 0.31-0.75, P= 7 x 10⁻⁴). Furthermore, significant association was found exclusively in patients with predominant IgG response (OR = 0.46, 95 CI = 0.28-0.74, P = 8 x 10⁻⁴) and in patients infected by DV serotype 2 (OR = 0.24, 95 CI = 0.08-0.74, P = 0.0045).

These results support a functional role of the non-synonymous polymorphism OAS3-R381S and demonstrate a role for *OAS3* in the dengue pathogenesis, and interaction between a variant of *OAS3* and serotype of the virus.

Our finding may have important consequences for the prediction of disease severity in DV infected patients, and for the development of OAS3-based prophylaxis and therapy against infections by Dengue virus and other positive-sense single-stranded RNA viruses of major medical importance.

## Claims

1. A method *in vitro* for evaluating the susceptibility of an individual to a dengue virus infection, comprising: the detection of a polymorphism in the 2'-5'-oligoadenylate synthetase 3 gene in a nucleic acid sample obtained from said individual, wherein said polymorphism is the change of the Arginine 381 codon to a Serine codon (R381S).

2. The method of claim 1, wherein said detection uses an OAS3 specific oligonucleotide selected in the group consisting of the sequences SEQ ID NO: 62 to 86 and 118 to 142.

3. The method of claim 1 or claim 2, wherein the S381 variant is associated with a dominant protection against the risk of dengue shock syndrome in dengue virus infected patients.

4. The method of any one of claims 1 to 3, wherein said polymorphism is detected by direct sequencing of genomic DNA amplified with the pair of PCR primers SEQ ID NO: 70 and 126.


SEQUENCE LISTING

<110>  INSTITUT PASTEUR
       BREHIN, Anne-Claire
       SAKUNTABHAI, Anavaj
       DESPRES, Philippe
       CASADEMONT Isabelle
       JULIER Cécile
       CHUANSUMRIT Ampaiwan
       MALASIT Prida
       PAULOUS Sylvie

<120>  THE LARGE FORM OF HUMAN 2',5'-OLIGOADENYLATE SYNTHETASE OAS3 FOR
       PREVENTING OR TREATING INFECTION WITH POSITIVE-SENSE SINGLE-STRANDED
       RNA VIRUSES

<130>  226EP158

<160>  142

<170>  PatentIn version 3.5

<210>  1
<211>  3264
<212>  DNA
<213>  Homo sapiens


<220>
<221>  CDS
<222>  (1)..(3264)

<400>  1
atg gac ttg tac agc acc ccg gcc gct gcg ctg gac agg ttc gtg gcc        48
Met Asp Leu Tyr Ser Thr Pro Ala Ala Ala Leu Asp Arg Phe Val Ala
1               5                   10                  15

aga agg ctg cag ccg cgg aag gag ttc gta gag aag gcg cgg cgc gct        96
Arg Arg Leu Gln Pro Arg Lys Glu Phe Val Glu Lys Ala Arg Arg Ala
            20                  25                  30

ctg ggc gcc ctg gcc gct gcc ctg agg gag cgc ggg ggc cgc ctc ggt       144
Leu Gly Ala Leu Ala Ala Ala Leu Arg Glu Arg Gly Gly Arg Leu Gly
        35                  40                  45

gct gct gcc ccg cgg gtg ctg aaa act gtc aag gga ggc tcc tcg ggc       192
Ala Ala Ala Pro Arg Val Leu Lys Thr Val Lys Gly Gly Ser Ser Gly
    50                  55                  60

cgg ggc aca gct ctc aag ggt ggc tgt gat tct gaa ctt gtc atc ttc       240
Arg Gly Thr Ala Leu Lys Gly Gly Cys Asp Ser Glu Leu Val Ile Phe
65                  70                  75                  80

ctc gac tgc ttc aag agc tat gtg gac cag agg gcc cgc cgt gca gag       288
Leu Asp Cys Phe Lys Ser Tyr Val Asp Gln Arg Ala Arg Arg Ala Glu
                85                  90                  95

atc ctc agt gag atg cgg gca tcg ctg gaa tcc tgg tgg cag aac cca       336
Ile Leu Ser Glu Met Arg Ala Ser Leu Glu Ser Trp Trp Gln Asn Pro
            100                 105                 110

gtc cct ggt ctg aga ctc acg ttt cct gag cag agc gtg cct ggg gcc       384
Val Pro Gly Leu Arg Leu Thr Phe Pro Glu Gln Ser Val Pro Gly Ala
        115                 120                 125

ctg cag ttc cgc ctg aca tcc gta gat ctt gag gac tgg atg gat gtt 432
Leu Gln Phe Arg Leu Thr Ser Val Asp Leu Glu Asp Trp Met Asp Val
    130                 135                 140

agc ctg gtg cct gcc ttc aat gtc ctg ggt cag gcc ggc tcc ggc gtc 480
Ser Leu Val Pro Ala Phe Asn Val Leu Gly Gln Ala Gly Ser Gly Val
145                 150                 155                 160

aaa ccc aag cca caa gtc tac tct acc ctc ctc aac agt ggc tgc caa 528
Lys Pro Lys Pro Gln Val Tyr Ser Thr Leu Leu Asn Ser Gly Cys Gln
                165                 170                 175

ggg ggc gag cat gcg gcc tgc ttc aca gag ctg cgg agg aac ttt gtg 576
Gly Gly Glu His Ala Ala Cys Phe Thr Glu Leu Arg Arg Asn Phe Val
            180                 185                 190

aac att cgc cca gcc aag ttg aag aac cta atc ttg ctg gtg aag cac 624
Asn Ile Arg Pro Ala Lys Leu Lys Asn Leu Ile Leu Leu Val Lys His
        195                 200                 205

tgg tac cac cag gtg tgc cta cag ggg ttg tgg aag gag acg ctg ccc 672
Trp Tyr His Gln Val Cys Leu Gln Gly Leu Trp Lys Glu Thr Leu Pro
    210                 215                 220

ccg gtc tat gcc ctg gaa ttg ctg acc atc ttc gcc tgg gag cag ggc 720
Pro Val Tyr Ala Leu Glu Leu Leu Thr Ile Phe Ala Trp Glu Gln Gly
225                 230                 235                 240

tgt aag aag gat gct ttc agc cta gcc gaa ggc ctc cga act gtc ctg 768
Cys Lys Lys Asp Ala Phe Ser Leu Ala Glu Gly Leu Arg Thr Val Leu
                245                 250                 255

ggc ctg atc caa cag cat cag cac ctg tgt gtt ttc tgg act gtc aac 816
Gly Leu Ile Gln Gln His Gln His Leu Cys Val Phe Trp Thr Val Asn
            260                 265                 270

tat ggc ttc gag gac cct gca gtt ggg cag ttc ttg cag cgg cag ctt 864
Tyr Gly Phe Glu Asp Pro Ala Val Gly Gln Phe Leu Gln Arg Gln Leu
        275                 280                 285

aag aga ccc agg cct gtg atc ctg gac cca gct gac ccc aca tgg gac 912
Lys Arg Pro Arg Pro Val Ile Leu Asp Pro Ala Asp Pro Thr Trp Asp
    290                 295                 300

ctg ggg aat ggg gca gcc tgg cac tgg gat ttg cta gcc cag gag gca 960
Leu Gly Asn Gly Ala Ala Trp His Trp Asp Leu Leu Ala Gln Glu Ala
305                 310                 315                 320

gca tcc tgc tat gac cac cca tgc ttt ctg agg ggg atg ggg gac cca 1008
Ala Ser Cys Tyr Asp His Pro Cys Phe Leu Arg Gly Met Gly Asp Pro
                325                 330                 335

gtg cag tct tgg aag ggg ccg ggc ctt cca cgt gct gga tgc tca ggt 1056
Val Gln Ser Trp Lys Gly Pro Gly Leu Pro Arg Ala Gly Cys Ser Gly
            340                 345                 350

ttg ggc cac ccc atc cag cta gac cct aac cag aag acc cct gaa aac 1104
Leu Gly His Pro Ile Gln Leu Asp Pro Asn Gln Lys Thr Pro Glu Asn
        355                 360                 365

agc aag agc ctc aat gct gtg tac cca aga gca ggg agc aaa cct ccc 1152
Ser Lys Ser Leu Asn Ala Val Tyr Pro Arg Ala Gly Ser Lys Pro Pro
    370                 375                 380

tca tgc cca gct cct ggc ccc act ggg gca gcc agc atc gtc ccc tct 1200

Ser Cys Pro Ala Pro Gly Pro Thr Gly Ala Ala Ser Ile Val Pro Ser
385                 390                 395                 400

gtg ccg gga atg gcc ttg gac ctg tct cag atc ccc acc aag gag ctg      1248
Val Pro Gly Met Ala Leu Asp Leu Ser Gln Ile Pro Thr Lys Glu Leu
                405                 410                 415

gac cgc ttc atc cag gac cac ctg aag ccg agc ccc cag ttc cag gag      1296
Asp Arg Phe Ile Gln Asp His Leu Lys Pro Ser Pro Gln Phe Gln Glu
            420                 425                 430

cag gtg aaa aag gcc atc gac atc atc ttg cgc tgc ctc cat gag aac      1344
Gln Val Lys Lys Ala Ile Asp Ile Ile Leu Arg Cys Leu His Glu Asn
        435                 440                 445

tgt gtt cac aag gcc tca aga gtc agt aaa ggg ggc tca ttt ggc cgg      1392
Cys Val His Lys Ala Ser Arg Val Ser Lys Gly Gly Ser Phe Gly Arg
    450                 455                 460

ggc aca gac cta agg gat ggc tgt gat gtt gaa ctc atc atc ttc ctc      1440
Gly Thr Asp Leu Arg Asp Gly Cys Asp Val Glu Leu Ile Ile Phe Leu
465                 470                 475                 480

aac tgc ttc acg gac tac aag gac cag ggg ccc cgc cgc gca gag atc      1488
Asn Cys Phe Thr Asp Tyr Lys Asp Gln Gly Pro Arg Arg Ala Glu Ile
                485                 490                 495

ctt gat gag atg cga gcg cag cta gaa tcc tgg tgg cag gac cag gtg      1536
Leu Asp Glu Met Arg Ala Gln Leu Glu Ser Trp Trp Gln Asp Gln Val
            500                 505                 510

ccc agc ctg agc ctt cag ttt cct gag cag aat gtg cct gag gct ctg      1584
Pro Ser Leu Ser Leu Gln Phe Pro Glu Gln Asn Val Pro Glu Ala Leu
        515                 520                 525

cag ttc cag ctg gtg tcc aca gcc ctg aag agc tgg acg gat gtt agc      1632
Gln Phe Gln Leu Val Ser Thr Ala Leu Lys Ser Trp Thr Asp Val Ser
    530                 535                 540

ctg ctg cct gcc ttc gat gct gtg ggg cag ctc agt tct ggc acc aaa      1680
Leu Leu Pro Ala Phe Asp Ala Val Gly Gln Leu Ser Ser Gly Thr Lys
545                 550                 555                 560

cca aat ccc cag gtc tac tcg agg ctc ctc acc agt ggc tgc cag gag      1728
Pro Asn Pro Gln Val Tyr Ser Arg Leu Leu Thr Ser Gly Cys Gln Glu
                565                 570                 575

ggc gag cat aag gcc tgc ttc gca gag ctg cgg agg aac ttc atg aac      1776
Gly Glu His Lys Ala Cys Phe Ala Glu Leu Arg Arg Asn Phe Met Asn
            580                 585                 590

att cgc cct gtc aag ctg aag aac ctg att ctg ctg gtg aag cac tgg      1824
Ile Arg Pro Val Lys Leu Lys Asn Leu Ile Leu Leu Val Lys His Trp
        595                 600                 605

tac cgc cag gtt gcg gct cag aac aaa gga aaa gga cca gcc cct gcc      1872
Tyr Arg Gln Val Ala Ala Gln Asn Lys Gly Lys Gly Pro Ala Pro Ala
    610                 615                 620

tct ctg ccc cca gcc tat gcc ctg gag ctc ctc acc atc ttt gcc tgg      1920
Ser Leu Pro Pro Ala Tyr Ala Leu Glu Leu Leu Thr Ile Phe Ala Trp
625                 630                 635                 640

gag cag ggc tgc agg cag gat tgt ttc aac atg gcc caa ggc ttc cgg      1968
Glu Gln Gly Cys Arg Gln Asp Cys Phe Asn Met Ala Gln Gly Phe Arg

645                 650                 655

acg gtg ctg ggg ctc gtg caa cag cat cag cag ctc tgt gtc tac tgg         2016
Thr Val Leu Gly Leu Val Gln Gln His Gln Gln Leu Cys Val Tyr Trp
            660                 665                 670

acg gtc aac tat agc act gag gac cca gcc atg aga atg cac ctt ctt         2064
Thr Val Asn Tyr Ser Thr Glu Asp Pro Ala Met Arg Met His Leu Leu
        675                 680                 685

ggc cag ctt cga aaa ccc aga ccc ctg gtc ctg gac ccc gct gat ccc         2112
Gly Gln Leu Arg Lys Pro Arg Pro Leu Val Leu Asp Pro Ala Asp Pro
    690                 695                 700

acc tgg aac gtg ggc cac ggt agc tgg gag ctg ttg gcc cag gaa gca         2160
Thr Trp Asn Val Gly His Gly Ser Trp Glu Leu Leu Ala Gln Glu Ala
705                 710                 715                 720

gca gcg ctg ggg atg cag gcc tgc ttt ctg agt aga gac ggg aca tct         2208
Ala Ala Leu Gly Met Gln Ala Cys Phe Leu Ser Arg Asp Gly Thr Ser
                725                 730                 735

gtg cag ccc tgg gat gtg atg cca gcc ctc ctt tac caa acc cca gct         2256
Val Gln Pro Trp Asp Val Met Pro Ala Leu Leu Tyr Gln Thr Pro Ala
            740                 745                 750

ggg gac ctt gac aag ttc atc agt gaa ttt ctc cag ccc aac cgc cag         2304
Gly Asp Leu Asp Lys Phe Ile Ser Glu Phe Leu Gln Pro Asn Arg Gln
        755                 760                 765

ttc ctg gcc cag gtg aac aag gcc gtt gat acc atc tgt tca ttt ttg         2352
Phe Leu Ala Gln Val Asn Lys Ala Val Asp Thr Ile Cys Ser Phe Leu
    770                 775                 780

aag gaa aac tgc ttc cgg aat tct ccc atc aaa gtg atc aag gtg gtc         2400
Lys Glu Asn Cys Phe Arg Asn Ser Pro Ile Lys Val Ile Lys Val Val
785                 790                 795                 800

aag ggt ggc tct tca gcc aaa ggc aca gct ctg cga ggc cgc tca gat         2448
Lys Gly Gly Ser Ser Ala Lys Gly Thr Ala Leu Arg Gly Arg Ser Asp
                805                 810                 815

gcc gac ctc gtg gtg ttc ctc agc tgc ttc agc cag ttc act gag cag         2496
Ala Asp Leu Val Val Phe Leu Ser Cys Phe Ser Gln Phe Thr Glu Gln
            820                 825                 830

ggc aac aag cgg gcc gag atc atc tcc gag atc cga gcc cag ctg gag         2544
Gly Asn Lys Arg Ala Glu Ile Ile Ser Glu Ile Arg Ala Gln Leu Glu
        835                 840                 845

gca tgt caa cag gag cgg cag ttc gag gtc aag ttt gaa gtc tcc aaa         2592
Ala Cys Gln Gln Glu Arg Gln Phe Glu Val Lys Phe Glu Val Ser Lys
    850                 855                 860

tgg gag aat ccc cgc gtg ctg agc ttc tca ctg aca tcc cag acg atg         2640
Trp Glu Asn Pro Arg Val Leu Ser Phe Ser Leu Thr Ser Gln Thr Met
865                 870                 875                 880

ctg gac cag agt gtg gac ttt gat gtg ctg cca gcc ttt gac gcc cta         2688
Leu Asp Gln Ser Val Asp Phe Asp Val Leu Pro Ala Phe Asp Ala Leu
                885                 890                 895

ggc cag ctg gtc tct ggc tcc agg ccc agc tct caa gtc tac gtc gac         2736
Gly Gln Leu Val Ser Gly Ser Arg Pro Ser Ser Gln Val Tyr Val Asp
            900                 905                 910

ctc atc cac agc tac agc aat gcg ggc gag tac tcc acc tgc ttc aca 2784
Leu Ile His Ser Tyr Ser Asn Ala Gly Glu Tyr Ser Thr Cys Phe Thr
        915                 920                 925

gag cta caa cgg gac ttc atc atc tct cgc cct acc aag ctg aag agc 2832
Glu Leu Gln Arg Asp Phe Ile Ile Ser Arg Pro Thr Lys Leu Lys Ser
    930                 935                 940

ctg atc cgg ctg gtg aag cac tgg tac cag cag tgt acc aag atc tcc 2880
Leu Ile Arg Leu Val Lys His Trp Tyr Gln Gln Cys Thr Lys Ile Ser
945                 950                 955                 960

aag ggg aga ggc tcc cta ccc cca cag cac ggg ctg gaa ctc ctg act 2928
Lys Gly Arg Gly Ser Leu Pro Pro Gln His Gly Leu Glu Leu Leu Thr
                965                 970                 975

gtg tat gcc tgg gag cag ggc ggg aag gac tcc cag ttc aac atg gct 2976
Val Tyr Ala Trp Glu Gln Gly Gly Lys Asp Ser Gln Phe Asn Met Ala
            980                 985                 990

gag ggc ttc cgc acg gtc ctg gag ctg gtc acc cag tac cgc cag ctc 3024
Glu Gly Phe Arg Thr Val Leu Glu Leu Val Thr Gln Tyr Arg Gln Leu
        995                 1000                1005

tgt atc tac tgg acc atc aac tac aac gcc aag gac aag act gtt 3069
Cys Ile Tyr Trp Thr Ile Asn Tyr Asn Ala Lys Asp Lys Thr Val
    1010                1015                1020

gga gac ttc ctg aaa cag cag ctt cag aag ccc agg cct atc atc 3114
Gly Asp Phe Leu Lys Gln Gln Leu Gln Lys Pro Arg Pro Ile Ile
    1025                1030                1035

ctg gat ccg gct gac ccg aca ggc aac ctg ggc cac aat gcc cgc 3159
Leu Asp Pro Ala Asp Pro Thr Gly Asn Leu Gly His Asn Ala Arg
    1040                1045                1050

tgg gac ctg ctg gcc aag gaa gct gca gcc tgc aca tct gcc ctg 3204
Trp Asp Leu Leu Ala Lys Glu Ala Ala Ala Cys Thr Ser Ala Leu
    1055                1060                1065

tgc tgc atg gga cgg aat ggc atc ccc atc cag cca tgg cca gtg 3249
Cys Cys Met Gly Arg Asn Gly Ile Pro Ile Gln Pro Trp Pro Val
    1070                1075                1080

aag gct gct gtg tga 3264
Lys Ala Ala Val
    1085

<210> 2
<211> 1087
<212> PRT
<213> Homo sapiens

<400> 2

Met Asp Leu Tyr Ser Thr Pro Ala Ala Ala Leu Asp Arg Phe Val Ala
1               5                   10                  15

Arg Arg Leu Gln Pro Arg Lys Glu Phe Val Glu Lys Ala Arg Arg Ala
            20                  25                  30

Leu Gly Ala Leu Ala Ala Ala Leu Arg Glu Arg Gly Gly Arg Leu Gly
        35                  40                  45

Ala Ala Ala Pro Arg Val Leu Lys Thr Val Lys Gly Gly Ser Ser Gly
    50                  55                  60

Arg Gly Thr Ala Leu Lys Gly Gly Cys Asp Ser Glu Leu Val Ile Phe
65                  70                  75                  80

Leu Asp Cys Phe Lys Ser Tyr Val Asp Gln Arg Ala Arg Arg Ala Glu
                85                  90                  95

Ile Leu Ser Glu Met Arg Ala Ser Leu Glu Ser Trp Trp Gln Asn Pro
            100                 105                 110

Val Pro Gly Leu Arg Leu Thr Phe Pro Glu Gln Ser Val Pro Gly Ala
        115                 120                 125

Leu Gln Phe Arg Leu Thr Ser Val Asp Leu Glu Asp Trp Met Asp Val
    130                 135                 140

Ser Leu Val Pro Ala Phe Asn Val Leu Gly Gln Ala Gly Ser Gly Val
145                 150                 155                 160

Lys Pro Lys Pro Gln Val Tyr Ser Thr Leu Leu Asn Ser Gly Cys Gln
                165                 170                 175

Gly Gly Glu His Ala Ala Cys Phe Thr Glu Leu Arg Arg Asn Phe Val
            180                 185                 190

Asn Ile Arg Pro Ala Lys Leu Lys Asn Leu Ile Leu Leu Val Lys His
        195                 200                 205

Trp Tyr His Gln Val Cys Leu Gln Gly Leu Trp Lys Glu Thr Leu Pro
    210                 215                 220

Pro Val Tyr Ala Leu Glu Leu Leu Thr Ile Phe Ala Trp Glu Gln Gly
225                 230                 235                 240

Cys Lys Lys Asp Ala Phe Ser Leu Ala Glu Gly Leu Arg Thr Val Leu
                245                 250                 255

Gly Leu Ile Gln Gln His Gln His Leu Cys Val Phe Trp Thr Val Asn
            260                 265                 270

Tyr Gly Phe Glu Asp Pro Ala Val Gly Gln Phe Leu Gln Arg Gln Leu
        275                 280                 285

Lys Arg Pro Arg Pro Val Ile Leu Asp Pro Ala Asp Pro Thr Trp Asp

290                 295                 300

Leu Gly Asn Gly Ala Ala Trp His Trp Asp Leu Leu Ala Gln Glu Ala
305                 310                 315                 320

Ala Ser Cys Tyr Asp His Pro Cys Phe Leu Arg Gly Met Gly Asp Pro
                325                 330                 335

Val Gln Ser Trp Lys Gly Pro Gly Leu Pro Arg Ala Gly Cys Ser Gly
            340                 345                 350

Leu Gly His Pro Ile Gln Leu Asp Pro Asn Gln Lys Thr Pro Glu Asn
        355                 360                 365

Ser Lys Ser Leu Asn Ala Val Tyr Pro Arg Ala Gly Ser Lys Pro Pro
    370                 375                 380

Ser Cys Pro Ala Pro Gly Pro Thr Gly Ala Ala Ser Ile Val Pro Ser
385                 390                 395                 400

Val Pro Gly Met Ala Leu Asp Leu Ser Gln Ile Pro Thr Lys Glu Leu
                405                 410                 415

Asp Arg Phe Ile Gln Asp His Leu Lys Pro Ser Pro Gln Phe Gln Glu
            420                 425                 430

Gln Val Lys Lys Ala Ile Asp Ile Ile Leu Arg Cys Leu His Glu Asn
        435                 440                 445

Cys Val His Lys Ala Ser Arg Val Ser Lys Gly Gly Ser Phe Gly Arg
    450                 455                 460

Gly Thr Asp Leu Arg Asp Gly Cys Asp Val Glu Leu Ile Ile Phe Leu
465                 470                 475                 480

Asn Cys Phe Thr Asp Tyr Lys Asp Gln Gly Pro Arg Arg Ala Glu Ile
                485                 490                 495

Leu Asp Glu Met Arg Ala Gln Leu Glu Ser Trp Trp Gln Asp Gln Val
            500                 505                 510

Pro Ser Leu Ser Leu Gln Phe Pro Glu Gln Asn Val Pro Glu Ala Leu
        515                 520                 525

Gln Phe Gln Leu Val Ser Thr Ala Leu Lys Ser Trp Thr Asp Val Ser
    530                 535                 540

Leu Leu Pro Ala Phe Asp Ala Val Gly Gln Leu Ser Ser Gly Thr Lys
545                 550                 555                 560

Pro Asn Pro Gln Val Tyr Ser Arg Leu Leu Thr Ser Gly Cys Gln Glu
                565                 570                 575

Gly Glu His Lys Ala Cys Phe Ala Glu Leu Arg Arg Asn Phe Met Asn
            580                 585                 590

Ile Arg Pro Val Lys Leu Lys Asn Leu Ile Leu Leu Val Lys His Trp
        595                 600                 605

Tyr Arg Gln Val Ala Ala Gln Asn Lys Gly Lys Gly Pro Ala Pro Ala
    610                 615                 620

Ser Leu Pro Pro Ala Tyr Ala Leu Glu Leu Leu Thr Ile Phe Ala Trp
625                 630                 635                 640

Glu Gln Gly Cys Arg Gln Asp Cys Phe Asn Met Ala Gln Gly Phe Arg
                645                 650                 655

Thr Val Leu Gly Leu Val Gln Gln His Gln Gln Leu Cys Val Tyr Trp
            660                 665                 670

Thr Val Asn Tyr Ser Thr Glu Asp Pro Ala Met Arg Met His Leu Leu
        675                 680                 685

Gly Gln Leu Arg Lys Pro Arg Pro Leu Val Leu Asp Pro Ala Asp Pro
    690                 695                 700

Thr Trp Asn Val Gly His Gly Ser Trp Glu Leu Leu Ala Gln Glu Ala
705                 710                 715                 720

Ala Ala Leu Gly Met Gln Ala Cys Phe Leu Ser Arg Asp Gly Thr Ser
                725                 730                 735

Val Gln Pro Trp Asp Val Met Pro Ala Leu Leu Tyr Gln Thr Pro Ala
            740                 745                 750

Gly Asp Leu Asp Lys Phe Ile Ser Glu Phe Leu Gln Pro Asn Arg Gln
        755                 760                 765

Phe Leu Ala Gln Val Asn Lys Ala Val Asp Thr Ile Cys Ser Phe Leu
    770                 775                 780

Lys Glu Asn Cys Phe Arg Asn Ser Pro Ile Lys Val Ile Lys Val Val
785                 790                 795                 800

Lys Gly Gly Ser Ser Ala Lys Gly Thr Ala Leu Arg Gly Arg Ser Asp
                805                 810                 815

Ala Asp Leu Val Val Phe Leu Ser Cys Phe Ser Gln Phe Thr Glu Gln
            820                 825                 830

Gly Asn Lys Arg Ala Glu Ile Ile Ser Glu Ile Arg Ala Gln Leu Glu
        835                 840                 845

Ala Cys Gln Gln Glu Arg Gln Phe Glu Val Lys Phe Glu Val Ser Lys
    850                 855                 860

Trp Glu Asn Pro Arg Val Leu Ser Phe Ser Leu Thr Ser Gln Thr Met
865                 870                 875                 880

Leu Asp Gln Ser Val Asp Phe Asp Val Leu Pro Ala Phe Asp Ala Leu
                885                 890                 895

Gly Gln Leu Val Ser Gly Ser Arg Pro Ser Ser Gln Val Tyr Val Asp
            900                 905                 910

Leu Ile His Ser Tyr Ser Asn Ala Gly Glu Tyr Ser Thr Cys Phe Thr
        915                 920                 925

Glu Leu Gln Arg Asp Phe Ile Ile Ser Arg Pro Thr Lys Leu Lys Ser
    930                 935                 940

Leu Ile Arg Leu Val Lys His Trp Tyr Gln Gln Cys Thr Lys Ile Ser
945                 950                 955                 960

Lys Gly Arg Gly Ser Leu Pro Pro Gln His Gly Leu Glu Leu Leu Thr
                965                 970                 975

Val Tyr Ala Trp Glu Gln Gly Gly Lys Asp Ser Gln Phe Asn Met Ala
            980                 985                 990

Glu Gly Phe Arg Thr Val Leu Glu Leu Val Thr Gln Tyr Arg Gln Leu
        995                 1000                1005

Cys Ile Tyr Trp Thr Ile Asn Tyr Asn Ala Lys Asp Lys Thr Val
    1010                1015                1020

Gly Asp Phe Leu Lys Gln Gln Leu Gln Lys Pro Arg Pro Ile Ile
    1025                1030                1035

Leu Asp Pro Ala Asp Pro Thr Gly Asn Leu Gly His Asn Ala Arg
    1040                1045                1050

Trp Asp Leu Leu Ala Lys Glu Ala Ala Ala Cys Thr Ser Ala Leu
    1055                1060                1065

Cys Cys Met Gly Arg Asn Gly Ile Pro Ile Gln Pro Trp Pro Val
    1070                1075                1080

Lys Ala Ala Val
    1085

<210> 3
<211> 338
<212> PRT
<213> artificial sequence

<220>
<223> OAS domain I

<400> 3

Thr Pro Ala Ala Ala Leu Asp Arg Phe Val Ala Arg Arg Leu Gln Pro
1               5                   10                  15

Arg Lys Glu Phe Val Glu Lys Ala Arg Arg Ala Leu Gly Ala Leu Ala
            20                  25                  30

Ala Ala Leu Arg Glu Arg Gly Gly Arg Leu Gly Ala Ala Ala Pro Arg
        35                  40                  45

Val Leu Lys Thr Val Lys Gly Gly Ser Ser Gly Arg Gly Thr Ala Leu
    50                  55                  60

Lys Gly Gly Cys Asp Ser Glu Leu Val Ile Phe Leu Asp Cys Phe Lys
65                  70                  75                  80

Ser Tyr Val Asp Gln Arg Ala Arg Arg Ala Glu Ile Leu Ser Glu Met
                85                  90                  95

Arg Ala Ser Leu Glu Ser Trp Trp Gln Asn Pro Val Pro Gly Leu Arg
            100                 105                 110

Leu Thr Phe Pro Glu Gln Ser Val Pro Gly Ala Leu Gln Phe Arg Leu
        115                 120                 125

Thr Ser Val Asp Leu Glu Asp Trp Met Asp Val Ser Leu Val Pro Ala
    130                 135                 140

Phe Asn Val Leu Gly Gln Ala Gly Ser Gly Val Lys Pro Lys Pro Gln
145                 150                 155                 160

Val Tyr Ser Thr Leu Leu Asn Ser Gly Cys Gln Gly Gly Glu His Ala
                165                 170                 175

Ala Cys Phe Thr Glu Leu Arg Arg Asn Phe Val Asn Ile Arg Pro Ala
            180                 185                 190

Lys Leu Lys Asn Leu Ile Leu Leu Val Lys His Trp Tyr His Gln Val
        195                 200                 205

Cys Leu Gln Gly Leu Trp Lys Glu Thr Leu Pro Pro Val Tyr Ala Leu
    210                 215                 220

Glu Leu Leu Thr Ile Phe Ala Trp Glu Gln Gly Cys Lys Lys Asp Ala
225                 230                 235                 240

Phe Ser Leu Ala Glu Gly Leu Arg Thr Val Leu Gly Leu Ile Gln Gln
                245                 250                 255

His Gln His Leu Cys Val Phe Trp Thr Val Asn Tyr Gly Phe Glu Asp
            260                 265                 270

Pro Ala Val Gly Gln Phe Leu Gln Arg Gln Leu Lys Arg Pro Arg Pro
        275                 280                 285

Val Ile Leu Asp Pro Ala Asp Pro Thr Trp Asp Leu Gly Asn Gly Ala
    290                 295                 300

Ala Trp His Trp Asp Leu Leu Ala Gln Glu Ala Ala Ser Cys Tyr Asp
305                 310                 315                 320

His Pro Cys Phe Leu Arg Gly Met Gly Asp Pro Val Gln Ser Trp Lys
                325                 330                 335

Gly Pro

<210>  4
<211>  332
<212>  PRT
<213>  artificial sequence

<220>
<223>  OAS domain II

<400>  4

Ile Pro Thr Lys Glu Leu Asp Arg Phe Ile Gln Asp His Leu Lys Pro
1               5                   10                  15

Ser Pro Gln Phe Gln Glu Gln Val Lys Lys Ala Ile Asp Ile Ile Leu
            20                  25                  30

Arg Cys Leu His Glu Asn Cys Val His Lys Ala Ser Arg Val Ser Lys
        35                  40                  45

Gly Gly Ser Phe Gly Arg Gly Thr Asp Leu Arg Asp Gly Cys Asp Val
    50                  55                  60

Glu Leu Ile Ile Phe Leu Asn Cys Phe Thr Asp Tyr Lys Asp Gln Gly
65                  70                  75                  80

Pro Arg Arg Ala Glu Ile Leu Asp Glu Met Arg Ala Gln Leu Glu Ser
                85                  90                  95

Trp Trp Gln Asp Gln Val Pro Ser Leu Ser Leu Gln Phe Pro Glu Gln
            100                 105                 110

Asn Val Pro Glu Ala Leu Gln Phe Gln Leu Val Ser Thr Ala Leu Lys
        115                 120                 125

Ser Trp Thr Asp Val Ser Leu Leu Pro Ala Phe Asp Ala Val Gly Gln
    130                 135                 140

Leu Ser Ser Gly Thr Lys Pro Asn Pro Gln Val Tyr Ser Arg Leu Leu
145                 150                 155                 160

Thr Ser Gly Cys Gln Glu Gly Glu His Lys Ala Cys Phe Ala Glu Leu
                165                 170                 175

Arg Arg Asn Phe Met Asn Ile Arg Pro Val Lys Leu Lys Asn Leu Ile
            180                 185                 190

Leu Leu Val Lys His Trp Tyr Arg Gln Val Ala Ala Gln Asn Lys Gly
        195                 200                 205

Lys Gly Pro Ala Pro Ala Ser Leu Pro Pro Ala Tyr Ala Leu Glu Leu
    210                 215                 220

Leu Thr Ile Phe Ala Trp Glu Gln Gly Cys Arg Gln Asp Cys Phe Asn
225                 230                 235                 240

Met Ala Gln Gly Phe Arg Thr Val Leu Gly Leu Val Gln Gln His Gln
                245                 250                 255

Gln Leu Cys Val Tyr Trp Thr Val Asn Tyr Ser Thr Glu Asp Pro Ala
            260                 265                 270

Met Arg Met His Leu Leu Gly Gln Leu Arg Lys Pro Arg Pro Leu Val
        275                 280                 285

Leu Asp Pro Ala Asp Pro Thr Trp Asn Val Gly His Gly Ser Trp Glu
    290                 295                 300

Leu Leu Ala Gln Glu Ala Ala Ala Leu Gly Met Gln Ala Cys Phe Leu
305                 310                 315                 320

Ser Arg Asp Gly Thr Ser Val Gln Pro Trp Asp Val
                325                 330

<210>  5
<211>  335
<212>  PRT
<213>  artificial sequence

<220>
<223>  OAS domain III

<400>  5

Thr Pro Ala Gly Asp Leu Asp Lys Phe Ile Ser Glu Phe Leu Gln Pro
1               5                   10                  15

Asn Arg Gln Phe Leu Ala Gln Val Asn Lys Ala Val Asp Thr Ile Cys
            20                  25                  30

Ser Phe Leu Lys Glu Asn Cys Phe Arg Asn Ser Pro Ile Lys Val Ile
        35                  40                  45

Lys Val Val Lys Gly Gly Ser Ser Ala Lys Gly Thr Ala Leu Arg Gly
    50                  55                  60

Arg Ser Asp Ala Asp Leu Val Val Phe Leu Ser Cys Phe Ser Gln Phe
65                  70                  75                  80

Thr Glu Gln Gly Asn Lys Arg Ala Glu Ile Ile Ser Glu Ile Arg Ala
                85                  90                  95

Gln Leu Glu Ala Cys Gln Gln Glu Arg Gln Phe Glu Val Lys Phe Glu
            100                 105                 110

Val Ser Lys Trp Glu Asn Pro Arg Val Leu Ser Phe Ser Leu Thr Ser
        115                 120                 125

Gln Thr Met Leu Asp Gln Ser Val Asp Phe Asp Val Leu Pro Ala Phe
    130                 135                 140

Asp Ala Leu Gly Gln Leu Val Ser Gly Ser Arg Pro Ser Ser Gln Val
145                 150                 155                 160

Tyr Val Asp Leu Ile His Ser Tyr Ser Asn Ala Gly Glu Tyr Ser Thr
                165                 170                 175

Cys Phe Thr Glu Leu Gln Arg Asp Phe Ile Ile Ser Arg Pro Thr Lys
            180                 185                 190

Leu Lys Ser Leu Ile Arg Leu Val Lys His Trp Tyr Gln Gln Cys Thr
        195                 200                 205

Lys Ile Ser Lys Gly Arg Gly Ser Leu Pro Pro Gln His Gly Leu Glu
    210                 215                 220

Leu Leu Thr Val Tyr Ala Trp Glu Gln Gly Gly Lys Asp Ser Gln Phe
225                 230                 235                 240

Asn Met Ala Glu Gly Phe Arg Thr Val Leu Glu Leu Val Thr Gln Tyr
                245                 250                 255

Arg Gln Leu Cys Ile Tyr Trp Thr Ile Asn Tyr Asn Ala Lys Asp Lys
            260                 265                 270

Thr Val Gly Asp Phe Leu Lys Gln Gln Leu Gln Lys Pro Arg Pro Ile
        275                 280                 285

Ile Leu Asp Pro Ala Asp Pro Thr Gly Asn Leu Gly His Asn Ala Arg
    290                 295                 300

Trp Asp Leu Leu Ala Lys Glu Ala Ala Ala Cys Thr Ser Ala Leu Cys
305                 310                 315                 320

Cys Met Gly Arg Asn Gly Ile Pro Ile Gln Pro Trp Pro Val Lys
                325                 330                 335

<210>  6
<211>  3264
<212>  DNA
<213>  artificial sequence

<220>
<223>  human recombinant OAS3

<220>
<221>  CDS
<222>  (1)..(3264)

<400>  6
atg gac ttg tac agc acc ccg gcc gct gcg ctg gac agg ttc gtg gcc        48
Met Asp Leu Tyr Ser Thr Pro Ala Ala Ala Leu Asp Arg Phe Val Ala
1               5                   10                  15

aga aag ctg cag ccg cgg aag gag ttc gta gag aag gcg cgg cgc gct        96
Arg Lys Leu Gln Pro Arg Lys Glu Phe Val Glu Lys Ala Arg Arg Ala
            20                  25                  30

ctg ggc gcc ctg gcc gct gct ctg agg gag cgc ggg ggc cgc ctc ggt       144
Leu Gly Ala Leu Ala Ala Ala Leu Arg Glu Arg Gly Gly Arg Leu Gly
        35                  40                  45

gct gct gcc ccg cgg gtg ctg aaa act gtc aag gga ggc tcc tcg ggc       192
Ala Ala Ala Pro Arg Val Leu Lys Thr Val Lys Gly Gly Ser Ser Gly
    50                  55                  60

cgg ggc aca gct ctc aag ggt ggc tgt gat tct gaa ctt gtc atc ttc       240

Arg Gly Thr Ala Leu Lys Gly Gly Cys Asp Ser Glu Leu Val Ile Phe
65                  70                  75                  80

ctc gac tgc ttc aag agc tat gtg gac cag agg gcc cgc cgt gca gag      288
Leu Asp Cys Phe Lys Ser Tyr Val Asp Gln Arg Ala Arg Arg Ala Glu
                85                  90                  95

atc ctc agt gag atg cgg gca tcg ctg gaa tcc tgg tgg cag aac cca      336
Ile Leu Ser Glu Met Arg Ala Ser Leu Glu Ser Trp Trp Gln Asn Pro
            100                 105                 110

gtc cct ggt ctg aga ctc acg ttt cct gag cag agc gtg cct ggg gcc      384
Val Pro Gly Leu Arg Leu Thr Phe Pro Glu Gln Ser Val Pro Gly Ala
        115                 120                 125

ctg cag ttc cgc ctg aca tcc gta gat ctt gag gac tgg atg gat gtt      432
Leu Gln Phe Arg Leu Thr Ser Val Asp Leu Glu Asp Trp Met Asp Val
    130                 135                 140

agc ctg gtg cct gcc ttc aat gtc ctg ggt cag gcc ggc tcc ggc gtc      480
Ser Leu Val Pro Ala Phe Asn Val Leu Gly Gln Ala Gly Ser Gly Val
145                 150                 155                 160

aaa ccc aag cca caa gtc tac tct acc ctc ctc aac agt ggc tgc caa      528
Lys Pro Lys Pro Gln Val Tyr Ser Thr Leu Leu Asn Ser Gly Cys Gln
                165                 170                 175

ggg ggc gag cat gcg gcc tgc ttc aca gag ctg cgg agg aac ttt gtg      576
Gly Gly Glu His Ala Ala Cys Phe Thr Glu Leu Arg Arg Asn Phe Val
            180                 185                 190

aac att cgc cca gcc aag ttg aag aac cta atc ttg ctg gtg aag cac      624
Asn Ile Arg Pro Ala Lys Leu Lys Asn Leu Ile Leu Leu Val Lys His
        195                 200                 205

tgg tac cac cag gtg tgc cta cag ggg ttg tgg aag gag acg ctg ccc      672
Trp Tyr His Gln Val Cys Leu Gln Gly Leu Trp Lys Glu Thr Leu Pro
    210                 215                 220

ccg gtc tat gcc ctg gaa ttg ctg acc atc ttc gcc tgg gag cag ggc      720
Pro Val Tyr Ala Leu Glu Leu Leu Thr Ile Phe Ala Trp Glu Gln Gly
225                 230                 235                 240

tgt aag aag gat gct ttc agc cta gcc gaa ggc ctc cga act gtc ctg      768
Cys Lys Lys Asp Ala Phe Ser Leu Ala Glu Gly Leu Arg Thr Val Leu
                245                 250                 255

ggc ctg atc caa cag cat cag cac ctg tgt gtt ttc tgg act gtc aac      816
Gly Leu Ile Gln Gln His Gln His Leu Cys Val Phe Trp Thr Val Asn
            260                 265                 270

tat ggc ttc gag gac cct gca gtt ggg cag ttc ttg cag cgg cag ctt      864
Tyr Gly Phe Glu Asp Pro Ala Val Gly Gln Phe Leu Gln Arg Gln Leu
        275                 280                 285

aag aga ccc agg cct gtg atc ctg gac cca gct gac ccc aca tgg gac      912
Lys Arg Pro Arg Pro Val Ile Leu Asp Pro Ala Asp Pro Thr Trp Asp
    290                 295                 300

ctg ggg aat ggg gca gcc tgg cac tgg gat ttg cta gcc cag gag gca      960
Leu Gly Asn Gly Ala Ala Trp His Trp Asp Leu Leu Ala Gln Glu Ala
305                 310                 315                 320

gca tcc tgc tat gac cac cca tgc ttt ctg agg ggg atg ggg gac cca     1008
Ala Ser Cys Tyr Asp His Pro Cys Phe Leu Arg Gly Met Gly Asp Pro

325                 330                 335

gtg cag tct tgg aag ggg ccg ggc ctt cca cgt gct gga tgc tca ggt      1056
Val Gln Ser Trp Lys Gly Pro Gly Leu Pro Arg Ala Gly Cys Ser Gly
            340                 345                 350

ttg ggc cac ccc atc cag cta gac cct aac cag aag acc cct gaa aac      1104
Leu Gly His Pro Ile Gln Leu Asp Pro Asn Gln Lys Thr Pro Glu Asn
        355                 360                 365

agc aag agc ctc aat gct gtg tac cca aga gca ggg agc aaa cct ccc      1152
Ser Lys Ser Leu Asn Ala Val Tyr Pro Arg Ala Gly Ser Lys Pro Pro
    370                 375                 380

tca tgc cca gct cct ggc ccc act ggg gca gcc agc atc gtc ccc tct      1200
Ser Cys Pro Ala Pro Gly Pro Thr Gly Ala Ala Ser Ile Val Pro Ser
385                 390                 395                 400

gtg ccg gga atg gcc ttg gac ctg tct cag atc ccc acc aag gag ctg      1248
Val Pro Gly Met Ala Leu Asp Leu Ser Gln Ile Pro Thr Lys Glu Leu
                405                 410                 415

gac cgc ttc atc cag gac cac ctg aag ccg agc ccc cag ttc cag gag      1296
Asp Arg Phe Ile Gln Asp His Leu Lys Pro Ser Pro Gln Phe Gln Glu
            420                 425                 430

cag gtg aaa aag gcc atc gac atc atc ttg cgc tgc ctc cat gag aac      1344
Gln Val Lys Lys Ala Ile Asp Ile Ile Leu Arg Cys Leu His Glu Asn
        435                 440                 445

tgt gtt cac aag gcc tca aga gtc agt aaa ggg ggc tca ttt ggc cgg      1392
Cys Val His Lys Ala Ser Arg Val Ser Lys Gly Gly Ser Phe Gly Arg
    450                 455                 460

ggc aca gac cta agg gat ggc tgt gat gtt gaa ctc atc atc ttc ctc      1440
Gly Thr Asp Leu Arg Asp Gly Cys Asp Val Glu Leu Ile Ile Phe Leu
465                 470                 475                 480

aac tgc ttc acg gac tac aag gac cag ggg ccc cgc cgc gca gag atc      1488
Asn Cys Phe Thr Asp Tyr Lys Asp Gln Gly Pro Arg Arg Ala Glu Ile
                485                 490                 495

ctt gat gag atg cga gcg cag cta gaa tcc tgg tgg cag gac cag gtg      1536
Leu Asp Glu Met Arg Ala Gln Leu Glu Ser Trp Trp Gln Asp Gln Val
            500                 505                 510

ccc agc ctg agc ctt cag ttt cct gag cag aat gtg cct gag gct ctg      1584
Pro Ser Leu Ser Leu Gln Phe Pro Glu Gln Asn Val Pro Glu Ala Leu
        515                 520                 525

cag ttc cag ctg gtg tcc aca gcc ctg aag agc tgg acg gat gtt agc      1632
Gln Phe Gln Leu Val Ser Thr Ala Leu Lys Ser Trp Thr Asp Val Ser
    530                 535                 540

ctg ctg cct gcc ttc gat gct gtg ggg cag ctc agt tct ggc acc aaa      1680
Leu Leu Pro Ala Phe Asp Ala Val Gly Gln Leu Ser Ser Gly Thr Lys
545                 550                 555                 560

cca aat ccc cag gtc tac tcg agg ctc ctc acc agt ggc tgc cag gag      1728
Pro Asn Pro Gln Val Tyr Ser Arg Leu Leu Thr Ser Gly Cys Gln Glu
                565                 570                 575

ggc gag cat aag gcc tgc ttc gca gag ctg cgg agg aac ttc atg aac      1776
Gly Glu His Lys Ala Cys Phe Ala Glu Leu Arg Arg Asn Phe Met Asn
            580                 585                 590

att cgc cct gtc aag ctg aag aac ctg att ctg ctg gtg aag cac tgg 1824
Ile Arg Pro Val Lys Leu Lys Asn Leu Ile Leu Leu Val Lys His Trp
        595                 600                 605

tac cgc cag gtt gcg gct cag aac aaa gga aaa gga cca gcc cct gcc 1872
Tyr Arg Gln Val Ala Ala Gln Asn Lys Gly Lys Gly Pro Ala Pro Ala
    610                 615                 620

tct ctg ccc cca gcc tat gcc ctg gag ctc ctc acc atc ttt gcc tgg 1920
Ser Leu Pro Pro Ala Tyr Ala Leu Glu Leu Leu Thr Ile Phe Ala Trp
625                 630                 635                 640

gag cag ggc tgc agg cag gat tgt ttc aac atg gcc caa ggc ttc cgg 1968
Glu Gln Gly Cys Arg Gln Asp Cys Phe Asn Met Ala Gln Gly Phe Arg
                645                 650                 655

acg gtg ctg ggg ctc gtg caa cag cat cag cag ctc tgt gtc tac tgg 2016
Thr Val Leu Gly Leu Val Gln Gln His Gln Gln Leu Cys Val Tyr Trp
            660                 665                 670

acg gtc aac tat agc act gag gac cca gcc atg aga atg cac ctt ctt 2064
Thr Val Asn Tyr Ser Thr Glu Asp Pro Ala Met Arg Met His Leu Leu
        675                 680                 685

ggc cag ctt cga aaa ccc aga ccc ctg gtc ctg gac ccc gct gat ccc 2112
Gly Gln Leu Arg Lys Pro Arg Pro Leu Val Leu Asp Pro Ala Asp Pro
    690                 695                 700

acc tgg aac gtg ggc cac ggt agc tgg gag ctg ttg gcc cag gaa gca 2160
Thr Trp Asn Val Gly His Gly Ser Trp Glu Leu Leu Ala Gln Glu Ala
705                 710                 715                 720

gca gcg ctg ggg atg cag gcc tgc ttt ctg agt aga gac ggg aca tct 2208
Ala Ala Leu Gly Met Gln Ala Cys Phe Leu Ser Arg Asp Gly Thr Ser
                725                 730                 735

gtg cag ccc tgg gat gtg atg cca gcc ctc ctt tac caa acc cca gct 2256
Val Gln Pro Trp Asp Val Met Pro Ala Leu Leu Tyr Gln Thr Pro Ala
            740                 745                 750

ggg gac ctt gac aag ttc atc agt gaa ttt ctc cag ccc aac cgc cag 2304
Gly Asp Leu Asp Lys Phe Ile Ser Glu Phe Leu Gln Pro Asn Arg Gln
        755                 760                 765

ttc ctg gcc cag gtg aac aag gcc gtt gat acc atc tgt tca ttt ttg 2352
Phe Leu Ala Gln Val Asn Lys Ala Val Asp Thr Ile Cys Ser Phe Leu
    770                 775                 780

aag gaa aac tgc ttc cgg aat tct ccc atc aaa gtg atc aag gtg gtc 2400
Lys Glu Asn Cys Phe Arg Asn Ser Pro Ile Lys Val Ile Lys Val Val
785                 790                 795                 800

aag ggt ggc tct tca gcc aaa ggc aca gct ctg cga ggc cgc tca gat 2448
Lys Gly Gly Ser Ser Ala Lys Gly Thr Ala Leu Arg Gly Arg Ser Asp
                805                 810                 815

gcc gac ctc gtg gtg ttc ctc agc tgc ttc agc cag ttc act gag cag 2496
Ala Asp Leu Val Val Phe Leu Ser Cys Phe Ser Gln Phe Thr Glu Gln
            820                 825                 830

ggc aac aag cgg gcc gag atc atc tcc gag atc cga gcc cag ctg gag 2544
Gly Asn Lys Arg Ala Glu Ile Ile Ser Glu Ile Arg Ala Gln Leu Glu
        835                 840                 845

gca tgt caa cag gag cgg cag ttc gag gtc aag ttt gaa gtc tcc aaa 2592
Ala Cys Gln Gln Glu Arg Gln Phe Glu Val Lys Phe Glu Val Ser Lys
    850                 855                 860

tcg gag aat ccc cgc gtg ctg agc ttc tca ctg aca tcc cag acg atg 2640
Ser Glu Asn Pro Arg Val Leu Ser Phe Ser Leu Thr Ser Gln Thr Met
865                 870                 875                 880

ctg gac cag agt gtg gac ttt gat gtg ctg cca gcc ttt gac gcc cta 2688
Leu Asp Gln Ser Val Asp Phe Asp Val Leu Pro Ala Phe Asp Ala Leu
                885                 890                 895

ggc cag ctg gtc tct ggc tcc agg ccc agc tct caa gtc tac gtc gac 2736
Gly Gln Leu Val Ser Gly Ser Arg Pro Ser Ser Gln Val Tyr Val Asp
            900                 905                 910

ctc atc cac agc tac agc aat gcg ggc gag tac tcc acc tgc ttc aca 2784
Leu Ile His Ser Tyr Ser Asn Ala Gly Glu Tyr Ser Thr Cys Phe Thr
        915                 920                 925

gag cta caa cgg gac ttc atc atc tct cgc cct acc aag ctg aag agc 2832
Glu Leu Gln Arg Asp Phe Ile Ile Ser Arg Pro Thr Lys Leu Lys Ser
    930                 935                 940

ctg atc cgg ctg gtg aag cac tgg tac cag cag tgt acc aag atc tcc 2880
Leu Ile Arg Leu Val Lys His Trp Tyr Gln Gln Cys Thr Lys Ile Ser
945                 950                 955                 960

aag ggg aga ggc tcc cta ccc cca cag cac ggg ctg gaa ctc ctg act 2928
Lys Gly Arg Gly Ser Leu Pro Pro Gln His Gly Leu Glu Leu Leu Thr
                965                 970                 975

gtg tat gcc tgg gag cag ggc ggg aag gac tcc cag ttc aac atg gct 2976
Val Tyr Ala Trp Glu Gln Gly Gly Lys Asp Ser Gln Phe Asn Met Ala
            980                 985                 990

gag ggc ttc cgc acg gtc ctg gag ctg gtc acc cag tac cgc cag ctc 3024
Glu Gly Phe Arg Thr Val Leu Glu Leu Val Thr Gln Tyr Arg Gln Leu
        995                 1000                1005

tgt atc tac tgg acc atc aac tac aac gcc aag gac aag act gtt 3069
Cys Ile Tyr Trp Thr Ile Asn Tyr Asn Ala Lys Asp Lys Thr Val
    1010                1015                1020

gga gac ttc ctg aaa cag cag ctt cag aag ccc agg cct atc atc 3114
Gly Asp Phe Leu Lys Gln Gln Leu Gln Lys Pro Arg Pro Ile Ile
    1025                1030                1035

ctg gat ccg gct gac ccg aca ggc aac ctg ggc cac aat gcc cgc 3159
Leu Asp Pro Ala Asp Pro Thr Gly Asn Leu Gly His Asn Ala Arg
    1040                1045                1050

tgg gac ctg ctg gcc aag gaa gct gca gcc tgc aca tct gcc ctg 3204
Trp Asp Leu Leu Ala Lys Glu Ala Ala Ala Cys Thr Ser Ala Leu
    1055                1060                1065

tgc tgc atg ggg cgg aat ggc atc ccc atc cag cca tgg cca gtg 3249
Cys Cys Met Gly Arg Asn Gly Ile Pro Ile Gln Pro Trp Pro Val
    1070                1075                1080

aag gct gct gtg tga 3264
Lys Ala Ala Val
    1085

<210> 7
<211> 1087
<212> PRT
<213> artificial sequence

<220>
<223> Synthetic Construct

<400> 7

Met Asp Leu Tyr Ser Thr Pro Ala Ala Ala Leu Asp Arg Phe Val Ala
1               5                   10                  15

Arg Lys Leu Gln Pro Arg Lys Glu Phe Val Glu Lys Ala Arg Arg Ala
            20                  25                  30

Leu Gly Ala Leu Ala Ala Ala Leu Arg Glu Arg Gly Gly Arg Leu Gly
        35                  40                  45

Ala Ala Ala Pro Arg Val Leu Lys Thr Val Lys Gly Gly Ser Ser Gly
    50                  55                  60

Arg Gly Thr Ala Leu Lys Gly Gly Cys Asp Ser Glu Leu Val Ile Phe
65                  70                  75                  80

Leu Asp Cys Phe Lys Ser Tyr Val Asp Gln Arg Ala Arg Arg Ala Glu
                85                  90                  95

Ile Leu Ser Glu Met Arg Ala Ser Leu Glu Ser Trp Trp Gln Asn Pro
            100                 105                 110

Val Pro Gly Leu Arg Leu Thr Phe Pro Glu Gln Ser Val Pro Gly Ala
        115                 120                 125

Leu Gln Phe Arg Leu Thr Ser Val Asp Leu Glu Asp Trp Met Asp Val
    130                 135                 140

Ser Leu Val Pro Ala Phe Asn Val Leu Gly Gln Ala Gly Ser Gly Val
145                 150                 155                 160

Lys Pro Lys Pro Gln Val Tyr Ser Thr Leu Leu Asn Ser Gly Cys Gln
                165                 170                 175

Gly Gly Glu His Ala Ala Cys Phe Thr Glu Leu Arg Arg Asn Phe Val
            180                 185                 190

Asn Ile Arg Pro Ala Lys Leu Lys Asn Leu Ile Leu Leu Val Lys His
        195                 200                 205

Trp Tyr His Gln Val Cys Leu Gln Gly Leu Trp Lys Glu Thr Leu Pro
    210                 215                 220

Pro Val Tyr Ala Leu Glu Leu Leu Thr Ile Phe Ala Trp Glu Gln Gly
225                 230                 235                 240

Cys Lys Lys Asp Ala Phe Ser Leu Ala Glu Gly Leu Arg Thr Val Leu
                245                 250                 255

Gly Leu Ile Gln Gln His Gln His Leu Cys Val Phe Trp Thr Val Asn
            260                 265                 270

Tyr Gly Phe Glu Asp Pro Ala Val Gly Gln Phe Leu Gln Arg Gln Leu
        275                 280                 285

Lys Arg Pro Arg Pro Val Ile Leu Asp Pro Ala Asp Pro Thr Trp Asp
    290                 295                 300

Leu Gly Asn Gly Ala Ala Trp His Trp Asp Leu Leu Ala Gln Glu Ala
305                 310                 315                 320

Ala Ser Cys Tyr Asp His Pro Cys Phe Leu Arg Gly Met Gly Asp Pro
                325                 330                 335

Val Gln Ser Trp Lys Gly Pro Gly Leu Pro Arg Ala Gly Cys Ser Gly
            340                 345                 350

Leu Gly His Pro Ile Gln Leu Asp Pro Asn Gln Lys Thr Pro Glu Asn
        355                 360                 365

Ser Lys Ser Leu Asn Ala Val Tyr Pro Arg Ala Gly Ser Lys Pro Pro
    370                 375                 380

Ser Cys Pro Ala Pro Gly Pro Thr Gly Ala Ala Ser Ile Val Pro Ser
385                 390                 395                 400

Val Pro Gly Met Ala Leu Asp Leu Ser Gln Ile Pro Thr Lys Glu Leu
                405                 410                 415

Asp Arg Phe Ile Gln Asp His Leu Lys Pro Ser Pro Gln Phe Gln Glu
            420                 425                 430

Gln Val Lys Lys Ala Ile Asp Ile Ile Leu Arg Cys Leu His Glu Asn
        435                 440                 445

Cys Val His Lys Ala Ser Arg Val Ser Lys Gly Gly Ser Phe Gly Arg
    450                 455                 460

Gly Thr Asp Leu Arg Asp Gly Cys Asp Val Glu Leu Ile Ile Phe Leu
465                 470                 475                 480

Asn Cys Phe Thr Asp Tyr Lys Asp Gln Gly Pro Arg Arg Ala Glu Ile
                485                 490                 495

Leu Asp Glu Met Arg Ala Gln Leu Glu Ser Trp Trp Gln Asp Gln Val
            500                 505                 510

Pro Ser Leu Ser Leu Gln Phe Pro Glu Gln Asn Val Pro Glu Ala Leu
        515                 520                 525

Gln Phe Gln Leu Val Ser Thr Ala Leu Lys Ser Trp Thr Asp Val Ser
    530                 535                 540

Leu Leu Pro Ala Phe Asp Ala Val Gly Gln Leu Ser Ser Gly Thr Lys
545                 550                 555                 560

Pro Asn Pro Gln Val Tyr Ser Arg Leu Leu Thr Ser Gly Cys Gln Glu
                565                 570                 575

Gly Glu His Lys Ala Cys Phe Ala Glu Leu Arg Arg Asn Phe Met Asn
            580                 585                 590

Ile Arg Pro Val Lys Leu Lys Asn Leu Ile Leu Leu Val Lys His Trp
        595                 600                 605

Tyr Arg Gln Val Ala Ala Gln Asn Lys Gly Lys Gly Pro Ala Pro Ala
    610                 615                 620

Ser Leu Pro Pro Ala Tyr Ala Leu Glu Leu Leu Thr Ile Phe Ala Trp
625                 630                 635                 640

Glu Gln Gly Cys Arg Gln Asp Cys Phe Asn Met Ala Gln Gly Phe Arg
                645                 650                 655

Thr Val Leu Gly Leu Val Gln Gln His Gln Gln Leu Cys Val Tyr Trp
            660                 665                 670

Thr Val Asn Tyr Ser Thr Glu Asp Pro Ala Met Arg Met His Leu Leu
        675                 680                 685

Gly Gln Leu Arg Lys Pro Arg Pro Leu Val Leu Asp Pro Ala Asp Pro
    690                 695                 700

Thr Trp Asn Val Gly His Gly Ser Trp Glu Leu Leu Ala Gln Glu Ala
705                 710                 715                 720

Ala Ala Leu Gly Met Gln Ala Cys Phe Leu Ser Arg Asp Gly Thr Ser
                725                 730                 735

Val Gln Pro Trp Asp Val Met Pro Ala Leu Leu Tyr Gln Thr Pro Ala

            740                 745                 750

Gly Asp Leu Asp Lys Phe Ile Ser Glu Phe Leu Gln Pro Asn Arg Gln
        755                 760                 765

Phe Leu Ala Gln Val Asn Lys Ala Val Asp Thr Ile Cys Ser Phe Leu
    770                 775                 780

Lys Glu Asn Cys Phe Arg Asn Ser Pro Ile Lys Val Ile Lys Val Val
785                 790                 795                 800

Lys Gly Gly Ser Ser Ala Lys Gly Thr Ala Leu Arg Gly Arg Ser Asp
                805                 810                 815

Ala Asp Leu Val Val Phe Leu Ser Cys Phe Ser Gln Phe Thr Glu Gln
            820                 825                 830

Gly Asn Lys Arg Ala Glu Ile Ile Ser Glu Ile Arg Ala Gln Leu Glu
        835                 840                 845

Ala Cys Gln Gln Glu Arg Gln Phe Glu Val Lys Phe Glu Val Ser Lys
    850                 855                 860

Ser Glu Asn Pro Arg Val Leu Ser Phe Ser Leu Thr Ser Gln Thr Met
865                 870                 875                 880

Leu Asp Gln Ser Val Asp Phe Asp Val Leu Pro Ala Phe Asp Ala Leu
                885                 890                 895

Gly Gln Leu Val Ser Gly Ser Arg Pro Ser Ser Gln Val Tyr Val Asp
            900                 905                 910

Leu Ile His Ser Tyr Ser Asn Ala Gly Glu Tyr Ser Thr Cys Phe Thr
        915                 920                 925

Glu Leu Gln Arg Asp Phe Ile Ile Ser Arg Pro Thr Lys Leu Lys Ser
    930                 935                 940

Leu Ile Arg Leu Val Lys His Trp Tyr Gln Gln Cys Thr Lys Ile Ser
945                 950                 955                 960

Lys Gly Arg Gly Ser Leu Pro Pro Gln His Gly Leu Glu Leu Leu Thr
                965                 970                 975

Val Tyr Ala Trp Glu Gln Gly Gly Lys Asp Ser Gln Phe Asn Met Ala
            980                 985                 990

Glu Gly Phe Arg Thr Val Leu Glu Leu Val Thr Gln Tyr Arg Gln Leu
        995                 1000                1005

Cys Ile Tyr Trp Thr Ile Asn Tyr Asn Ala Lys Asp Lys Thr Val
    1010                1015                1020

Gly Asp Phe Leu Lys Gln Gln Leu Gln Lys Pro Arg Pro Ile Ile
    1025                1030                1035

Leu Asp Pro Ala Asp Pro Thr Gly Asn Leu Gly His Asn Ala Arg
    1040                1045                1050

Trp Asp Leu Leu Ala Lys Glu Ala Ala Ala Cys Thr Ser Ala Leu
    1055                1060                1065

Cys Cys Met Gly Arg Asn Gly Ile Pro Ile Gln Pro Trp Pro Val
    1070                1075                1080

Lys Ala Ala Val
    1085

<210>  8
<211>  2532
<212>  DNA
<213>  artificial sequence

<220>
<223>  OAS3 truncated protein (OAS3 1-843  or OAS3 delta-C-term)

<220>
<221>  CDS
<222>  (1)..(2532)

<400>  8
atg gac ttg tac agc acc ccg gcc gct gcg ctg gac agg ttc gtg gcc      48
Met Asp Leu Tyr Ser Thr Pro Ala Ala Ala Leu Asp Arg Phe Val Ala
1               5                   10                  15

aga aag ctg cag ccg cgg aag gag ttc gta gag aag gcg cgg cgc gct      96
Arg Lys Leu Gln Pro Arg Lys Glu Phe Val Glu Lys Ala Arg Arg Ala
            20                  25                  30

ctg ggc gcc ctg gcc gct gct ctg agg gag cgc ggg ggc cgc ctc ggt      144
Leu Gly Ala Leu Ala Ala Ala Leu Arg Glu Arg Gly Gly Arg Leu Gly
        35                  40                  45

gct gct gcc ccg cgg gtg ctg aaa act gtc aag gga ggc tcc tcg ggc      192
Ala Ala Ala Pro Arg Val Leu Lys Thr Val Lys Gly Gly Ser Ser Gly
    50                  55                  60

cgg ggc aca gct ctc aag ggt ggc tgt gat tct gaa ctt gtc atc ttc      240
Arg Gly Thr Ala Leu Lys Gly Gly Cys Asp Ser Glu Leu Val Ile Phe
65                  70                  75                  80

ctc gac tgc ttc aag agc tat gtg gac cag agg gcc cgc cgt gca gag      288
Leu Asp Cys Phe Lys Ser Tyr Val Asp Gln Arg Ala Arg Arg Ala Glu
                85                  90                  95

atc ctc agt gag atg cgg gca tcg ctg gaa tcc tgg tgg cag aac cca      336

Ile Leu Ser Glu Met Arg Ala Ser Leu Glu Ser Trp Trp Gln Asn Pro
            100                 105                 110

gtc cct ggt ctg aga ctc acg ttt cct gag cag agc gtg cct ggg gcc 384
Val Pro Gly Leu Arg Leu Thr Phe Pro Glu Gln Ser Val Pro Gly Ala
        115                 120                 125

ctg cag ttc cgc ctg aca tcc gta gat ctt gag gac tgg atg gat gtt 432
Leu Gln Phe Arg Leu Thr Ser Val Asp Leu Glu Asp Trp Met Asp Val
    130                 135                 140

agc ctg gtg cct gcc ttc aat gtc ctg ggt cag gcc ggc tcc ggc gtc 480
Ser Leu Val Pro Ala Phe Asn Val Leu Gly Gln Ala Gly Ser Gly Val
145                 150                 155                 160

aaa ccc aag cca caa gtc tac tct acc ctc ctc aac agt ggc tgc caa 528
Lys Pro Lys Pro Gln Val Tyr Ser Thr Leu Leu Asn Ser Gly Cys Gln
                165                 170                 175

ggg ggc gag cat gcg gcc tgc ttc aca gag ctg cgg agg aac ttt gtg 576
Gly Gly Glu His Ala Ala Cys Phe Thr Glu Leu Arg Arg Asn Phe Val
            180                 185                 190

aac att cgc cca gcc aag ttg aag aac cta atc ttg ctg gtg aag cac 624
Asn Ile Arg Pro Ala Lys Leu Lys Asn Leu Ile Leu Leu Val Lys His
        195                 200                 205

tgg tac cac cag gtg tgc cta cag ggg ttg tgg aag gag acg ctg ccc 672
Trp Tyr His Gln Val Cys Leu Gln Gly Leu Trp Lys Glu Thr Leu Pro
    210                 215                 220

ccg gtc tat gcc ctg gaa ttg ctg acc atc ttc gcc tgg gag cag ggc 720
Pro Val Tyr Ala Leu Glu Leu Leu Thr Ile Phe Ala Trp Glu Gln Gly
225                 230                 235                 240

tgt aag aag gat gct ttc agc cta gcc gaa ggc ctc cga act gtc ctg 768
Cys Lys Lys Asp Ala Phe Ser Leu Ala Glu Gly Leu Arg Thr Val Leu
                245                 250                 255

ggc ctg atc caa cag cat cag cac ctg tgt gtt ttc tgg act gtc aac 816
Gly Leu Ile Gln Gln His Gln His Leu Cys Val Phe Trp Thr Val Asn
            260                 265                 270

tat ggc ttc gag gac cct gca gtt ggg cag ttc ttg cag cgg cag ctt 864
Tyr Gly Phe Glu Asp Pro Ala Val Gly Gln Phe Leu Gln Arg Gln Leu
        275                 280                 285

aag aga ccc agg cct gtg atc ctg gac cca gct gac ccc aca tgg gac 912
Lys Arg Pro Arg Pro Val Ile Leu Asp Pro Ala Asp Pro Thr Trp Asp
    290                 295                 300

ctg ggg aat ggg gca gcc tgg cac tgg gat ttg cta gcc cag gag gca 960
Leu Gly Asn Gly Ala Ala Trp His Trp Asp Leu Leu Ala Gln Glu Ala
305                 310                 315                 320

gca tcc tgc tat gac cac cca tgc ttt ctg agg ggg atg ggg gac cca 1008
Ala Ser Cys Tyr Asp His Pro Cys Phe Leu Arg Gly Met Gly Asp Pro
                325                 330                 335

gtg cag tct tgg aag ggg ccg ggc ctt cca cgt gct gga tgc tca ggt 1056
Val Gln Ser Trp Lys Gly Pro Gly Leu Pro Arg Ala Gly Cys Ser Gly
            340                 345                 350

ttg ggc cac ccc atc cag cta gac cct aac cag aag acc cct gaa aac 1104
Leu Gly His Pro Ile Gln Leu Asp Pro Asn Gln Lys Thr Pro Glu Asn

355                 360                 365

agc aag agc ctc aat gct gtg tac cca aga gca ggg agc aaa cct ccc       1152
Ser Lys Ser Leu Asn Ala Val Tyr Pro Arg Ala Gly Ser Lys Pro Pro
    370                 375                 380

tca tgc cca gct cct ggc ccc act ggg gca gcc agc atc gtc ccc tct       1200
Ser Cys Pro Ala Pro Gly Pro Thr Gly Ala Ala Ser Ile Val Pro Ser
385                 390                 395                 400

gtg ccg gga atg gcc ttg gac ctg tct cag atc ccc acc aag gag ctg       1248
Val Pro Gly Met Ala Leu Asp Leu Ser Gln Ile Pro Thr Lys Glu Leu
                405                 410                 415

gac cgc ttc atc cag gac cac ctg aag ccg agc ccc cag ttc cag gag       1296
Asp Arg Phe Ile Gln Asp His Leu Lys Pro Ser Pro Gln Phe Gln Glu
            420                 425                 430

cag gtg aaa aag gcc atc gac atc atc ttg cgc tgc ctc cat gag aac       1344
Gln Val Lys Lys Ala Ile Asp Ile Ile Leu Arg Cys Leu His Glu Asn
        435                 440                 445

tgt gtt cac aag gcc tca aga gtc agt aaa ggg ggc tca ttt ggc cgg       1392
Cys Val His Lys Ala Ser Arg Val Ser Lys Gly Gly Ser Phe Gly Arg
    450                 455                 460

ggc aca gac cta agg gat ggc tgt gat gtt gaa ctc atc atc ttc ctc       1440
Gly Thr Asp Leu Arg Asp Gly Cys Asp Val Glu Leu Ile Ile Phe Leu
465                 470                 475                 480

aac tgc ttc acg gac tac aag gac cag ggg ccc cgc cgc gca gag atc       1488
Asn Cys Phe Thr Asp Tyr Lys Asp Gln Gly Pro Arg Arg Ala Glu Ile
                485                 490                 495

ctt gat gag atg cga gcg cag cta gaa tcc tgg tgg cag gac cag gtg       1536
Leu Asp Glu Met Arg Ala Gln Leu Glu Ser Trp Trp Gln Asp Gln Val
            500                 505                 510

ccc agc ctg agc ctt cag ttt cct gag cag aat gtg cct gag gct ctg       1584
Pro Ser Leu Ser Leu Gln Phe Pro Glu Gln Asn Val Pro Glu Ala Leu
        515                 520                 525

cag ttc cag ctg gtg tcc aca gcc ctg aag agc tgg acg gat gtt agc       1632
Gln Phe Gln Leu Val Ser Thr Ala Leu Lys Ser Trp Thr Asp Val Ser
    530                 535                 540

ctg ctg cct gcc ttc gat gct gtg ggg cag ctc agt tct ggc acc aaa       1680
Leu Leu Pro Ala Phe Asp Ala Val Gly Gln Leu Ser Ser Gly Thr Lys
545                 550                 555                 560

cca aat ccc cag gtc tac tcg agg ctc ctc acc agt ggc tgc cag gag       1728
Pro Asn Pro Gln Val Tyr Ser Arg Leu Leu Thr Ser Gly Cys Gln Glu
                565                 570                 575

ggc gag cat aag gcc tgc ttc gca gag ctg cgg agg aac ttc atg aac       1776
Gly Glu His Lys Ala Cys Phe Ala Glu Leu Arg Arg Asn Phe Met Asn
            580                 585                 590

att cgc cct gtc aag ctg aag aac ctg att ctg ctg gtg aag cac tgg       1824
Ile Arg Pro Val Lys Leu Lys Asn Leu Ile Leu Leu Val Lys His Trp
        595                 600                 605

tac cgc cag gtt gcg gct cag aac aaa gga aaa gga cca gcc cct gcc       1872
Tyr Arg Gln Val Ala Ala Gln Asn Lys Gly Lys Gly Pro Ala Pro Ala
    610                 615                 620

tct ctg ccc cca gcc tat gcc ctg gag ctc ctc acc atc ttt gcc tgg 1920
Ser Leu Pro Pro Ala Tyr Ala Leu Glu Leu Leu Thr Ile Phe Ala Trp
625                 630                 635                 640

gag cag ggc tgc agg cag gat tgt ttc aac atg gcc caa ggc ttc cgg 1968
Glu Gln Gly Cys Arg Gln Asp Cys Phe Asn Met Ala Gln Gly Phe Arg
                645                 650                 655

acg gtg ctg ggg ctc gtg caa cag cat cag cag ctc tgt gtc tac tgg 2016
Thr Val Leu Gly Leu Val Gln Gln His Gln Gln Leu Cys Val Tyr Trp
            660                 665                 670

acg gtc aac tat agc act gag gac cca gcc atg aga atg cac ctt ctt 2064
Thr Val Asn Tyr Ser Thr Glu Asp Pro Ala Met Arg Met His Leu Leu
        675                 680                 685

ggc cag ctt cga aaa ccc aga ccc ctg gtc ctg gac ccc gct gat ccc 2112
Gly Gln Leu Arg Lys Pro Arg Pro Leu Val Leu Asp Pro Ala Asp Pro
    690                 695                 700

acc tgg aac gtg ggc cac ggt agc tgg gag ctg ttg gcc cag gaa gca 2160
Thr Trp Asn Val Gly His Gly Ser Trp Glu Leu Leu Ala Gln Glu Ala
705                 710                 715                 720

gca gcg ctg ggg atg cag gcc tgc ttt ctg agt aga gac ggg aca tct 2208
Ala Ala Leu Gly Met Gln Ala Cys Phe Leu Ser Arg Asp Gly Thr Ser
                725                 730                 735

gtg cag ccc tgg gat gtg atg cca gcc ctc ctt tac caa acc cca gct 2256
Val Gln Pro Trp Asp Val Met Pro Ala Leu Leu Tyr Gln Thr Pro Ala
            740                 745                 750

ggg gac ctt gac aag ttc atc agt gaa ttt ctc cag ccc aac cgc cag 2304
Gly Asp Leu Asp Lys Phe Ile Ser Glu Phe Leu Gln Pro Asn Arg Gln
        755                 760                 765

ttc ctg gcc cag gtg aac aag gcc gtt gat acc atc tgt tca ttt ttg 2352
Phe Leu Ala Gln Val Asn Lys Ala Val Asp Thr Ile Cys Ser Phe Leu
    770                 775                 780

aag gaa aac tgc ttc cgg aat tct ccc atc aaa gtg atc aag gtg gtc 2400
Lys Glu Asn Cys Phe Arg Asn Ser Pro Ile Lys Val Ile Lys Val Val
785                 790                 795                 800

aag ggt ggc tct tca gcc aaa ggc aca gct ctg cga ggc cgc tca gat 2448
Lys Gly Gly Ser Ser Ala Lys Gly Thr Ala Leu Arg Gly Arg Ser Asp
                805                 810                 815

gcc gac ctc gtg gtg ttc ctc agc tgc ttc agc cag ttc act gag cag 2496
Ala Asp Leu Val Val Phe Leu Ser Cys Phe Ser Gln Phe Thr Glu Gln
            820                 825                 830

ggc aac aag cgg gcc gag atc atc tcc gag atc tga 2532
Gly Asn Lys Arg Ala Glu Ile Ile Ser Glu Ile
        835                 840

<210> 9
<211> 843
<212> PRT
<213> artificial sequence

<220>
<223> Synthetic Construct

<400>  9

Met Asp Leu Tyr Ser Thr Pro Ala Ala Ala Leu Asp Arg Phe Val Ala
1               5                   10                  15

Arg Lys Leu Gln Pro Arg Lys Glu Phe Val Glu Lys Ala Arg Arg Ala
            20                  25                  30

Leu Gly Ala Leu Ala Ala Ala Leu Arg Glu Arg Gly Gly Arg Leu Gly
        35                  40                  45

Ala Ala Ala Pro Arg Val Leu Lys Thr Val Lys Gly Gly Ser Ser Gly
    50                  55                  60

Arg Gly Thr Ala Leu Lys Gly Gly Cys Asp Ser Glu Leu Val Ile Phe
65                  70                  75                  80

Leu Asp Cys Phe Lys Ser Tyr Val Asp Gln Arg Ala Arg Arg Ala Glu
                85                  90                  95

Ile Leu Ser Glu Met Arg Ala Ser Leu Glu Ser Trp Trp Gln Asn Pro
            100                 105                 110

Val Pro Gly Leu Arg Leu Thr Phe Pro Glu Gln Ser Val Pro Gly Ala
        115                 120                 125

Leu Gln Phe Arg Leu Thr Ser Val Asp Leu Glu Asp Trp Met Asp Val
    130                 135                 140

Ser Leu Val Pro Ala Phe Asn Val Leu Gly Gln Ala Gly Ser Gly Val
145                 150                 155                 160

Lys Pro Lys Pro Gln Val Tyr Ser Thr Leu Leu Asn Ser Gly Cys Gln
                165                 170                 175

Gly Gly Glu His Ala Ala Cys Phe Thr Glu Leu Arg Arg Asn Phe Val
            180                 185                 190

Asn Ile Arg Pro Ala Lys Leu Lys Asn Leu Ile Leu Leu Val Lys His
        195                 200                 205

Trp Tyr His Gln Val Cys Leu Gln Gly Leu Trp Lys Glu Thr Leu Pro
    210                 215                 220

Pro Val Tyr Ala Leu Glu Leu Leu Thr Ile Phe Ala Trp Glu Gln Gly
225                 230                 235                 240

Cys Lys Lys Asp Ala Phe Ser Leu Ala Glu Gly Leu Arg Thr Val Leu
                245                 250                 255

Gly Leu Ile Gln Gln His Gln His Leu Cys Val Phe Trp Thr Val Asn
        260                 265                 270

Tyr Gly Phe Glu Asp Pro Ala Val Gly Gln Phe Leu Gln Arg Gln Leu
        275                 280                 285

Lys Arg Pro Arg Pro Val Ile Leu Asp Pro Ala Asp Pro Thr Trp Asp
    290                 295                 300

Leu Gly Asn Gly Ala Ala Trp His Trp Asp Leu Leu Ala Gln Glu Ala
305                 310                 315                 320

Ala Ser Cys Tyr Asp His Pro Cys Phe Leu Arg Gly Met Gly Asp Pro
                325                 330                 335

Val Gln Ser Trp Lys Gly Pro Gly Leu Pro Arg Ala Gly Cys Ser Gly
            340                 345                 350

Leu Gly His Pro Ile Gln Leu Asp Pro Asn Gln Lys Thr Pro Glu Asn
        355                 360                 365

Ser Lys Ser Leu Asn Ala Val Tyr Pro Arg Ala Gly Ser Lys Pro Pro
    370                 375                 380

Ser Cys Pro Ala Pro Gly Pro Thr Gly Ala Ala Ser Ile Val Pro Ser
385                 390                 395                 400

Val Pro Gly Met Ala Leu Asp Leu Ser Gln Ile Pro Thr Lys Glu Leu
                405                 410                 415

Asp Arg Phe Ile Gln Asp His Leu Lys Pro Ser Pro Gln Phe Gln Glu
            420                 425                 430

Gln Val Lys Lys Ala Ile Asp Ile Ile Leu Arg Cys Leu His Glu Asn
        435                 440                 445

Cys Val His Lys Ala Ser Arg Val Ser Lys Gly Gly Ser Phe Gly Arg
    450                 455                 460

Gly Thr Asp Leu Arg Asp Gly Cys Asp Val Glu Leu Ile Ile Phe Leu
465                 470                 475                 480

Asn Cys Phe Thr Asp Tyr Lys Asp Gln Gly Pro Arg Arg Ala Glu Ile
                485                 490                 495

Leu Asp Glu Met Arg Ala Gln Leu Glu Ser Trp Trp Gln Asp Gln Val
            500                 505                 510

Pro Ser Leu Ser Leu Gln Phe Pro Glu Gln Asn Val Pro Glu Ala Leu
        515                 520                 525

Gln Phe Gln Leu Val Ser Thr Ala Leu Lys Ser Trp Thr Asp Val Ser
    530                 535                 540

Leu Leu Pro Ala Phe Asp Ala Val Gly Gln Leu Ser Ser Gly Thr Lys
545                 550                 555                 560

Pro Asn Pro Gln Val Tyr Ser Arg Leu Leu Thr Ser Gly Cys Gln Glu
                565                 570                 575

Gly Glu His Lys Ala Cys Phe Ala Glu Leu Arg Arg Asn Phe Met Asn
            580                 585                 590

Ile Arg Pro Val Lys Leu Lys Asn Leu Ile Leu Leu Val Lys His Trp
        595                 600                 605

Tyr Arg Gln Val Ala Ala Gln Asn Lys Gly Lys Gly Pro Ala Pro Ala
    610                 615                 620

Ser Leu Pro Pro Ala Tyr Ala Leu Glu Leu Leu Thr Ile Phe Ala Trp
625                 630                 635                 640

Glu Gln Gly Cys Arg Gln Asp Cys Phe Asn Met Ala Gln Gly Phe Arg
                645                 650                 655

Thr Val Leu Gly Leu Val Gln Gln His Gln Gln Leu Cys Val Tyr Trp
            660                 665                 670

Thr Val Asn Tyr Ser Thr Glu Asp Pro Ala Met Arg Met His Leu Leu
        675                 680                 685

Gly Gln Leu Arg Lys Pro Arg Pro Leu Val Leu Asp Pro Ala Asp Pro
    690                 695                 700

Thr Trp Asn Val Gly His Gly Ser Trp Glu Leu Leu Ala Gln Glu Ala
705                 710                 715                 720

Ala Ala Leu Gly Met Gln Ala Cys Phe Leu Ser Arg Asp Gly Thr Ser
                725                 730                 735

Val Gln Pro Trp Asp Val Met Pro Ala Leu Leu Tyr Gln Thr Pro Ala
            740                 745                 750

Gly Asp Leu Asp Lys Phe Ile Ser Glu Phe Leu Gln Pro Asn Arg Gln
        755                 760                 765

Phe Leu Ala Gln Val Asn Lys Ala Val Asp Thr Ile Cys Ser Phe Leu
    770                 775                 780

Lys Glu Asn Cys Phe Arg Asn Ser Pro Ile Lys Val Ile Lys Val Val
785                 790                 795                 800

Lys Gly Gly Ser Ser Ala Lys Gly Thr Ala Leu Arg Gly Arg Ser Asp
                805                 810                 815

Ala Asp Leu Val Val Phe Leu Ser Cys Phe Ser Gln Phe Thr Glu Gln
            820                 825                 830

Gly Asn Lys Arg Ala Glu Ile Ile Ser Glu Ile
        835                 840

<210>  10
<211>  10
<212>  PRT
<213>  artificial sequence

<220>
<223>  cmyc tag

<400>  10

Glu Gln Lys Leu Ile Ser Lys Glu Asp Leu
1               5                   10

<210>  11
<211>  20
<212>  DNA
<213>  artificial sequence

<220>
<223>  primer

<400>  11
gtggtgttcc tcagctgctt                                                  20

<210>  12
<211>  20
<212>  DNA
<213>  artificial sequence

<220>
<223>  primer

<400>  12
catcgtctgg gatgtcagtg                                                  20

<210>  13
<211>  38
<212>  DNA
<213>  artificial sequence

<220>
<223>  primer

<400>  13
attaatgcgg ccgcaacgaa accagaaatc cgaaggcc                              38


<210>  14
<211>  78
<212>  DNA
<213>  artificial sequence

<220>
<223>  primer

<400>  14
aactaactat agtcaaaggt cctccttgga gatgagcttc tgttccacag cagccttcac      60

tggccatggc tggatggg                                                    78


<210>  15
<211>  44
<212>  DNA
<213>  artificial sequence

<220>
<223>  primer

<400>  15
attaatgcgg ccgcatggac ttgtacagca ccccggccgc tgcg                       44


<210>  16
<211>  81
<212>  DNA
<213>  artificial sequence

<220>
<223>  primer

<400>  16
aactaactat agtcaaaggt cctccttgga gatgagcttc tgttcgatct cggagatgat      60

ctcggcccgc cgcttgttgc c                                                81


<210>  17
<211>  21
<212>  DNA
<213>  artificial sequence

<220>
<223>  primer

<400>  17
cggacggtgc tggggctcgt g                                                21


<210>  18
<211>  24
<212>  DNA
<213>  artificial sequence

<220>
<223>  primer

<400>  18
cttgtcttcg agtagaggct tctc                                              24


<210>  19
<211>  17
<212>  DNA
<213>  artificial sequence

<220>
<223>  primer

<400>  19
caccgccgca actaccg                                                      17


<210>  20
<211>  17
<212>  DNA
<213>  artificial sequence

<220>
<223>  primer

<400>  20
gattggtgac cgcggca                                                      17


<210>  21
<211>  14
<212>  DNA
<213>  artificial sequence

<220>
<223>  primer

<400>  21
gggagccaaa aggg                                                         14


<210>  22
<211>  14
<212>  DNA
<213>  artificial sequence

<220>
<223>  primer

<400>  22
ggggacacgg aagg                                                         14


<210>  23
<211>  3308
<212>  DNA
<213>  artificial sequence

<220>
<223>  OAS3 c-myc tag


<220>
<221>  CDS
<222>  (9)..(3302)

<400> 23
gcggccgc atg gac ttg tac agc acc ccg gcc gct gcg ctg gac agg ttc 50
Met Asp Leu Tyr Ser Thr Pro Ala Ala Ala Leu Asp Arg Phe
1               5                   10

gtg gcc aga aag ctg cag ccg cgg aag gag ttc gta gag aag gcg cgg 98
Val Ala Arg Lys Leu Gln Pro Arg Lys Glu Phe Val Glu Lys Ala Arg
15                  20                  25                  30

cgc gct ctg ggc gcc ctg gcc gct gct ctg agg gag cgc ggg ggc cgc 146
Arg Ala Leu Gly Ala Leu Ala Ala Ala Leu Arg Glu Arg Gly Gly Arg
                35                  40                  45

ctc ggt gct gct gcc ccg cgg gtg ctg aaa act gtc aag gga ggc tcc 194
Leu Gly Ala Ala Ala Pro Arg Val Leu Lys Thr Val Lys Gly Gly Ser
            50                  55                  60

tcg ggc cgg ggc aca gct ctc aag ggt ggc tgt gat tct gaa ctt gtc 242
Ser Gly Arg Gly Thr Ala Leu Lys Gly Gly Cys Asp Ser Glu Leu Val
        65                  70                  75

atc ttc ctc gac tgc ttc aag agc tat gtg gac cag agg gcc cgc cgt 290
Ile Phe Leu Asp Cys Phe Lys Ser Tyr Val Asp Gln Arg Ala Arg Arg
    80                  85                  90

gca gag atc ctc agt gag atg cgg gca tcg ctg gaa tcc tgg tgg cag 338
Ala Glu Ile Leu Ser Glu Met Arg Ala Ser Leu Glu Ser Trp Trp Gln
95                  100                 105                 110

aac cca gtc cct ggt ctg aga ctc acg ttt cct gag cag agc gtg cct 386
Asn Pro Val Pro Gly Leu Arg Leu Thr Phe Pro Glu Gln Ser Val Pro
                115                 120                 125

ggg gcc ctg cag ttc cgc ctg aca tcc gta gat ctt gag gac tgg atg 434
Gly Ala Leu Gln Phe Arg Leu Thr Ser Val Asp Leu Glu Asp Trp Met
            130                 135                 140

gat gtt agc ctg gtg cct gcc ttc aat gtc ctg ggt cag gcc ggc tcc 482
Asp Val Ser Leu Val Pro Ala Phe Asn Val Leu Gly Gln Ala Gly Ser
        145                 150                 155

ggc gtc aaa ccc aag cca caa gtc tac tct acc ctc ctc aac agt ggc 530
Gly Val Lys Pro Lys Pro Gln Val Tyr Ser Thr Leu Leu Asn Ser Gly
    160                 165                 170

tgc caa ggg ggc gag cat gcg gcc tgc ttc aca gag ctg cgg agg aac 578
Cys Gln Gly Gly Glu His Ala Ala Cys Phe Thr Glu Leu Arg Arg Asn
175                 180                 185                 190

ttt gtg aac att cgc cca gcc aag ttg aag aac cta atc ttg ctg gtg 626
Phe Val Asn Ile Arg Pro Ala Lys Leu Lys Asn Leu Ile Leu Leu Val
                195                 200                 205

aag cac tgg tac cac cag gtg tgc cta cag ggg ttg tgg aag gag acg 674
Lys His Trp Tyr His Gln Val Cys Leu Gln Gly Leu Trp Lys Glu Thr
            210                 215                 220

ctg ccc ccg gtc tat gcc ctg gaa ttg ctg acc atc ttc gcc tgg gag 722
Leu Pro Pro Val Tyr Ala Leu Glu Leu Leu Thr Ile Phe Ala Trp Glu
        225                 230                 235

cag ggc tgt aag aag gat gct ttc agc cta gcc gaa ggc ctc cga act 770
Gln Gly Cys Lys Lys Asp Ala Phe Ser Leu Ala Glu Gly Leu Arg Thr
    240                 245                 250

gtc ctg ggc ctg atc caa cag cat cag cac ctg tgt gtt ttc tgg act 818
Val Leu Gly Leu Ile Gln Gln His Gln His Leu Cys Val Phe Trp Thr
255                 260                 265                 270

gtc aac tat ggc ttc gag gac cct gca gtt ggg cag ttc ttg cag cgg 866
Val Asn Tyr Gly Phe Glu Asp Pro Ala Val Gly Gln Phe Leu Gln Arg
                275                 280                 285

cag ctt aag aga ccc agg cct gtg atc ctg gac cca gct gac ccc aca 914
Gln Leu Lys Arg Pro Arg Pro Val Ile Leu Asp Pro Ala Asp Pro Thr
            290                 295                 300

tgg gac ctg ggg aat ggg gca gcc tgg cac tgg gat ttg cta gcc cag 962
Trp Asp Leu Gly Asn Gly Ala Ala Trp His Trp Asp Leu Leu Ala Gln
        305                 310                 315

gag gca gca tcc tgc tat gac cac cca tgc ttt ctg agg ggg atg ggg 1010
Glu Ala Ala Ser Cys Tyr Asp His Pro Cys Phe Leu Arg Gly Met Gly
    320                 325                 330

gac cca gtg cag tct tgg aag ggg ccg ggc ctt cca cgt gct gga tgc 1058
Asp Pro Val Gln Ser Trp Lys Gly Pro Gly Leu Pro Arg Ala Gly Cys
335                 340                 345                 350

tca ggt ttg ggc cac ccc atc cag cta gac cct aac cag aag acc cct 1106
Ser Gly Leu Gly His Pro Ile Gln Leu Asp Pro Asn Gln Lys Thr Pro
                355                 360                 365

gaa aac agc aag agc ctc aat gct gtg tac cca aga gca ggg agc aaa 1154
Glu Asn Ser Lys Ser Leu Asn Ala Val Tyr Pro Arg Ala Gly Ser Lys
            370                 375                 380

cct ccc tca tgc cca gct cct ggc ccc act ggg gca gcc agc atc gtc 1202
Pro Pro Ser Cys Pro Ala Pro Gly Pro Thr Gly Ala Ala Ser Ile Val
        385                 390                 395

ccc tct gtg ccg gga atg gcc ttg gac ctg tct cag atc ccc acc aag 1250
Pro Ser Val Pro Gly Met Ala Leu Asp Leu Ser Gln Ile Pro Thr Lys
    400                 405                 410

gag ctg gac cgc ttc atc cag gac cac ctg aag ccg agc ccc cag ttc 1298
Glu Leu Asp Arg Phe Ile Gln Asp His Leu Lys Pro Ser Pro Gln Phe
415                 420                 425                 430

cag gag cag gtg aaa aag gcc atc gac atc atc ttg cgc tgc ctc cat 1346
Gln Glu Gln Val Lys Lys Ala Ile Asp Ile Ile Leu Arg Cys Leu His
                435                 440                 445

gag aac tgt gtt cac aag gcc tca aga gtc agt aaa ggg ggc tca ttt 1394
Glu Asn Cys Val His Lys Ala Ser Arg Val Ser Lys Gly Gly Ser Phe
            450                 455                 460

ggc cgg ggc aca gac cta agg gat ggc tgt gat gtt gaa ctc atc atc 1442
Gly Arg Gly Thr Asp Leu Arg Asp Gly Cys Asp Val Glu Leu Ile Ile
        465                 470                 475

ttc ctc aac tgc ttc acg gac tac aag gac cag ggg ccc cgc cgc gca 1490
Phe Leu Asn Cys Phe Thr Asp Tyr Lys Asp Gln Gly Pro Arg Arg Ala
    480                 485                 490

gag atc ctt gat gag atg cga gcg cag cta gaa tcc tgg tgg cag gac 1538
Glu Ile Leu Asp Glu Met Arg Ala Gln Leu Glu Ser Trp Trp Gln Asp
495                 500                 505                 510

cag gtg ccc agc ctg agc ctt cag ttt cct gag cag aat gtg cct gag 1586

Gln Val Pro Ser Leu Ser Leu Gln Phe Pro Glu Gln Asn Val Pro Glu
                515                 520                 525

gct ctg cag ttc cag ctg gtg tcc aca gcc ctg aag agc tgg acg gat 1634
Ala Leu Gln Phe Gln Leu Val Ser Thr Ala Leu Lys Ser Trp Thr Asp
            530                 535                 540

gtt agc ctg ctg cct gcc ttc gat gct gtg ggg cag ctc agt tct ggc 1682
Val Ser Leu Leu Pro Ala Phe Asp Ala Val Gly Gln Leu Ser Ser Gly
        545                 550                 555

acc aaa cca aat ccc cag gtc tac tcg agg ctc ctc acc agt ggc tgc 1730
Thr Lys Pro Asn Pro Gln Val Tyr Ser Arg Leu Leu Thr Ser Gly Cys
    560                 565                 570

cag gag ggc gag cat aag gcc tgc ttc gca gag ctg cgg agg aac ttc 1778
Gln Glu Gly Glu His Lys Ala Cys Phe Ala Glu Leu Arg Arg Asn Phe
575                 580                 585                 590

atg aac att cgc cct gtc aag ctg aag aac ctg att ctg ctg gtg aag 1826
Met Asn Ile Arg Pro Val Lys Leu Lys Asn Leu Ile Leu Leu Val Lys
                595                 600                 605

cac tgg tac cgc cag gtt gcg gct cag aac aaa gga aaa gga cca gcc 1874
His Trp Tyr Arg Gln Val Ala Ala Gln Asn Lys Gly Lys Gly Pro Ala
            610                 615                 620

cct gcc tct ctg ccc cca gcc tat gcc ctg gag ctc ctc acc atc ttt 1922
Pro Ala Ser Leu Pro Pro Ala Tyr Ala Leu Glu Leu Leu Thr Ile Phe
        625                 630                 635

gcc tgg gag cag ggc tgc agg cag gat tgt ttc aac atg gcc caa ggc 1970
Ala Trp Glu Gln Gly Cys Arg Gln Asp Cys Phe Asn Met Ala Gln Gly
    640                 645                 650

ttc cgg acg gtg ctg ggg ctc gtg caa cag cat cag cag ctc tgt gtc 2018
Phe Arg Thr Val Leu Gly Leu Val Gln Gln His Gln Gln Leu Cys Val
655                 660                 665                 670

tac tgg acg gtc aac tat agc act gag gac cca gcc atg aga atg cac 2066
Tyr Trp Thr Val Asn Tyr Ser Thr Glu Asp Pro Ala Met Arg Met His
                675                 680                 685

ctt ctt ggc cag ctt cga aaa ccc aga ccc ctg gtc ctg gac ccc gct 2114
Leu Leu Gly Gln Leu Arg Lys Pro Arg Pro Leu Val Leu Asp Pro Ala
            690                 695                 700

gat ccc acc tgg aac gtg ggc cac ggt agc tgg gag ctg ttg gcc cag 2162
Asp Pro Thr Trp Asn Val Gly His Gly Ser Trp Glu Leu Leu Ala Gln
        705                 710                 715

gaa gca gca gcg ctg ggg atg cag gcc tgc ttt ctg agt aga gac ggg 2210
Glu Ala Ala Ala Leu Gly Met Gln Ala Cys Phe Leu Ser Arg Asp Gly
    720                 725                 730

aca tct gtg cag ccc tgg gat gtg atg cca gcc ctc ctt tac caa acc 2258
Thr Ser Val Gln Pro Trp Asp Val Met Pro Ala Leu Leu Tyr Gln Thr
735                 740                 745                 750

cca gct ggg gac ctt gac aag ttc atc agt gaa ttt ctc cag ccc aac 2306
Pro Ala Gly Asp Leu Asp Lys Phe Ile Ser Glu Phe Leu Gln Pro Asn
                755                 760                 765

cgc cag ttc ctg gcc cag gtg aac aag gcc gtt gat acc atc tgt tca 2354
Arg Gln Phe Leu Ala Gln Val Asn Lys Ala Val Asp Thr Ile Cys Ser

770                 775                 780

ttt ttg aag gaa aac tgc ttc cgg aat tct ccc atc aaa gtg atc aag          2402
Phe Leu Lys Glu Asn Cys Phe Arg Asn Ser Pro Ile Lys Val Ile Lys
        785                 790                 795

gtg gtc aag ggt ggc tct tca gcc aaa ggc aca gct ctg cga ggc cgc          2450
Val Val Lys Gly Gly Ser Ser Ala Lys Gly Thr Ala Leu Arg Gly Arg
    800                 805                 810

tca gat gcc gac ctc gtg gtg ttc ctc agc tgc ttc agc cag ttc act          2498
Ser Asp Ala Asp Leu Val Val Phe Leu Ser Cys Phe Ser Gln Phe Thr
815                 820                 825                 830

gag cag ggc aac aag cgg gcc gag atc atc tcc gag atc cga gcc cag          2546
Glu Gln Gly Asn Lys Arg Ala Glu Ile Ile Ser Glu Ile Arg Ala Gln
                835                 840                 845

ctg gag gca tgt caa cag gag cgg cag ttc gag gtc aag ttt gaa gtc          2594
Leu Glu Ala Cys Gln Gln Glu Arg Gln Phe Glu Val Lys Phe Glu Val
            850                 855                 860

tcc aaa tcg gag aat ccc cgc gtg ctg agc ttc tca ctg aca tcc cag          2642
Ser Lys Ser Glu Asn Pro Arg Val Leu Ser Phe Ser Leu Thr Ser Gln
        865                 870                 875

acg atg ctg gac cag agt gtg gac ttt gat gtg ctg cca gcc ttt gac          2690
Thr Met Leu Asp Gln Ser Val Asp Phe Asp Val Leu Pro Ala Phe Asp
    880                 885                 890

gcc cta ggc cag ctg gtc tct ggc tcc agg ccc agc tct caa gtc tac          2738
Ala Leu Gly Gln Leu Val Ser Gly Ser Arg Pro Ser Ser Gln Val Tyr
895                 900                 905                 910

gtc gac ctc atc cac agc tac agc aat gcg ggc gag tac tcc acc tgc          2786
Val Asp Leu Ile His Ser Tyr Ser Asn Ala Gly Glu Tyr Ser Thr Cys
                915                 920                 925

ttc aca gag cta caa cgg gac ttc atc atc tct cgc cct acc aag ctg          2834
Phe Thr Glu Leu Gln Arg Asp Phe Ile Ile Ser Arg Pro Thr Lys Leu
            930                 935                 940

aag agc ctg atc cgg ctg gtg aag cac tgg tac cag cag tgt acc aag          2882
Lys Ser Leu Ile Arg Leu Val Lys His Trp Tyr Gln Gln Cys Thr Lys
        945                 950                 955

atc tcc aag ggg aga ggc tcc cta ccc cca cag cac ggg ctg gaa ctc          2930
Ile Ser Lys Gly Arg Gly Ser Leu Pro Pro Gln His Gly Leu Glu Leu
    960                 965                 970

ctg act gtg tat gcc tgg gag cag ggc ggg aag gac tcc cag ttc aac          2978
Leu Thr Val Tyr Ala Trp Glu Gln Gly Gly Lys Asp Ser Gln Phe Asn
975                 980                 985                 990

atg gct gag ggc ttc cgc acg gtc ctg gag  ctg gtc acc cag tac  cgc          3026
Met Ala Glu Gly Phe Arg Thr Val Leu Glu  Leu Val Thr Gln Tyr  Arg
                995                 1000                1005

cag ctc tgt atc  tac tgg acc atc aac  tac aac gcc aag gac  aag          3071
Gln Leu Cys Ile  Tyr Trp Thr Ile Asn  Tyr Asn Ala Lys Asp  Lys
        1010                1015                1020

act gtt gga gac  ttc ctg aaa cag cag  ctt cag aag ccc agg  cct          3116
Thr Val Gly Asp  Phe Leu Lys Gln Gln  Leu Gln Lys Pro Arg  Pro
        1025                1030                1035

atc atc ctg gat ccg gct gac ccg aca ggc aac ctg ggc cac aat 3161
Ile Ile Leu Asp Pro Ala Asp Pro Thr Gly Asn Leu Gly His Asn
            1040                1045                1050

gcc cgc tgg gac ctg ctg gcc aag gaa gct gca gcc tgc aca tct 3206
Ala Arg Trp Asp Leu Leu Ala Lys Glu Ala Ala Ala Cys Thr Ser
            1055                1060                1065

gcc ctg tgc tgc atg ggg cgg aat ggc atc ccc atc cag cca tgg 3251
Ala Leu Cys Cys Met Gly Arg Asn Gly Ile Pro Ile Gln Pro Trp
            1070                1075                1080

cca gtg aag gct gct gtg gaa cag aag ctc atc tcc aag gag gac 3296
Pro Val Lys Ala Ala Val Glu Gln Lys Leu Ile Ser Lys Glu Asp
            1085                1090                1095

ctt tga ctatag 3308
Leu

<210> 24
<211> 1097
<212> PRT
<213> artificial sequence

<220>
<223> Synthetic Construct

<400> 24

Met Asp Leu Tyr Ser Thr Pro Ala Ala Ala Leu Asp Arg Phe Val Ala
1               5                   10                  15

Arg Lys Leu Gln Pro Arg Lys Glu Phe Val Glu Lys Ala Arg Arg Ala
            20                  25                  30

Leu Gly Ala Leu Ala Ala Ala Leu Arg Glu Arg Gly Gly Arg Leu Gly
        35                  40                  45

Ala Ala Ala Pro Arg Val Leu Lys Thr Val Lys Gly Gly Ser Ser Gly
    50                  55                  60

Arg Gly Thr Ala Leu Lys Gly Gly Cys Asp Ser Glu Leu Val Ile Phe
65                  70                  75                  80

Leu Asp Cys Phe Lys Ser Tyr Val Asp Gln Arg Ala Arg Arg Ala Glu
                85                  90                  95

Ile Leu Ser Glu Met Arg Ala Ser Leu Glu Ser Trp Trp Gln Asn Pro
            100                 105                 110

Val Pro Gly Leu Arg Leu Thr Phe Pro Glu Gln Ser Val Pro Gly Ala
        115                 120                 125

Leu Gln Phe Arg Leu Thr Ser Val Asp Leu Glu Asp Trp Met Asp Val

130                 135                 140

Ser Leu Val Pro Ala Phe Asn Val Leu Gly Gln Ala Gly Ser Gly Val
145                 150                 155                 160

Lys Pro Lys Pro Gln Val Tyr Ser Thr Leu Leu Asn Ser Gly Cys Gln
                165                 170                 175

Gly Gly Glu His Ala Ala Cys Phe Thr Glu Leu Arg Arg Asn Phe Val
            180                 185                 190

Asn Ile Arg Pro Ala Lys Leu Lys Asn Leu Ile Leu Leu Val Lys His
        195                 200                 205

Trp Tyr His Gln Val Cys Leu Gln Gly Leu Trp Lys Glu Thr Leu Pro
    210                 215                 220

Pro Val Tyr Ala Leu Glu Leu Leu Thr Ile Phe Ala Trp Glu Gln Gly
225                 230                 235                 240

Cys Lys Lys Asp Ala Phe Ser Leu Ala Glu Gly Leu Arg Thr Val Leu
                245                 250                 255

Gly Leu Ile Gln Gln His Gln His Leu Cys Val Phe Trp Thr Val Asn
            260                 265                 270

Tyr Gly Phe Glu Asp Pro Ala Val Gly Gln Phe Leu Gln Arg Gln Leu
        275                 280                 285

Lys Arg Pro Arg Pro Val Ile Leu Asp Pro Ala Asp Pro Thr Trp Asp
    290                 295                 300

Leu Gly Asn Gly Ala Ala Trp His Trp Asp Leu Leu Ala Gln Glu Ala
305                 310                 315                 320

Ala Ser Cys Tyr Asp His Pro Cys Phe Leu Arg Gly Met Gly Asp Pro
                325                 330                 335

Val Gln Ser Trp Lys Gly Pro Gly Leu Pro Arg Ala Gly Cys Ser Gly
            340                 345                 350

Leu Gly His Pro Ile Gln Leu Asp Pro Asn Gln Lys Thr Pro Glu Asn
        355                 360                 365

Ser Lys Ser Leu Asn Ala Val Tyr Pro Arg Ala Gly Ser Lys Pro Pro
    370                 375                 380

Ser Cys Pro Ala Pro Gly Pro Thr Gly Ala Ala Ser Ile Val Pro Ser
385                 390                 395                 400

Val Pro Gly Met Ala Leu Asp Leu Ser Gln Ile Pro Thr Lys Glu Leu
                405                 410                 415

Asp Arg Phe Ile Gln Asp His Leu Lys Pro Ser Pro Gln Phe Gln Glu
            420                 425                 430

Gln Val Lys Lys Ala Ile Asp Ile Ile Leu Arg Cys Leu His Glu Asn
        435                 440                 445

Cys Val His Lys Ala Ser Arg Val Ser Lys Gly Gly Ser Phe Gly Arg
    450                 455                 460

Gly Thr Asp Leu Arg Asp Gly Cys Asp Val Glu Leu Ile Ile Phe Leu
465                 470                 475                 480

Asn Cys Phe Thr Asp Tyr Lys Asp Gln Gly Pro Arg Arg Ala Glu Ile
                485                 490                 495

Leu Asp Glu Met Arg Ala Gln Leu Glu Ser Trp Trp Gln Asp Gln Val
            500                 505                 510

Pro Ser Leu Ser Leu Gln Phe Pro Glu Gln Asn Val Pro Glu Ala Leu
        515                 520                 525

Gln Phe Gln Leu Val Ser Thr Ala Leu Lys Ser Trp Thr Asp Val Ser
    530                 535                 540

Leu Leu Pro Ala Phe Asp Ala Val Gly Gln Leu Ser Ser Gly Thr Lys
545                 550                 555                 560

Pro Asn Pro Gln Val Tyr Ser Arg Leu Leu Thr Ser Gly Cys Gln Glu
                565                 570                 575

Gly Glu His Lys Ala Cys Phe Ala Glu Leu Arg Arg Asn Phe Met Asn
            580                 585                 590

Ile Arg Pro Val Lys Leu Lys Asn Leu Ile Leu Leu Val Lys His Trp
        595                 600                 605

Tyr Arg Gln Val Ala Ala Gln Asn Lys Gly Lys Gly Pro Ala Pro Ala
    610                 615                 620

Ser Leu Pro Pro Ala Tyr Ala Leu Glu Leu Leu Thr Ile Phe Ala Trp
625                 630                 635                 640

Glu Gln Gly Cys Arg Gln Asp Cys Phe Asn Met Ala Gln Gly Phe Arg
                645                 650                 655

Thr Val Leu Gly Leu Val Gln Gln His Gln Gln Leu Cys Val Tyr Trp
        660                 665                 670

Thr Val Asn Tyr Ser Thr Glu Asp Pro Ala Met Arg Met His Leu Leu
    675                 680                 685

Gly Gln Leu Arg Lys Pro Arg Pro Leu Val Leu Asp Pro Ala Asp Pro
    690                 695                 700

Thr Trp Asn Val Gly His Gly Ser Trp Glu Leu Leu Ala Gln Glu Ala
705                 710                 715                 720

Ala Ala Leu Gly Met Gln Ala Cys Phe Leu Ser Arg Asp Gly Thr Ser
                725                 730                 735

Val Gln Pro Trp Asp Val Met Pro Ala Leu Leu Tyr Gln Thr Pro Ala
            740                 745                 750

Gly Asp Leu Asp Lys Phe Ile Ser Glu Phe Leu Gln Pro Asn Arg Gln
        755                 760                 765

Phe Leu Ala Gln Val Asn Lys Ala Val Asp Thr Ile Cys Ser Phe Leu
    770                 775                 780

Lys Glu Asn Cys Phe Arg Asn Ser Pro Ile Lys Val Ile Lys Val Val
785                 790                 795                 800

Lys Gly Gly Ser Ser Ala Lys Gly Thr Ala Leu Arg Gly Arg Ser Asp
                805                 810                 815

Ala Asp Leu Val Val Phe Leu Ser Cys Phe Ser Gln Phe Thr Glu Gln
            820                 825                 830

Gly Asn Lys Arg Ala Glu Ile Ile Ser Glu Ile Arg Ala Gln Leu Glu
        835                 840                 845

Ala Cys Gln Gln Glu Arg Gln Phe Glu Val Lys Phe Glu Val Ser Lys
    850                 855                 860

Ser Glu Asn Pro Arg Val Leu Ser Phe Ser Leu Thr Ser Gln Thr Met
865                 870                 875                 880

Leu Asp Gln Ser Val Asp Phe Asp Val Leu Pro Ala Phe Asp Ala Leu
                885                 890                 895

Gly Gln Leu Val Ser Gly Ser Arg Pro Ser Ser Gln Val Tyr Val Asp
            900                 905                 910

Leu Ile His Ser Tyr Ser Asn Ala Gly Glu Tyr Ser Thr Cys Phe Thr
        915                 920                 925

Glu Leu Gln Arg Asp Phe Ile Ile Ser Arg Pro Thr Lys Leu Lys Ser
    930                 935                 940

Leu Ile Arg Leu Val Lys His Trp Tyr Gln Gln Cys Thr Lys Ile Ser
945                 950                 955                 960

Lys Gly Arg Gly Ser Leu Pro Pro Gln His Gly Leu Glu Leu Leu Thr
                965                 970                 975

Val Tyr Ala Trp Glu Gln Gly Gly Lys Asp Ser Gln Phe Asn Met Ala
            980                 985                 990

Glu Gly Phe Arg Thr Val Leu Glu Leu Val Thr Gln Tyr Arg Gln Leu
        995                 1000                1005

Cys Ile Tyr Trp Thr Ile Asn Tyr Asn Ala Lys Asp Lys Thr Val
    1010                1015                1020

Gly Asp Phe Leu Lys Gln Gln Leu Gln Lys Pro Arg Pro Ile Ile
    1025                1030                1035

Leu Asp Pro Ala Asp Pro Thr Gly Asn Leu Gly His Asn Ala Arg
    1040                1045                1050

Trp Asp Leu Leu Ala Lys Glu Ala Ala Ala Cys Thr Ser Ala Leu
    1055                1060                1065

Cys Cys Met Gly Arg Asn Gly Ile Pro Ile Gln Pro Trp Pro Val
    1070                1075                1080

Lys Ala Ala Val Glu Gln Lys Leu Ile Ser Lys Glu Asp Leu
    1085                1090                1095

<210> 25
<211> 2576
<212> DNA
<213> artificial sequence

<220>
<223> OAS3 truncated protein c-myc tag

<220>
<221> CDS
<222> (9)..(2570)

<400> 25
gcggccgc atg gac ttg tac agc acc ccg gcc gct gcg ctg gac agg ttc 50
Met Asp Leu Tyr Ser Thr Pro Ala Ala Ala Leu Asp Arg Phe
1               5                   10

gtg gcc aga aag ctg cag ccg cgg aag gag ttc gta gag aag gcg cgg 98
Val Ala Arg Lys Leu Gln Pro Arg Lys Glu Phe Val Glu Lys Ala Arg
15                  20                  25                  30

cgc gct ctg ggc gcc ctg gcc gct gct ctg agg gag cgc ggg ggc cgc 146
Arg Ala Leu Gly Ala Leu Ala Ala Ala Leu Arg Glu Arg Gly Gly Arg
                35                  40                  45

ctc ggt gct gct gcc ccg cgg gtg ctg aaa act gtc aag gga ggc tcc 194
Leu Gly Ala Ala Ala Pro Arg Val Leu Lys Thr Val Lys Gly Gly Ser
            50                  55                  60

tcg ggc cgg ggc aca gct ctc aag ggt ggc tgt gat tct gaa ctt gtc 242
Ser Gly Arg Gly Thr Ala Leu Lys Gly Gly Cys Asp Ser Glu Leu Val
        65                  70                  75

atc ttc ctc gac tgc ttc aag agc tat gtg gac cag agg gcc cgc cgt 290
Ile Phe Leu Asp Cys Phe Lys Ser Tyr Val Asp Gln Arg Ala Arg Arg
    80                  85                  90

gca gag atc ctc agt gag atg cgg gca tcg ctg gaa tcc tgg tgg cag 338
Ala Glu Ile Leu Ser Glu Met Arg Ala Ser Leu Glu Ser Trp Trp Gln
95                  100                 105                 110

aac cca gtc cct ggt ctg aga ctc acg ttt cct gag cag agc gtg cct 386
Asn Pro Val Pro Gly Leu Arg Leu Thr Phe Pro Glu Gln Ser Val Pro
                115                 120                 125

ggg gcc ctg cag ttc cgc ctg aca tcc gta gat ctt gag gac tgg atg 434
Gly Ala Leu Gln Phe Arg Leu Thr Ser Val Asp Leu Glu Asp Trp Met
            130                 135                 140

gat gtt agc ctg gtg cct gcc ttc aat gtc ctg ggt cag gcc ggc tcc 482
Asp Val Ser Leu Val Pro Ala Phe Asn Val Leu Gly Gln Ala Gly Ser
        145                 150                 155

ggc gtc aaa ccc aag cca caa gtc tac tct acc ctc ctc aac agt ggc 530
Gly Val Lys Pro Lys Pro Gln Val Tyr Ser Thr Leu Leu Asn Ser Gly
    160                 165                 170

tgc caa ggg ggc gag cat gcg gcc tgc ttc aca gag ctg cgg agg aac 578
Cys Gln Gly Gly Glu His Ala Ala Cys Phe Thr Glu Leu Arg Arg Asn
175                 180                 185                 190

ttt gtg aac att cgc cca gcc aag ttg aag aac cta atc ttg ctg gtg 626
Phe Val Asn Ile Arg Pro Ala Lys Leu Lys Asn Leu Ile Leu Leu Val
                195                 200                 205

aag cac tgg tac cac cag gtg tgc cta cag ggg ttg tgg aag gag acg 674
Lys His Trp Tyr His Gln Val Cys Leu Gln Gly Leu Trp Lys Glu Thr
            210                 215                 220

ctg ccc ccg gtc tat gcc ctg gaa ttg ctg acc atc ttc gcc tgg gag 722
Leu Pro Pro Val Tyr Ala Leu Glu Leu Leu Thr Ile Phe Ala Trp Glu
        225                 230                 235

cag ggc tgt aag aag gat gct ttc agc cta gcc gaa ggc ctc cga act 770
Gln Gly Cys Lys Lys Asp Ala Phe Ser Leu Ala Glu Gly Leu Arg Thr
    240                 245                 250

gtc ctg ggc ctg atc caa cag cat cag cac ctg tgt gtt ttc tgg act 818
Val Leu Gly Leu Ile Gln Gln His Gln His Leu Cys Val Phe Trp Thr
255                 260                 265                 270

gtc aac tat ggc ttc gag gac cct gca gtt ggg cag ttc ttg cag cgg 866
Val Asn Tyr Gly Phe Glu Asp Pro Ala Val Gly Gln Phe Leu Gln Arg
                275                 280                 285

cag ctt aag aga ccc agg cct gtg atc ctg gac cca gct gac ccc aca 914
Gln Leu Lys Arg Pro Arg Pro Val Ile Leu Asp Pro Ala Asp Pro Thr
            290                 295                 300

tgg gac ctg ggg aat ggg gca gcc tgg cac tgg gat ttg cta gcc cag 962
Trp Asp Leu Gly Asn Gly Ala Ala Trp His Trp Asp Leu Leu Ala Gln
        305                 310                 315

gag gca gca tcc tgc tat gac cac cca tgc ttt ctg agg ggg atg ggg 1010
Glu Ala Ala Ser Cys Tyr Asp His Pro Cys Phe Leu Arg Gly Met Gly
    320                 325                 330

gac cca gtg cag tct tgg aag ggg ccg ggc ctt cca cgt gct gga tgc 1058
Asp Pro Val Gln Ser Trp Lys Gly Pro Gly Leu Pro Arg Ala Gly Cys
335                 340                 345                 350

tca ggt ttg ggc cac ccc atc cag cta gac cct aac cag aag acc cct 1106
Ser Gly Leu Gly His Pro Ile Gln Leu Asp Pro Asn Gln Lys Thr Pro
                355                 360                 365

gaa aac agc aag agc ctc aat gct gtg tac cca aga gca ggg agc aaa 1154
Glu Asn Ser Lys Ser Leu Asn Ala Val Tyr Pro Arg Ala Gly Ser Lys
            370                 375                 380

cct ccc tca tgc cca gct cct ggc ccc act ggg gca gcc agc atc gtc 1202
Pro Pro Ser Cys Pro Ala Pro Gly Pro Thr Gly Ala Ala Ser Ile Val
        385                 390                 395

ccc tct gtg ccg gga atg gcc ttg gac ctg tct cag atc ccc acc aag 1250
Pro Ser Val Pro Gly Met Ala Leu Asp Leu Ser Gln Ile Pro Thr Lys
    400                 405                 410

gag ctg gac cgc ttc atc cag gac cac ctg aag ccg agc ccc cag ttc 1298
Glu Leu Asp Arg Phe Ile Gln Asp His Leu Lys Pro Ser Pro Gln Phe
415                 420                 425                 430

cag gag cag gtg aaa aag gcc atc gac atc atc ttg cgc tgc ctc cat 1346
Gln Glu Gln Val Lys Lys Ala Ile Asp Ile Ile Leu Arg Cys Leu His
                435                 440                 445

gag aac tgt gtt cac aag gcc tca aga gtc agt aaa ggg ggc tca ttt 1394
Glu Asn Cys Val His Lys Ala Ser Arg Val Ser Lys Gly Gly Ser Phe
            450                 455                 460

ggc cgg ggc aca gac cta agg gat ggc tgt gat gtt gaa ctc atc atc 1442
Gly Arg Gly Thr Asp Leu Arg Asp Gly Cys Asp Val Glu Leu Ile Ile
        465                 470                 475

ttc ctc aac tgc ttc acg gac tac aag gac cag ggg ccc cgc cgc gca 1490
Phe Leu Asn Cys Phe Thr Asp Tyr Lys Asp Gln Gly Pro Arg Arg Ala
    480                 485                 490

gag atc ctt gat gag atg cga gcg cag cta gaa tcc tgg tgg cag gac 1538
Glu Ile Leu Asp Glu Met Arg Ala Gln Leu Glu Ser Trp Trp Gln Asp
495                 500                 505                 510

cag gtg ccc agc ctg agc ctt cag ttt cct gag cag aat gtg cct gag 1586
Gln Val Pro Ser Leu Ser Leu Gln Phe Pro Glu Gln Asn Val Pro Glu
                515                 520                 525

gct ctg cag ttc cag ctg gtg tcc aca gcc ctg aag agc tgg acg gat 1634

Ala Leu Gln Phe Gln Leu Val Ser Thr Ala Leu Lys Ser Trp Thr Asp
            530                 535                 540

gtt agc ctg ctg cct gcc ttc gat gct gtg ggg cag ctc agt tct ggc 1682
Val Ser Leu Leu Pro Ala Phe Asp Ala Val Gly Gln Leu Ser Ser Gly
        545                 550                 555

acc aaa cca aat ccc cag gtc tac tcg agg ctc ctc acc agt ggc tgc 1730
Thr Lys Pro Asn Pro Gln Val Tyr Ser Arg Leu Leu Thr Ser Gly Cys
    560                 565                 570

cag gag ggc gag cat aag gcc tgc ttc gca gag ctg cgg agg aac ttc 1778
Gln Glu Gly Glu His Lys Ala Cys Phe Ala Glu Leu Arg Arg Asn Phe
575                 580                 585                 590

atg aac att cgc cct gtc aag ctg aag aac ctg att ctg ctg gtg aag 1826
Met Asn Ile Arg Pro Val Lys Leu Lys Asn Leu Ile Leu Leu Val Lys
                595                 600                 605

cac tgg tac cgc cag gtt gcg gct cag aac aaa gga aaa gga cca gcc 1874
His Trp Tyr Arg Gln Val Ala Ala Gln Asn Lys Gly Lys Gly Pro Ala
            610                 615                 620

cct gcc tct ctg ccc cca gcc tat gcc ctg gag ctc ctc acc atc ttt 1922
Pro Ala Ser Leu Pro Pro Ala Tyr Ala Leu Glu Leu Leu Thr Ile Phe
        625                 630                 635

gcc tgg gag cag ggc tgc agg cag gat tgt ttc aac atg gcc caa ggc 1970
Ala Trp Glu Gln Gly Cys Arg Gln Asp Cys Phe Asn Met Ala Gln Gly
    640                 645                 650

ttc cgg acg gtg ctg ggg ctc gtg caa cag cat cag cag ctc tgt gtc 2018
Phe Arg Thr Val Leu Gly Leu Val Gln Gln His Gln Gln Leu Cys Val
655                 660                 665                 670

tac tgg acg gtc aac tat agc act gag gac cca gcc atg aga atg cac 2066
Tyr Trp Thr Val Asn Tyr Ser Thr Glu Asp Pro Ala Met Arg Met His
                675                 680                 685

ctt ctt ggc cag ctt cga aaa ccc aga ccc ctg gtc ctg gac ccc gct 2114
Leu Leu Gly Gln Leu Arg Lys Pro Arg Pro Leu Val Leu Asp Pro Ala
            690                 695                 700

gat ccc acc tgg aac gtg ggc cac ggt agc tgg gag ctg ttg gcc cag 2162
Asp Pro Thr Trp Asn Val Gly His Gly Ser Trp Glu Leu Leu Ala Gln
        705                 710                 715

gaa gca gca gcg ctg ggg atg cag gcc tgc ttt ctg agt aga gac ggg 2210
Glu Ala Ala Ala Leu Gly Met Gln Ala Cys Phe Leu Ser Arg Asp Gly
    720                 725                 730

aca tct gtg cag ccc tgg gat gtg atg cca gcc ctc ctt tac caa acc 2258
Thr Ser Val Gln Pro Trp Asp Val Met Pro Ala Leu Leu Tyr Gln Thr
735                 740                 745                 750

cca gct ggg gac ctt gac aag ttc atc agt gaa ttt ctc cag ccc aac 2306
Pro Ala Gly Asp Leu Asp Lys Phe Ile Ser Glu Phe Leu Gln Pro Asn
                755                 760                 765

cgc cag ttc ctg gcc cag gtg aac aag gcc gtt gat acc atc tgt tca 2354
Arg Gln Phe Leu Ala Gln Val Asn Lys Ala Val Asp Thr Ile Cys Ser
            770                 775                 780

ttt ttg aag gaa aac tgc ttc cgg aat tct ccc atc aaa gtg atc aag 2402
Phe Leu Lys Glu Asn Cys Phe Arg Asn Ser Pro Ile Lys Val Ile Lys

785                 790                 795

gtg gtc aag ggt ggc tct tca gcc aaa ggc aca gct ctg cga ggc cgc      2450
Val Val Lys Gly Gly Ser Ser Ala Lys Gly Thr Ala Leu Arg Gly Arg
    800                 805                 810

tca gat gcc gac ctc gtg gtg ttc ctc agc tgc ttc agc cag ttc act      2498
Ser Asp Ala Asp Leu Val Val Phe Leu Ser Cys Phe Ser Gln Phe Thr
815                 820                 825                 830

gag cag ggc aac aag cgg gcc gag atc atc tcc gag atc gaa cag aag      2546
Glu Gln Gly Asn Lys Arg Ala Glu Ile Ile Ser Glu Ile Glu Gln Lys
                835                 840                 845

ctc atc tcc aag gag gac ctt tga ctatag                               2576
Leu Ile Ser Lys Glu Asp Leu
            850

<210>  26
<211>  853
<212>  PRT
<213>  artificial sequence

<220>
<223>  Synthetic Construct

<400>  26

Met Asp Leu Tyr Ser Thr Pro Ala Ala Ala Leu Asp Arg Phe Val Ala
1               5                   10                  15

Arg Lys Leu Gln Pro Arg Lys Glu Phe Val Glu Lys Ala Arg Arg Ala
            20                  25                  30

Leu Gly Ala Leu Ala Ala Ala Leu Arg Glu Arg Gly Gly Arg Leu Gly
        35                  40                  45

Ala Ala Ala Pro Arg Val Leu Lys Thr Val Lys Gly Gly Ser Ser Gly
    50                  55                  60

Arg Gly Thr Ala Leu Lys Gly Gly Cys Asp Ser Glu Leu Val Ile Phe
65                  70                  75                  80

Leu Asp Cys Phe Lys Ser Tyr Val Asp Gln Arg Ala Arg Arg Ala Glu
                85                  90                  95

Ile Leu Ser Glu Met Arg Ala Ser Leu Glu Ser Trp Trp Gln Asn Pro
            100                 105                 110

Val Pro Gly Leu Arg Leu Thr Phe Pro Glu Gln Ser Val Pro Gly Ala
        115                 120                 125

Leu Gln Phe Arg Leu Thr Ser Val Asp Leu Glu Asp Trp Met Asp Val
    130                 135                 140

Ser Leu Val Pro Ala Phe Asn Val Leu Gly Gln Ala Gly Ser Gly Val
145                 150                 155                 160

Lys Pro Lys Pro Gln Val Tyr Ser Thr Leu Leu Asn Ser Gly Cys Gln
                165                 170                 175

Gly Gly Glu His Ala Ala Cys Phe Thr Glu Leu Arg Arg Asn Phe Val
            180                 185                 190

Asn Ile Arg Pro Ala Lys Leu Lys Asn Leu Ile Leu Leu Val Lys His
        195                 200                 205

Trp Tyr His Gln Val Cys Leu Gln Gly Leu Trp Lys Glu Thr Leu Pro
    210                 215                 220

Pro Val Tyr Ala Leu Glu Leu Leu Thr Ile Phe Ala Trp Glu Gln Gly
225                 230                 235                 240

Cys Lys Lys Asp Ala Phe Ser Leu Ala Glu Gly Leu Arg Thr Val Leu
                245                 250                 255

Gly Leu Ile Gln Gln His Gln His Leu Cys Val Phe Trp Thr Val Asn
            260                 265                 270

Tyr Gly Phe Glu Asp Pro Ala Val Gly Gln Phe Leu Gln Arg Gln Leu
        275                 280                 285

Lys Arg Pro Arg Pro Val Ile Leu Asp Pro Ala Asp Pro Thr Trp Asp
    290                 295                 300

Leu Gly Asn Gly Ala Ala Trp His Trp Asp Leu Leu Ala Gln Glu Ala
305                 310                 315                 320

Ala Ser Cys Tyr Asp His Pro Cys Phe Leu Arg Gly Met Gly Asp Pro
                325                 330                 335

Val Gln Ser Trp Lys Gly Pro Gly Leu Pro Arg Ala Gly Cys Ser Gly
            340                 345                 350

Leu Gly His Pro Ile Gln Leu Asp Pro Asn Gln Lys Thr Pro Glu Asn
        355                 360                 365

Ser Lys Ser Leu Asn Ala Val Tyr Pro Arg Ala Gly Ser Lys Pro Pro
    370                 375                 380

Ser Cys Pro Ala Pro Gly Pro Thr Gly Ala Ala Ser Ile Val Pro Ser
385                 390                 395                 400

Val Pro Gly Met Ala Leu Asp Leu Ser Gln Ile Pro Thr Lys Glu Leu

                405                 410                 415

Asp Arg Phe Ile Gln Asp His Leu Lys Pro Ser Pro Gln Phe Gln Glu
            420                 425                 430

Gln Val Lys Lys Ala Ile Asp Ile Ile Leu Arg Cys Leu His Glu Asn
        435                 440                 445

Cys Val His Lys Ala Ser Arg Val Ser Lys Gly Gly Ser Phe Gly Arg
    450                 455                 460

Gly Thr Asp Leu Arg Asp Gly Cys Asp Val Glu Leu Ile Ile Phe Leu
465                 470                 475                 480

Asn Cys Phe Thr Asp Tyr Lys Asp Gln Gly Pro Arg Arg Ala Glu Ile
                485                 490                 495

Leu Asp Glu Met Arg Ala Gln Leu Glu Ser Trp Trp Gln Asp Gln Val
            500                 505                 510

Pro Ser Leu Ser Leu Gln Phe Pro Glu Gln Asn Val Pro Glu Ala Leu
        515                 520                 525

Gln Phe Gln Leu Val Ser Thr Ala Leu Lys Ser Trp Thr Asp Val Ser
    530                 535                 540

Leu Leu Pro Ala Phe Asp Ala Val Gly Gln Leu Ser Ser Gly Thr Lys
545                 550                 555                 560

Pro Asn Pro Gln Val Tyr Ser Arg Leu Leu Thr Ser Gly Cys Gln Glu
                565                 570                 575

Gly Glu His Lys Ala Cys Phe Ala Glu Leu Arg Arg Asn Phe Met Asn
            580                 585                 590

Ile Arg Pro Val Lys Leu Lys Asn Leu Ile Leu Leu Val Lys His Trp
        595                 600                 605

Tyr Arg Gln Val Ala Ala Gln Asn Lys Gly Lys Gly Pro Ala Pro Ala
    610                 615                 620

Ser Leu Pro Pro Ala Tyr Ala Leu Glu Leu Leu Thr Ile Phe Ala Trp
625                 630                 635                 640

Glu Gln Gly Cys Arg Gln Asp Cys Phe Asn Met Ala Gln Gly Phe Arg
                645                 650                 655

Thr Val Leu Gly Leu Val Gln Gln His Gln Gln Leu Cys Val Tyr Trp
            660                 665                 670

Thr Val Asn Tyr Ser Thr Glu Asp Pro Ala Met Arg Met His Leu Leu
        675                 680                 685

Gly Gln Leu Arg Lys Pro Arg Pro Leu Val Leu Asp Pro Ala Asp Pro
    690                 695                 700

Thr Trp Asn Val Gly His Gly Ser Trp Glu Leu Leu Ala Gln Glu Ala
705                 710                 715                 720

Ala Ala Leu Gly Met Gln Ala Cys Phe Leu Ser Arg Asp Gly Thr Ser
                725                 730                 735

Val Gln Pro Trp Asp Val Met Pro Ala Leu Leu Tyr Gln Thr Pro Ala
            740                 745                 750

Gly Asp Leu Asp Lys Phe Ile Ser Glu Phe Leu Gln Pro Asn Arg Gln
        755                 760                 765

Phe Leu Ala Gln Val Asn Lys Ala Val Asp Thr Ile Cys Ser Phe Leu
    770                 775                 780

Lys Glu Asn Cys Phe Arg Asn Ser Pro Ile Lys Val Ile Lys Val Val
785                 790                 795                 800

Lys Gly Gly Ser Ser Ala Lys Gly Thr Ala Leu Arg Gly Arg Ser Asp
                805                 810                 815

Ala Asp Leu Val Val Phe Leu Ser Cys Phe Ser Gln Phe Thr Glu Gln
            820                 825                 830

Gly Asn Lys Arg Ala Glu Ile Ile Ser Glu Ile Glu Gln Lys Leu Ile
        835                 840                 845

Ser Lys Glu Asp Leu
    850

<210>  27
<211>  6646
<212>  DNA
<213>  Homo sapiens

<400>  27
gttcggagag ccgggcggga aaacgaaacc agaaatccga aggccgcgcc agagccctgc      60

ttccccttgc acctgcgccg ggcggccatg gacttgtaca gcaccccggc cgctgcgctg     120

gacaggttcg tggccagaag gctgcagccg cggaaggagt tcgtagagaa ggcgcggcgc     180

gctctgggcg ccctggccgc tgccctgagg gagcgcgggg gccgcctcgg tgctgctgcc     240

ccgcgggtgc tgaaaactgt caagggaggc tcctcgggcc ggggcacagc tctcaagggt     300

ggctgtgatt ctgaacttgt catcttcctc gactgcttca agagctatgt ggaccagagg 360
gcccgccgtg cagagatcct cagtgagatg cgggcatcgc tggaatcctg gtggcagaac 420
ccagtccctg gtctgagact cacgtttcct gagcagagcg tgcctggggc cctgcagttc 480
cgcctgacat ccgtagatct tgaggactgg atggatgtta gcctggtgcc tgccttcaat 540
gtcctgggtc aggccggctc cggcgtcaaa cccaagccac aagtctactc taccctcctc 600
aacagtggct gccaaggggg cgagcatgcg gcctgcttca cagagctgcg gaggaacttt 660
gtgaacattc gcccagccaa gttgaagaac ctaatcttgc tggtgaagca ctggtaccac 720
caggtgtgcc tacaggggtt gtggaaggag acgctgcccc cggtctatgc cctggaattg 780
ctgaccatct tcgcctggga gcagggctgt aagaaggatg ctttcagcct agccgaaggc 840
ctccgaactg tcctgggcct gatccaacag catcagcacc tgtgtgtttt ctggactgtc 900
aactatggct tcgaggaccc tgcagttggg cagttcttgc agcggcagct taagagaccc 960
aggcctgtga tcctggaccc agctgacccc acatgggacc tggggaatgg ggcagcctgg 1020
cactgggatt tgctagccca ggaggcagca tcctgctatg accacccatg ctttctgagg 1080
gggatggggg acccagtgca gtcttggaag gggccgggcc ttccacgtgc tggatgctca 1140
ggtttgggcc accccatcca gctagaccct aaccagaaga cccctgaaaa cagcaagagc 1200
ctcaatgctg tgtacccaag agcagggagc aaacctccct catgcccagc tcctggcccc 1260
actggggcag ccagcatcgt cccctctgtg ccgggaatgg ccttggacct gtctcagatc 1320
cccaccaagg agctggaccg cttcatccag gaccacctga agccgagccc ccagttccag 1380
gagcaggtga aaaaggccat cgacatcatc ttgcgctgcc tccatgagaa ctgtgttcac 1440
aaggcctcaa gagtcagtaa agggggctca tttggccggg gcacagacct aagggatggc 1500
tgtgatgttg aactcatcat cttcctcaac tgcttcacgg actacaagga ccaggggccc 1560
cgccgcgcag agatccttga tgagatgcga gcgcagctag aatcctggtg gcaggaccag 1620
gtgcccagcc tgagccttca gtttcctgag cagaatgtgc ctgaggctct gcagttccag 1680
ctggtgtcca cagccctgaa gagctggacg gatgttagcc tgctgcctgc cttcgatgct 1740
gtggggcagc tcagttctgg caccaaacca aatccccagg tctactcgag gctcctcacc 1800
agtggctgcc aggagggcga gcataaggcc tgcttcgcag agctgcggag gaacttcatg 1860
aacattcgcc ctgtcaagct gaagaacctg attctgctgg tgaagcactg gtaccgccag 1920
gttgcggctc agaacaaagg aaaaggacca gcccctgcct ctctgccccc agcctatgcc 1980
ctggagctcc tcaccatctt tgcctgggag cagggctgca ggcaggattg tttcaacatg 2040
gcccaaggct tccggacggt gctggggctc gtgcaacagc atcagcagct ctgtgtctac 2100
tggacggtca actatagcac tgaggaccca gccatgagaa tgcaccttct tggccagctt 2160
cgaaaaccca gacccctggt cctggacccc gctgatccca cctggaacgt gggccacggt 2220

agctgggagc tgttggccca ggaagcagca gcgctgggga tgcaggcctg ctttctgagt 2280

agagacggga catctgtgca gccctgggat gtgatgccag ccctccttta ccaaacccca 2340

gctggggacc ttgacaagtt catcagtgaa tttctccagc ccaaccgcca gttcctggcc 2400

caggtgaaca aggccgttga taccatctgt tcatttttga aggaaaactg cttccggaat 2460

tctcccatca aagtgatcaa ggtggtcaag ggtggctctt cagccaaagg cacagctctg 2520

cgaggccgct cagatgccga cctcgtggtg ttcctcagct gcttcagcca gttcactgag 2580

cagggcaaca agcgggccga gatcatctcc gagatccgag cccagctgga ggcatgtcaa 2640

caggagcggc agttcgaggt caagtttgaa gtctccaaat gggagaatcc ccgcgtgctg 2700

agcttctcac tgacatccca gacgatgctg gaccagagtg tggactttga tgtgctgcca 2760

gcctttgacg ccctaggcca gctggtctct ggctccaggc ccagctctca agtctacgtc 2820

gacctcatcc acagctacag caatgcgggc gagtactcca cctgcttcac agagctacaa 2880

cgggacttca tcatctctcg ccctaccaag ctgaagagcc tgatccggct ggtgaagcac 2940

tggtaccagc agtgtaccaa gatctccaag gggagaggct ccctaccccc acagcacggg 3000

ctggaactcc tgactgtgta tgcctgggag cagggcggga aggactccca gttcaacatg 3060

gctgagggct tccgcacggt cctggagctg gtcacccagt accgccagct ctgtatctac 3120

tggaccatca actacaacgc caaggacaag actgttggag acttcctgaa acagcagctt 3180

cagaagccca ggcctatcat cctggatccg gctgacccga caggcaacct gggccacaat 3240

gcccgctggg acctgctggc caaggaagct gcagcctgca catctgccct gtgctgcatg 3300

ggacggaatg gcatccccat ccagccatgg ccagtgaagg ctgctgtgtg aagttgagaa 3360

aatcagcggt cctactggat gaagagaaga tggacaccag ccctcagcat gaggaaattc 3420

agggtcccct accagatgag agagattgtg tacatgtgtg tgtgagcaca tgtgtgcatg 3480

tgtgtgcaca cgtgtgcatg tgtgtgtttt agtgaatctg ctctcccagc tcacacactc 3540

ccctgcctcc catggcttac acactaggat ccagactcca tggtttgaca ccagcctgcg 3600

tttgcagctt ctctgtcact tccatgactc tatcctcata ccaccactgc tgcttcccac 3660

ccagctgaga atgccccctc ctccctgact cctctctgcc catgcaaatt agctcacatc 3720

tttcctcctg ctgcaatcca tcccttcctc ccattggcct ctccttgcca aatctaaata 3780

gtttatatag ggatggcaga gagttcccat ctcatctgtc agccacagtc atttggtact 3840

ggctacctgg agccttatct tctgaagggt tttaaagaat ggccaattag ctgagaagaa 3900

ttatctaatc aattagtgat gtctgccatg gatgcagtag aggaaagtgg tggtacaagt 3960

gccatgattg attagcaatg tctgcactgg atacggaaaa aagaaggtgc ttgcaggttt 4020

acagtgtata tgtgggctat tgaagagccc tctgagctcg gttgctagca ggagagcatg 4080

cccatattgg cttactttgt ctgccacaga cacagacaga gggagttggg acatgcatgc 4140

tatggggacc ctcttgttgg acacctaatt ggatgcctct tcatgagagg cctccttttc 4200

ttcacctttt atgctgcact cctcccctag tttacacatc ttgatgctgt ggctcagttt 4260

gccttcctga atttttattg ggtccctgtt ttctctccta acatgctgag attctgcatc 4320

cccacagcct aaactgagcc agtggccaaa caaccgtgct cagcctgttt ctctctgccc 4380

tctagagcaa ggcccaccag gtccatccag gaggctctcc tgacctcaag tccaacaaca 4440

gtgtccacac tagtcaaggt tcagcccaga aaacagaaag cactctagga atcttaggca 4500

gaaagggatt ttatctaaat cactggaaag gctggaggag cagaaggcag aggccaccac 4560

tggactattg gtttcaatat tagaccactg tagccgaatc agaggccaga gagcagccac 4620

tgctactgct aatgccacca ctacccctgc catcactgcc ccacatggac aaaactggag 4680

tcgagaccta ggttagattc ctgcaaccac aaacatccat cagggatggc cagctgccag 4740

agctgcggga agacggatcc cacctccctt tcttagcaga atctaaatta cagccagacc 4800

tctggctgca gaggagtctg agacatgtat gattgaatgg gtgccaagtg ccagggggcg 4860

gagtccccag cagatgcatc ctggccatct gttgcgtgga tgagggagtg ggtctatctc 4920

agaggaagga acaggaaaca aagaaaggaa gccactgaac atcccttctc tgctccacag 4980

gagtgcctta gacagcctga ctctccacaa accactgtta aaacttacct gctaggaatg 5040

ctagattgaa tgggatggga agagccttcc ctcattattg tcattcttgg agagaggtga 5100

gcaaccaagg gaagctcctc tgattcacct agaacctgtt ctctgccgtc tttggctcag 5160

cctacagaga ctagagtagg tgaagggaca gaggacaggg cttctaatac ctgtgccata 5220

ttgacagcct ccatccctgt cccccatctt ggtgctgaac caacgctaag ggcaccttct 5280

tagactcacc tcatcgatac tgcctggtaa tccaaagcta gaactctcag gaccccaaac 5340

tccacctctt ggattggccc tggctgctgc cacacacata tccaagagct cagggccagt 5400

tctggtgggc agcagagacc tgctctgcca agttgtccag cagcagagtg gccctggcct 5460

gggcatcaca agccagtgat gctcctggga agaccaggtg gcaggtcgca gttgggtacc 5520

ttccattccc accacacaga ctctgggcct ccccgcaaaa tggctccaga attagagtaa 5580

ttatgagatg gtgggaacca gagcaactca ggtgcatgat acaaggagag gttgtcatct 5640

gggtagggca gagaggaggg cttgctcatc tgaacagggg tgtatttcat tccaggccct 5700

cagtctttgg caatggccac cctggtgttg gcatattggc cccactgtaa cttttggggg 5760

cttcccggtc tagccacacc ctcggatgga aagacttgac tgcataaaga tgtcagttct 5820

ccctgagttg attgataggc ttaatggtca ccctaaaaac acccacatat gcttttcgat 5880

ggaaccaggt aagttgacgc taaagttctt atggaaaaat acacacgcaa tagctaggaa 5940

aacacaggga aagaagagtt ctgagcaggg cctagtctta gccaatatta aaacatacta 6000

tgaagcctct gatacttaaa cagcatggcg ctggtacgta aatagaccaa tgcagttagg 6060

tggctctttc caagactctg gggaaaaaag tagtaaaaag ctaaatgcaa tcaatcagca 6120

attgaaagct aagtgagaga gccagagggc ctccttggtg gtaaaagagg gttgcatttc 6180

ttgcagccag aaggcagaga aagtgaagac caagtccaga actgaatcct aagaaatgca 6240

ggactgcaaa gaaattggtg tgtgtgtgtg tgtgtgtgtg tgtgtgtgtg tttaattttt 6300

aaaaagtttt tattgagata caagtcaata ccataaagct ctcacccttc taaagtgtac 6360

aattcagtgg tgtgagtata ttcataagat ttatacttgg tgtctattca taagacttat 6420

atccagcata ttcataacta gagccatatc acagatgcat tcatcataat aattccagac 6480

attttcatca ccctaaaagg aaaccctgaa acccattagc agtcattccc cattcctcca 6540

acccattctc tccctaatcc ctagaaacca ccaatctgct gtgtatttca tctattgcca 6600

acatttcata taaatggcat catacaaaaa aaaaaaaaaa aaaaaa 6646

<210> 28
<211> 6646
<212> DNA
<213> Homo sapiens

<400> 28
gttcggagag ccgggcggga aaacgaaacc agaaatccga aggccgcgcc agagccctgc 60

ttccccttgc acctgcgccg ggcggccatg gacttgtaca gcaccccggc cgctgcgctg 120

gacaggttcg tggccagaag gctgcagccg cggaaggagt tcgtagagaa ggcgcggcgc 180

gctctgggcg ccctggccgc tgccctgagg gagcgcgggg gccgcctcgg tgctgctgcc 240

ccgcgggtgc tgaaaactgt caagggaggc tcctcgggcc ggggcacagc tctcaagggt 300

ggctgtgatt ctgaacttgt catcttcctc gactgcttca agagctatgt ggaccagagg 360

gcccgccgtg cagagatcct cagtgagatg cgggcatcgc tggaatcctg gtggcagaac 420

ccagtccctg gtctgagact cacgtttcct gagcagagcg tgcctggggc cctgcagttc 480

cgcctgacat ccgtagatct tgaggactgg atggatgtta gcctggtgcc tgccttcaat 540

gtcctgggtc aggccggctc cggcgtcaaa cccaagccac aagtctactc taccctcctc 600

aacagtggct gccaaggggg cgagcatgcg gcctgcttca cagagctgcg gaggaacttt 660

gtgaacattc gcccagccaa gttgaagaac ctaatcttgc tggtgaagca ctggtaccac 720

caggtgtgcc tacaggggtt gtggaaggag acgctgcccc cggtctatgc cctggaattg 780

ctgaccatct tcgcctggga gcagggctgt aagaaggatg ctttcagcct agccgaaggc 840

ctccgaactg tcctgggcct gatccaacag catcagcacc tgtgtgtttt ctggactgtc 900

aactatggct tcgaggaccc tgcagttggg cagttcttgc agcggcagct taagagaccc 960

aggcctgtga tcctggaccc agctgacccc acatgggacc tggggaatgg ggcagcctgg 1020

cactgggatt tgctagccca ggaggcagca tcctgctatg accacccatg ctttctgagg 1080

gggatggggg acccagtgca gtcttggaag gggccgggcc ttccacgtgc tggatgctca 1140

ggtttgggcc accccatcca gctagaccct aaccagaaga cccctgaaaa cagcaagagc 1200

ctcaatgctg tgtacccaag agcagggagg aaacctccct catgcccagc tcctggcccc 1260

actggggcag ccagcatcgt cccctctgtg ccgggaatgg ccttggacct gtctcagatc 1320

cccaccaagg agctggaccg cttcatccag gaccacctga agccgagccc ccagttccag 1380

gagcaggtga aaaaggccat cgacatcatc ttgcgctgcc tccatgagaa ctgtgttcac 1440

aaggcctcaa gagtcagtaa agggggctca tttggccggg gcacagacct aagggatggc 1500

tgtgatgttg aactcatcat cttcctcaac tgcttcacgg actacaagga ccaggggccc 1560

cgccgcgcag agatccttga tgagatgcga gcgcagctag aatcctggtg gcaggaccag 1620

gtgcccagcc tgagccttca gtttcctgag cagaatgtgc ctgaggctct gcagttccag 1680

ctggtgtcca cagccctgaa gagctggacg gatgttagcc tgctgcctgc cttcgatgct 1740

gtggggcagc tcagttctgg caccaaacca aatccccagg tctactcgag gctcctcacc 1800

agtggctgcc aggagggcga gcataaggcc tgcttcgcag agctgcggag gaacttcatg 1860

aacattcgcc ctgtcaagct gaagaacctg attctgctgg tgaagcactg gtaccgccag 1920

gttgcggctc agaacaaagg aaaaggacca gcccctgcct ctctgccccc agcctatgcc 1980

ctggagctcc tcaccatctt tgcctgggag cagggctgca ggcaggattg tttcaacatg 2040

gcccaaggct tccggacggt gctggggctc gtgcaacagc atcagcagct ctgtgtctac 2100

tggacggtca actatagcac tgaggaccca gccatgagaa tgcaccttct tggccagctt 2160

cgaaaaccca gacccctggt cctggacccc gctgatccca cctggaacgt gggccacggt 2220

agctgggagc tgttggccca ggaagcagca gcgctgggga tgcaggcctg ctttctgagt 2280

agagacggga catctgtgca gccctgggat gtgatgccag ccctccttta ccaaacccca 2340

gctggggacc ttgacaagtt catcagtgaa tttctccagc ccaaccgcca gttcctggcc 2400

caggtgaaca aggccgttga taccatctgt tcatttttga aggaaaactg cttccggaat 2460

tctcccatca aagtgatcaa ggtggtcaag ggtggctctt cagccaaagg cacagctctg 2520

cgaggccgct cagatgccga cctcgtggtg ttcctcagct gcttcagcca gttcactgag 2580

cagggcaaca agcgggccga gatcatctcc gagatccgag cccagctgga ggcatgtcaa 2640

caggagcggc agttcgaggt caagtttgaa gtctccaaat gggagaatcc ccgcgtgctg 2700

agcttctcac tgacatccca gacgatgctg gaccagagtg tggactttga tgtgctgcca 2760

gcctttgacg ccctaggcca gctggtctct ggctccaggc ccagctctca agtctacgtc 2820

gacctcatcc acagctacag caatgcgggc gagtactcca cctgcttcac agagctacaa 2880

cgggacttca tcatctctcg ccctaccaag ctgaagagcc tgatccggct ggtgaagcac 2940

tggtaccagc agtgtaccaa gatctccaag gggagaggct ccctaccccc acagcacggg 3000

ctggaactcc tgactgtgta tgcctgggag cagggcggga aggactccca gttcaacatg 3060

gctgagggct tccgcacggt cctggagctg gtcacccagt accgccagct ctgtatctac 3120

tggaccatca actacaacgc caaggacaag actgttggag acttcctgaa acagcagctt 3180

cagaagccca ggcctatcat cctggatccg gctgacccga caggcaacct gggccacaat 3240
gcccgctggg acctgctggc caaggaagct gcagcctgca catctgccct gtgctgcatg 3300
ggacggaatg gcatccccat ccagccatgg ccagtgaagg ctgctgtgtg aagttgagaa 3360
aatcagcggt cctactggat gaagagaaga tggacaccag ccctcagcat gaggaaattc 3420
agggtcccct accagatgag agagattgtg tacatgtgtg tgtgagcaca tgtgtgcatg 3480
tgtgtgcaca cgtgtgcatg tgtgtgtttt agtgaatctg ctctcccagc tcacacactc 3540
ccctgcctcc catggcttac acactaggat ccagactcca tggtttgaca ccagcctgcg 3600
tttgcagctt ctctgtcact tccatgactc tatcctcata ccaccactgc tgcttcccac 3660
ccagctgaga atgccccctc ctccctgact cctctctgcc catgcaaatt agctcacatc 3720
tttcctcctg ctgcaatcca tcccttcctc ccattggcct ctccttgcca aatctaaata 3780
gtttatatag ggatggcaga gagttcccat ctcatctgtc agccacagtc atttggtact 3840
ggctacctgg agccttatct tctgaagggt tttaaagaat ggccaattag ctgagaagaa 3900
ttatctaatc aattagtgat gtctgccatg gatgcagtag aggaaagtgg tggtacaagt 3960
gccatgattg attagcaatg tctgcactgg atacggaaaa aagaaggtgc ttgcaggttt 4020
acagtgtata tgtgggctat tgaagagccc tctgagctcg gttgctagca ggagagcatg 4080
cccatattgg cttactttgt ctgccacaga cacagacaga gggagttggg acatgcatgc 4140
tatggggacc ctcttgttgg acacctaatt ggatgcctct tcatgagagg cctccttttc 4200
ttcacctttt atgctgcact cctcccctag tttacacatc ttgatgctgt ggctcagttt 4260
gccttcctga atttttattg ggtccctgtt ttctctccta acatgctgag attctgcatc 4320
cccacagcct aaactgagcc agtggccaaa caaccgtgct cagcctgttt ctctctgccc 4380
tctagagcaa ggcccaccag gtccatccag gaggctctcc tgacctcaag tccaacaaca 4440
gtgtccacac tagtcaaggt tcagcccaga aaacagaaag cactctagga atcttaggca 4500
gaaagggatt ttatctaaat cactggaaag gctggaggag cagaaggcag aggccaccac 4560
tggactattg gtttcaatat tagaccactg tagccgaatc agaggccaga gagcagccac 4620
tgctactgct aatgccacca ctacccctgc catcactgcc ccacatggac aaaactggag 4680
tcgagaccta ggttagattc ctgcaaccac aaacatccat cagggatggc cagctgccag 4740
agctgcggga agacggatcc cacctccctt tcttagcaga atctaaatta cagccagacc 4800
tctggctgca gaggagtctg agacatgtat gattgaatgg gtgccaagtg ccagggggcg 4860
gagtccccag cagatgcatc ctggccatct gttgcgtgga tgagggagtg ggtctatctc 4920
agaggaagga acaggaaaca aagaaaggaa gccactgaac atcccttctc tgctccacag 4980
gagtgcctta gacagcctga ctctccacaa accactgtta aaacttacct gctaggaatg 5040
ctagattgaa tgggatggga agagccttcc ctcattattg tcattcttgg agagaggtga 5100

gcaaccaagg gaagctcctc tgattcacct agaacctgtt ctctgccgtc tttggctcag 5160

cctacagaga ctagagtagg tgaagggaca gaggacaggg cttctaatac ctgtgccata 5220

ttgacagcct ccatccctgt cccccatctt ggtgctgaac caacgctaag ggcaccttct 5280

tagactcacc tcatcgatac tgcctggtaa tccaaagcta gaactctcag gaccccaaac 5340

tccacctctt ggattggccc tggctgctgc cacacacata tccaagagct cagggccagt 5400

tctggtgggc agcagagacc tgctctgcca agttgtccag cagcagagtg gccctggcct 5460

gggcatcaca agccagtgat gctcctggga agaccaggtg gcaggtcgca gttgggtacc 5520

ttccattccc accacacaga ctctgggcct ccccgcaaaa tggctccaga attagagtaa 5580

ttatgagatg gtgggaacca gagcaactca ggtgcatgat acaaggagag gttgtcatct 5640

gggtagggca gagaggaggg cttgctcatc tgaacagggg tgtatttcat tccaggccct 5700

cagtctttgg caatggccac cctggtgttg gcatattggc cccactgtaa cttttggggg 5760

cttcccggtc tagccacacc ctcggatgga aagacttgac tgcataaaga tgtcagttct 5820

ccctgagttg attgataggc ttaatggtca ccctaaaaac acccacatat gcttttcgat 5880

ggaaccaggt aagttgacgc taaagttctt atggaaaaat acacacgcaa tagctaggaa 5940

aacacaggga aagaagagtt ctgagcaggg cctagtctta gccaatatta aaacatacta 6000

tgaagcctct gatacttaaa cagcatggcg ctggtacgta aatagaccaa tgcagttagg 6060

tggctctttc caagactctg gggaaaaaag tagtaaaaag ctaaatgcaa tcaatcagca 6120

attgaaagct aagtgagaga gccagagggc ctccttggtg gtaaaagagg gttgcatttc 6180

ttgcagccag aaggcagaga aagtgaagac caagtccaga actgaatcct aagaaatgca 6240

ggactgcaaa gaaattggtg tgtgtgtgtg tgtgtgtgtg tgtgtgtgtg tttaattttt 6300

aaaaagtttt tattgagata caagtcaata ccataaagct ctcacccttc taaagtgtac 6360

aattcagtgg tgtgagtata ttcataagat ttatacttgg tgtctattca taagacttat 6420

atccagcata ttcataacta gagccatatc acagatgcat tcatcataat aattccagac 6480

attttcatca ccctaaaagg aaaccctgaa acccattagc agtcattccc cattcctcca 6540

acccattctc tccctaatcc ctagaaacca ccaatctgct gtgtatttca tctattgcca 6600

acatttcata taaatggcat catacaaaaa aaaaaaaaaa aaaaaa 6646

<210> 29
<211> 3264
<212> DNA
<213> Homo sapiens


<220>
<221> CDS
<222> (1)..(3264)

<400> 29
atg gac ttg tac agc acc ccg gcc gct gcg ctg gac agg ttc gtg gcc 48
aga agg ctg cag ccg cgg aag gag ttc gta gag aag gcg cgg cgc gct      96
ctg ggc gcc ctg gcc gct gcc ctg agg gag cgc ggg ggc cgc ctc ggt      144
gct gct gcc ccg cgg gtg ctg aaa act gtc aag gga ggc tcc tcg ggc      192
cgg ggc aca gct ctc aag ggt ggc tgt gat tct gaa ctt gtc atc ttc      240
ctc gac tgc ttc aag agc tat gtg gac cag agg gcc cgc cgt gca gag      288
atc ctc agt gag atg cgg gca tcg ctg gaa tcc tgg tgg cag aac cca      336
gtc cct ggt ctg aga ctc acg ttt cct gag cag agc gtg cct ggg gcc      384
ctg cag ttc cgc ctg aca tcc gta gat ctt gag gac tgg atg gat gtt      432
agc ctg gtg cct gcc ttc aat gtc ctg ggt cag gcc ggc tcc ggc gtc      480
aaa ccc aag cca caa gtc tac tct acc ctc ctc aac agt ggc tgc caa      528
ggg ggc gag cat gcg gcc tgc ttc aca gag ctg cgg agg aac ttt gtg      576
aac att cgc cca gcc aag ttg aag aac cta atc ttg ctg gtg aag cac      624
tgg tac cac cag gtg tgc cta cag ggg ttg tgg aag gag acg ctg ccc      672
ccg gtc tat gcc ctg gaa ttg ctg acc atc ttc gcc tgg gag cag ggc      720
tgt aag aag gat gct ttc agc cta gcc gaa ggc ctc cga act gtc ctg      768
ggc ctg atc caa cag cat cag cac ctg tgt gtt ttc tgg act gtc aac      816
tat ggc ttc gag gac cct gca gtt ggg cag ttc ttg cag cgg cag ctt         864
aag aga ccc agg cct gtg atc ctg gac cca gct gac ccc aca tgg gac         912
ctg ggg aat ggg gca gcc tgg cac tgg gat ttg cta gcc cag gag gca         960
gca tcc tgc tat gac cac cca tgc ttt ctg agg ggg atg ggg gac cca        1008
gtg cag tct tgg aag ggg ccg ggc ctt cca cgt gct gga tgc tca ggt        1056
ttg ggc cac ccc atc cag cta gac cct aac cag aag acc cct gaa aac        1104
agc aag agc ctc aat gct gtg tac cca aga gca ggg agg aaa cct ccc        1152
tca tgc cca gct cct ggc ccc act ggg gca gcc agc atc gtc ccc tct        1200
gtg ccg gga atg gcc ttg gac ctg tct cag atc ccc acc aag gag ctg        1248
gac cgc ttc atc cag gac cac ctg aag ccg agc ccc cag ttc cag gag        1296
cag gtg aaa aag gcc atc gac atc atc ttg cgc tgc ctc cat gag aac        1344
tgt gtt cac aag gcc tca aga gtc agt aaa ggg ggc tca ttt ggc cgg        1392
ggc aca gac cta agg gat ggc tgt gat gtt gaa ctc atc atc ttc ctc        1440
aac tgc ttc acg gac tac aag gac cag ggg ccc cgc cgc gca gag atc        1488
ctt gat gag atg cga gcg cag cta gaa tcc tgg tgg cag gac cag gtg        1536
ccc agc ctg agc ctt cag ttt cct gag cag aat gtg cct gag gct ctg        1584
cag ttc cag ctg gtg tcc aca gcc ctg aag agc tgg acg gat gtt agc 1632
ctg ctg cct gcc ttc gat gct gtg ggg cag ctc agt tct ggc acc aaa 1680
cca aat ccc cag gtc tac tcg agg ctc ctc acc agt ggc tgc cag gag 1728
ggc gag cat aag gcc tgc ttc gca gag ctg cgg agg aac ttc atg aac 1776
att cgc cct gtc aag ctg aag aac ctg att ctg ctg gtg aag cac tgg 1824
tac cgc cag gtt gcg gct cag aac aaa gga aaa gga cca gcc cct gcc 1872
tct ctg ccc cca gcc tat gcc ctg gag ctc ctc acc atc ttt gcc tgg 1920
gag cag ggc tgc agg cag gat tgt ttc aac atg gcc caa ggc ttc cgg 1968
acg gtg ctg ggg ctc gtg caa cag cat cag cag ctc tgt gtc tac tgg 2016
acg gtc aac tat agc act gag gac cca gcc atg aga atg cac ctt ctt 2064
ggc cag ctt cga aaa ccc aga ccc ctg gtc ctg gac ccc gct gat ccc 2112
acc tgg aac gtg ggc cac ggt agc tgg gag ctg ttg gcc cag gaa gca 2160
gca gcg ctg ggg atg cag gcc tgc ttt ctg agt aga gac ggg aca tct 2208
gtg cag ccc tgg gat gtg atg cca gcc ctc ctt tac caa acc cca gct 2256
ggg gac ctt gac aag ttc atc agt gaa ttt ctc cag ccc aac cgc cag 2304
ttc ctg gcc cag gtg aac aag gcc gtt gat acc atc tgt tca ttt ttg 2352
aag gaa aac tgc ttc cgg aat tct ccc atc aaa gtg atc aag gtg gtc 2400
aag ggt ggc tct tca gcc aaa ggc aca gct ctg cga ggc cgc tca gat 2448
gcc gac ctc gtg gtg ttc ctc agc tgc ttc agc cag ttc act gag cag 2496
ggc aac aag cgg gcc gag atc atc tcc gag atc cga gcc cag ctg gag 2544
gca tgt caa cag gag cgg cag ttc gag gtc aag ttt gaa gtc tcc aaa 2592
tgg gag aat ccc cgc gtg ctg agc ttc tca ctg aca tcc cag acg atg 2640
ctg gac cag agt gtg gac ttt gat gtg ctg cca gcc ttt gac gcc cta 2688
ggc cag ctg gtc tct ggc tcc agg ccc agc tct caa gtc tac gtc gac 2736
ctc atc cac agc tac agc aat gcg ggc gag tac tcc acc tgc ttc aca 2784
gag cta caa cgg gac ttc atc atc tct cgc cct acc aag ctg aag agc 2832
ctg atc cgg ctg gtg aag cac tgg tac cag cag tgt acc aag atc tcc 2880
aag ggg aga ggc tcc cta ccc cca cag cac ggg ctg gaa ctc ctg act 2928
gtg tat gcc tgg gag cag ggc ggg aag gac tcc cag ttc aac atg gct 2976
gag ggc ttc cgc acg gtc ctg gag ctg gtc acc cag tac cgc cag ctc 3024
tgt atc tac tgg acc atc aac tac aac gcc aag gac aag act gtt 3069
gga gac ttc ctg aaa cag cag ctt cag aag ccc agg cct atc atc 3114
ctg gat ccg gct gac ccg aca ggc aac ctg ggc cac aat gcc cgc 3159
tgg gac ctg ctg gcc aag gaa gct gca gcc tgc aca tct gcc ctg 3204
tgc tgc atg gga cgg aat ggc atc ccc atc cag cca tgg cca gtg 3249
aag gct gct gtg tga 3264

<210> 30
<211> 1087
<212> PRT
<213> Homo sapiens

<400> 30

<210>  31
<211>  20
<212>  DNA
<213>  artificial sequence

<220>
<223>  OAS1/pro1 F-primer

<400>  31
ccaggagttc gagaccagac                                                20

<210>  32
<211>  20
<212>  DNA
<213>  artificial sequence

<220>
<223>  OAS1/pro1 F-primer

<400>  32
ggtgaggcat aggggatttt                                                20

<210>  33
<211>  20

<212>  DNA
<213>  artificial sequence

<220>
<223>  OAS1/pro3 F-primer

<400>  33
ccttgaaccc aggaagttga                                                    20


<210>  34
<211>  20
<212>  DNA
<213>  artificial sequence

<220>
<223>  OAS1/pro4 F-primer

<400>  34
ctttttgcag tgggcatgta                                                    20


<210>  35
<211>  20
<212>  DNA
<213>  artificial sequence

<220>
<223>  OAS1/exon1 F-primer

<400>  35
tttgcaaaag gaaagtgcaa                                                    20


<210>  36
<211>  20
<212>  DNA
<213>  artificial sequence

<220>
<223>  OAS1/exon2 F-primer

<400>  36
agcatccatt ttcccatctg                                                    20


<210>  37
<211>  20
<212>  DNA
<213>  artificial sequence

<220>
<223>  OAS1/exon3 F-primer

<400>  37
ggatcaggaa tggacctcaa                                                    20


<210>  38
<211>  20
<212>  DNA
<213>  artificial sequence

<220>
<223>  OAS1/exon4 F-primer

<400>  38
aatgaatgag cctggattcg                                                   20


<210>  39
<211>  20
<212>  DNA
<213>  artificial sequence

<220>
<223>  OAS1/exon5 F-primer

<400>  39
gagcccttcc tcatgttctg                                                   20


<210>  40
<211>  20
<212>  DNA
<213>  artificial sequence

<220>
<223>  OAS1/exon6-1 F-primer

<400>  40
agctggggct tgttagtcct                                                   20


<210>  41
<211>  20
<212>  DNA
<213>  artificial sequence

<220>
<223>  OAS1/exon6-2 F-primer

<400>  41
gctcctcagt gagctggtgt                                                   20


<210>  42
<211>  21
<212>  DNA
<213>  artificial sequence

<220>
<223>  OAS1/exon6-3 F-primer

<400>  42
cggccatgtc attgttttta t                                                 21


<210>  43
<211>  20
<212>  DNA
<213>  artificial sequence

<220>
<223>  OAS2/pro1 F-primer

<400>  43
aaagcacagg ctttggagtc                                                   20

<210>  44
<211>  20
<212>  DNA
<213>  artificial sequence

<220>
<223>  OAS2/pro2 F-primer

<400>  44
ttgggggagc tcagtcttta                                                    20


<210>  45
<211>  20
<212>  DNA
<213>  artificial sequence

<220>
<223>  OAS2/pro3 F-primer

<400>  45
tttctggctc acacacttgg                                                    20


<210>  46
<211>  20
<212>  DNA
<213>  artificial sequence

<220>
<223>  OAS2/pro4 F-primer

<400>  46
cgatgtgctc aaggacagac                                                    20


<210>  47
<211>  20
<212>  DNA
<213>  artificial sequence

<220>
<223>  OAS2/exon1 F-primer

<400>  47
cctcccatcc taccattcac                                                    20


<210>  48
<211>  20
<212>  DNA
<213>  artificial sequence

<220>
<223>  OAS2/exon2 F-primer

<400>  48
atgagcatcc caatcaggtc                                                    20


<210>  49
<211>  21
<212>  DNA
<213>  artificial sequence

<220>
<223>  OAS2/exon3 F-primer

<400>  49
tgaccgatta agcaagagtc g                                                21


<210>  50
<211>  20
<212>  DNA
<213>  artificial sequence

<220>
<223>  OAS2/exon4 F-primer

<400>  50
taagagaggt ccctgctcca                                                  20


<210>  51
<211>  20
<212>  DNA
<213>  artificial sequence

<220>
<223>  OAS2/exon5 F-primer

<400>  51
gaggggcttg atgtcgtaga                                                  20


<210>  52
<211>  20
<212>  DNA
<213>  artificial sequence

<220>
<223>  OAS2/exon6 F-primer

<400>  52
ttgtgtgcca ctccaaactc                                                  20


<210>  53
<211>  20
<212>  DNA
<213>  artificial sequence

<220>
<223>  OAS2/exon7 F-primer

<400>  53
cagaacttgg ccttggaaac                                                  20


<210>  54
<211>  20
<212>  DNA
<213>  artificial sequence

<220>
<223>  OAS2/exon8 F-primer

<400>  54
ggagatgctc cctgtgtctt                                                  20

<210>  55
<211>  20
<212>  DNA
<213>  artificial sequence

<220>
<223>  OAS2/exon9 F-primer

<400>  55
tccaagctgc agagtgtgag                                                    20


<210>  56
<211>  20
<212>  DNA
<213>  artificial sequence

<220>
<223>  OAS2/exon10-1 F-primer

<400>  56
cgcggcctct agaaagttac                                                    20


<210>  57
<211>  20
<212>  DNA
<213>  artificial sequence

<220>
<223>  OAS2/exon10-2 F-primer

<400>  57
aggcaggtaa ccccagattc                                                    20


<210>  58
<211>  20
<212>  DNA
<213>  artificial sequence

<220>
<223>  OAS2/exon10-3 F-primer

<400>  58
tggttctctc caccaggttc                                                    20


<210>  59
<211>  20
<212>  DNA
<213>  artificial sequence

<220>
<223>  OAS2/exon11-1 F-primer

<400>  59
gtagggaaga ggtggccaag                                                    20


<210>  60
<211>  20
<212>  DNA

<213>  artificial sequence

<220>
<223>  OAS2/exon11-2 F-primer

<400>  60
gtcttgcctt gctgaactcc                                                    20


<210>  61
<211>  20
<212>  DNA
<213>  artificial sequence

<220>
<223>  OAS2/exon11-3 F-primer

<400>  61
ccagccaacc ctttcattag                                                    20


<210>  62
<211>  20
<212>  DNA
<213>  artificial sequence

<220>
<223>  OAS3/pro1 F-primer

<400>  62
tattgaccct tctgcgttcc                                                    20


<210>  63
<211>  20
<212>  DNA
<213>  artificial sequence

<220>
<223>  OAS3/pro2 F-primer

<400>  63
ggagagagga cctgggagtt                                                    20


<210>  64
<211>  20
<212>  DNA
<213>  artificial sequence

<220>
<223>  OAS3/pro3 F-primer

<400>  64
ccagccagac aactttccag                                                    20


<210>  65
<211>  20
<212>  DNA
<213>  artificial sequence

<220>
<223>  OAS3/exon1 F-primer

<400>  65
aagtgggtgt caggtccaag                                                   20


<210>  66
<211>  20
<212>  DNA
<213>  artificial sequence

<220>
<223>  OAS3/exon2 F-primer

<400>  66
acagtctctc cccagcacac                                                   20


<210>  67
<211>  20
<212>  DNA
<213>  artificial sequence

<220>
<223>  OAS3/exon3 F-primer

<400>  67
aaagctccag gctctcttcc                                                   20


<210>  68
<211>  20
<212>  DNA
<213>  artificial sequence

<220>
<223>  OAS3/exon4 F-primer

<400>  68
ggctgaagct accagtgagg                                                   20


<210>  69
<211>  20
<212>  DNA
<213>  artificial sequence

<220>
<223>  OAS3/exon5 F-primer

<400>  69
tcgaggctca gagagggtaa                                                   20


<210>  70
<211>  20
<212>  DNA
<213>  artificial sequence

<220>
<223>  OAS3/exon6 F-primer

<400>  70
ggcacctttt tcaactctgc                                                   20


<210>  71

<211>  20
<212>  DNA
<213>  artificial sequence

<220>
<223>  OAS3/exon7 F-primer

<400>  71
catgcagtcg acctttgtca                                                    20


<210>  72
<211>  21
<212>  DNA
<213>  artificial sequence

<220>
<223>  OAS3/exon8 F-primer

<400>  72
ccactgtgca gttactggat g                                                  21


<210>  73
<211>  20
<212>  DNA
<213>  artificial sequence

<220>
<223>  OAS3/exon9 F-primer

<400>  73
ctaggcaaaa gccacgtctg                                                    20


<210>  74
<211>  20
<212>  DNA
<213>  artificial sequence

<220>
<223>  OAS3/exon10 F-primer

<400>  74
cccaatagct ttccaaccaa                                                    20


<210>  75
<211>  20
<212>  DNA
<213>  artificial sequence

<220>
<223>  OAS3/exon11 F-primer

<400>  75
cctcagaacc tacccaccag                                                    20


<210>  76
<211>  21
<212>  DNA
<213>  artificial sequence

<220>

<223>  OAS3/exon12 F-primer

<400>  76
tgacttgtcc aaggtcacac a                                                21


<210>  77
<211>  20
<212>  DNA
<213>  artificial sequence

<220>
<223>  OAS3/exon13 F-primer

<400>  77
atcccagacc acccttagga                                                  20


<210>  78
<211>  20
<212>  DNA
<213>  artificial sequence

<220>
<223>  OAS3/exon14 f-primer

<400>  78
aaagttttgg ggttgctttg                                                  20


<210>  79
<211>  20
<212>  DNA
<213>  artificial sequence

<220>
<223>  OAS3/exon15 F-primer

<400>  79
tctagcccct gcaaagtgtt                                                  20


<210>  80
<211>  20
<212>  DNA
<213>  artificial sequence

<220>
<223>  OAS3/exon16-1 F-primer

<400>  80
atctgtggtg ccaaaggaag                                                  20


<210>  81
<211>  20
<212>  DNA
<213>  artificial sequence

<220>
<223>  OAS3/exon16-2 F-primer

<400>  81
cctcccatgg cttacacact                                                  20

<210>  82
<211>  20
<212>  DNA
<213>  artificial sequence

<220>
<223>  OAS3/exon16-3 F-primer

<400>  82
caggagagca tgcccatatt                                                    20


<210>  83
<211>  20
<212>  DNA
<213>  artificial sequence

<220>
<223>  OAS3/exon16-4 F-primer

<400>  83
gagattctgc atccccacag                                                    20


<210>  84
<211>  20
<212>  DNA
<213>  artificial sequence

<220>
<223>  OAS3/exon16-5 F-primer

<400>  84
ctctggctgc agaggagtct                                                    20


<210>  85
<211>  20
<212>  DNA
<213>  artificial sequence

<220>
<223>  OAS3/exon16-6 F-primer

<400>  85
aacgctaagg gcaccttctt                                                    20


<210>  86
<211>  20
<212>  DNA
<213>  artificial sequence

<220>
<223>  OAS3/exon16-7 F-primer

<400>  86
caccctggtg ttggcatatt                                                    20


<210>  87
<211>  20
<212>  DNA
<213>  artificial sequence

<220>
<223>  OAS1/pro1 R-primer

<400>  87
tgcagggaca gtcacagaac                                                   20


<210>  88
<211>  20
<212>  DNA
<213>  artificial sequence

<220>
<223>  OAS1/pro1 R-primer

<400>  88
ataggaggtg gggcttgact                                                   20


<210>  89
<211>  20
<212>  DNA
<213>  artificial sequence

<220>
<223>  OAS1/pro3 R-primer

<400>  89
cctttgtcct ttagccagca                                                   20


<210>  90
<211>  20
<212>  DNA
<213>  artificial sequence

<220>
<223>  OAS1/pro4 R-primer

<400>  90
tgtcaatggc atggttgatt                                                   20


<210>  91
<211>  20
<212>  DNA
<213>  artificial sequence

<220>
<223>  OAS1/exon1 R-primer

<400>  91
gaaaccacca tcccagaaga                                                   20


<210>  92
<211>  20
<212>  DNA
<213>  artificial sequence

<220>
<223>  OAS1/exon2 R-primer

<400>  92

cccaccctgc tttagagaga                                                    20

<210>  93
<211>  20
<212>  DNA
<213>  artificial sequence

<220>
<223>  OAS1/exon3 R-primer

<400>  93
ggctcctctc tccacctctt                                                    20

<210>  94
<211>  20
<212>  DNA
<213>  artificial sequence

<220>
<223>  OAS1/exon4  R-primer

<400>  94
tacaatgatg ggcaacagga                                                    20

<210>  95
<211>  20
<212>  DNA
<213>  artificial sequence

<220>
<223>  OAS1/exon5 R-primer

<400>  95
ataaatggcc tttggcaaga                                                    20

<210>  96
<211>  20
<212>  DNA
<213>  artificial sequence

<220>
<223>  OAS1/exon6-1 R-primer

<400>  96
tggaatgaag tgaggctgtg                                                    20

<210>  97
<211>  20
<212>  DNA
<213>  artificial sequence

<220>
<223>  OAS1/exon6-2 R-primer

<400>  97
tccatgagga cagggatttt                                                    20

<210>  98
<211>  20

<212>  DNA
<213>  artificial sequence

<220>
<223>  OAS1/exon6-3 R-primer

<400>  98
actgccagag tggagatgct                                                    20


<210>  99
<211>  20
<212>  DNA
<213>  artificial sequence

<220>
<223>  OAS2/pro1 R-primer

<400>  99
tgagcccagg tacccagata                                                    20


<210>  100
<211>  20
<212>  DNA
<213>  artificial sequence

<220>
<223>  OAS2/pro2 R-primer

<400>  100
ctgacttgct cagggacaca                                                    20


<210>  101
<211>  20
<212>  DNA
<213>  artificial sequence

<220>
<223>  OAS2/pro3 R-primer

<400>  101
agcagggaaa ccaaaactga                                                    20


<210>  102
<211>  20
<212>  DNA
<213>  artificial sequence

<220>
<223>  OAS2/pro4 R-primer

<400>  102
ccatttccca ttgctctcag                                                    20


<210>  103
<211>  20
<212>  DNA
<213>  artificial sequence

<220>
<223>  OAS2/exon1 R-primer

<400>  103
gtcacacagc tctggagcaa                                                   20


<210>  104
<211>  20
<212>  DNA
<213>  artificial sequence

<220>
<223>  OAS2/exon2 R-primer

<400>  104
gagagcgagt ccagggtaga                                                   20


<210>  105
<211>  22
<212>  DNA
<213>  artificial sequence

<220>
<223>  OAS2/exon3 R-primer

<400>  105
tgttgcactt tactggatgt ca                                                22


<210>  106
<211>  20
<212>  DNA
<213>  artificial sequence

<220>
<223>  OAS2/exon4 R-primer

<400>  106
ggacctcccc tctttcagac                                                   20


<210>  107
<211>  20
<212>  DNA
<213>  artificial sequence

<220>
<223>  OAS2/exon5 R-primer

<400>  107
ctctgccaac accctgattt                                                   20


<210>  108
<211>  20
<212>  DNA
<213>  artificial sequence

<220>
<223>  OAS2/exon6 R-primer

<400>  108
taactcctgg cttcccttcc                                                   20

<210>  109
<211>  20
<212>  DNA
<213>  artificial sequence

<220>
<223>  OAS2/exon7 R-primer

<400>  109
cccatgcttg gaaaagagaa                                                   20


<210>  110
<211>  20
<212>  DNA
<213>  artificial sequence

<220>
<223>  OAS2/exon8 R-primer

<400>  110
gggttagtga tgggaacgtc                                                   20


<210>  111
<211>  20
<212>  DNA
<213>  artificial sequence

<220>
<223>  OAS2/exon9 R-primer

<400>  111
gcccctgcta ggttttatcc                                                   20


<210>  112
<211>  20
<212>  DNA
<213>  artificial sequence

<220>
<223>  OAS2/exon10-1 R-primer

<400>  112
ggttgtcact gggagaggag                                                   20


<210>  113
<211>  20
<212>  DNA
<213>  artificial sequence

<220>
<223>  OAS2/exon10-2 R-primer

<400>  113
gcacaagaga gcctccaaac                                                   20


<210>  114
<211>  20
<212>  DNA
<213>  artificial sequence

<220>
<223>  OAS2/exon10-3 R-primer

<400>  114
ggatggatcc tagctccaca                                                    20


<210>  115
<211>  21
<212>  DNA
<213>  artificial sequence

<220>
<223>  OAS2/exon11-1 R-primer

<400>  115
gactggaagg aaaaaggagg a                                                  21


<210>  116
<211>  20
<212>  DNA
<213>  artificial sequence

<220>
<223>  OAS2/exon11-2 R-primer

<400>  116
aaggctaaag cccaaggaaa                                                    20


<210>  117
<211>  20
<212>  DNA
<213>  artificial sequence

<220>
<223>  OAS2/exon11-3 R-primer

<400>  117
cacatgcttg gtctttctgg                                                    20


<210>  118
<211>  20
<212>  DNA
<213>  artificial sequence

<220>
<223>  OAS3/pro1 R-primer

<400>  118
gcacacctga gctctcttcc                                                    20


<210>  119
<211>  20
<212>  DNA
<213>  artificial sequence

<220>
<223>  OAS3/pro2 R-primer

<400>  119
cgaccatggt gtaggtgcta                                                    20

<210>  120
<211>  20
<212>  DNA
<213>  artificial sequence

<220>
<223>  OAS3/pro3 R-primer

<400>  120
tcggatttct ggtttcgttt                                                    20


<210>  121
<211>  20
<212>  DNA
<213>  artificial sequence

<220>
<223>  OAS3/exon1 R-primer

<400>  121
tgtcaagcgg gcttaagagt                                                    20


<210>  122
<211>  20
<212>  DNA
<213>  artificial sequence

<220>
<223>  OAS3/exon2 R-primer

<400>  122
cccaggtcaa acagcaagtt                                                    20


<210>  123
<211>  21
<212>  DNA
<213>  artificial sequence

<220>
<223>  OAS3/exon3 R-primer

<400>  123
tgcacaaggg acataaacag a                                                  21


<210>  124
<211>  20
<212>  DNA
<213>  artificial sequence

<220>
<223>  OAS3/exon4 R-primer

<400>  124
ctaatgtgca tctgggctga                                                    20


<210>  125
<211>  20
<212>  DNA

<213>  artificial sequence

<220>
<223>  OAS3/exon5 R-primer

<400>  125
gcctcccaat gtgttgagat                                                    20


<210>  126
<211>  20
<212>  DNA
<213>  artificial sequence

<220>
<223>  OAS3/exon6 R-primer

<400>  126
ccagcaaatc atccttcctg                                                    20


<210>  127
<211>  22
<212>  DNA
<213>  artificial sequence

<220>
<223>  OAS3/exon7 R-primer

<400>  127
tcaatgcttg gtcaagtagg ag                                                 22


<210>  128
<211>  20
<212>  DNA
<213>  artificial sequence

<220>
<223>  OAS3/exon8 R-primer

<400>  128
aagcaaagga ggggagatgt                                                    20


<210>  129
<211>  20
<212>  DNA
<213>  artificial sequence

<220>
<223>  OAS3/exon9 R-primer

<400>  129
ggaagcagag tgaggagtgg                                                    20


<210>  130
<211>  20
<212>  DNA
<213>  artificial sequence

<220>
<223>  OAS3/exon10 R-primer

<400>  130
gctgggacgt agcaatctgt                                                    20


<210>  131
<211>  20
<212>  DNA
<213>  artificial sequence

<220>
<223>  OAS3/exon11 R-primer

<400>  131
ggacatatct gggccctctc                                                    20


<210>  132
<211>  20
<212>  DNA
<213>  artificial sequence

<220>
<223>  OAS3/exon12 R-primer

<400>  132
aaaggggccc tcttctttag                                                    20


<210>  133
<211>  19
<212>  DNA
<213>  artificial sequence

<220>
<223>  OAS3/exon13 R-primer

<400>  133
ctccaccaac ccaggatct                                                     19


<210>  134
<211>  20
<212>  DNA
<213>  artificial sequence

<220>
<223>  OAS3/exon14 R-primer

<400>  134
attgaccgtt tgtccctcag                                                    20


<210>  135
<211>  20
<212>  DNA
<213>  artificial sequence

<220>
<223>  OAS3/exon15 R-primer

<400>  135
atgggaaaat ggaagcacag                                                    20


<210>  136

<211>  20
<212>  DNA
<213>  artificial sequence

<220>
<223>  OAS3/exon16-1 R-primer

<400>  136
gcaggaggaa agatgtgagc                                                    20


<210>  137
<211>  20
<212>  DNA
<213>  artificial sequence

<220>
<223>  OAS3/exon16-2 R-primer

<400>  137
catagcatgc atgtcccaac                                                    20


<210>  138
<211>  19
<212>  DNA
<213>  artificial sequence

<220>
<223>  OAS3/exon16-3 R-primer

<400>  138
gagagcctcc tggatggac                                                     19


<210>  139
<211>  20
<212>  DNA
<213>  artificial sequence

<220>
<223>  OAS3/exon16-4 R-primer

<400>  139
tagacccact ccctcatcca                                                    20


<210>  140
<211>  20
<212>  DNA
<213>  artificial sequence

<220>
<223>  OAS3/exon16-5 R-primer

<400>  140
gccctgagct cttggatatg                                                    20


<210>  141
<211>  20
<212>  DNA
<213>  artificial sequence

<220>

<223>  OAS3/exon16-6 R-primer

<400>  141
gggtgttttt agggtgacca                                                    20


<210>  142
<211>  21
<212>  DNA
<213>  artificial sequence

<220>
<223>  OAS3/exon16-7 R-primer

<400>  142
agaagggtga gagctttatg g                                                  21